# EUROPEAN PATENT APPLICATION

(11) **EP 3 279 337 A1**
(43) Date of publication of application: **07.02.2018**
(21) Application number: 16382389.1
(22) Date of filing: 04.08.2016
(51) Int. Cl.: C12Q 1/68, C12Q 1/70

(54) **USE OF SHORT PROBES BETWEEN 8 AND 9 NUCLEOTIDES IN MULTIPLEX ASSAYS**

(71) Applicant: Servizo Galego de Saúde (SERGAS), 15703 Santiago de Composteia (ES); Fundación Profesor Novoa Santos, 15006 A Coruna (ES)
(72) Inventor: TOMÁS CARMONA, María del Mar, 15006 A Coruña (ES); BOU ARÉVALO, Germán, 15006 A Coruña (ES); FERNÁNDEZ GONZÁLEZ, Ana, 15006 A Coruña (ES); MOURE GONZÁLEZ, Raquel, 15006 A Coruña (ES)
(74) Representative: Hoffmann Eitle

(57) **Abstract**

The present invention provides a new PCR technique-based method for simultaneously detecting several pathogens present in one and the same sample. The present invention therefore relates to a method for the detection, quantification and identification of pathogens of two or more different strains or species of microorganisms in one and the same biological sample isolated from a subject through polymerase chain reaction (PCR), preferably performed in a quantitative manner in real time, which comprises detecting, identifying and quantifying the presence or absence of said microorganisms in said biological sample through the determination and quantification of the presence or absence of amplicons from the polymerase chain reaction (PCR), preferably performed in a quantitative manner in real time.

## Description

### Technical field of the invention

The present invention generally relates to the detection, identification and quantification of microorganisms, preferably pathogenic bacteria, and particularly to a method for the detection and multiple quantification of any combination of microorganisms, by means of multiplex amplification reaction using real-time polymerase chain reaction using fluorescently labeled probes consisting of a fluorophore-labeled oligonucleotide with a length of 8 to 9 nucleotides.

### Background of the invention

Today, the detection of pathogenic microorganisms, such as bacteria (*Escherichia coli, Streptococcus agalactiae, Listeria monocytogenes, Mycobacterium tuberculosis* complex, *Streptococcus pneumoniae, S. pyogenes, Haemophilus influenzae, Staphylococcus aureus, Kingella kingae, Bordetella pertussis, B. parapertussis, B. holmesii, Mycoplasma pneumoniae, Coxiella burnetti, Chlamydia pneumoniae and Legionella pneumophila),* virus (herpes simplex virus 1, herpes simplex virus 2, varicella zoster virus, VHS-8 [human herpesvirus 8], herpesvirus 4 [Epstein Barr], cytomegalovirus and adenovirus), fungi (*Candida albicans, C. parapsilopsis, C. glabrata, C. krusei, C. tropicalis, C. guilliermondii, Aspergillus flavus, A. fumigatus and Pneumocystis jiroveci)* and finally, parasites *(Dientamoeba fragilis, Giardia lamblia A, Giardia lamblia* B, *Crystosporidium parvum*/*hominis, Plasmodiun falciparum, P. ovale* and/or *P. vivax)* is a very important task in the field of medicine, particularly in the field for the medical diagnosis of infectious diseases such as tuberculosis, neonatal sepsis, septic arthritis, whooping cough, atypical pneumoniae, viral meningitis, virosis in transplant recipients, invasive fungal infections and parasitemias, so various methods for their detection and identification have been described.

Current methodologies that are considered more effective for the detection, quantification and identification of pathogens include, among others, those based on molecular techniques. These techniques include, among others, the polymerase chain reaction method, commonly known as PCR. The PCR method is generally considered as the quickest and most sensitive methodology used for detecting nucleic acids of pathogens in a specific test sample in particular, and the description thereof in the state of the art can be found in the works by Kary B. Mullis *et al.* in the patent family of US patents US-4683195, US-4683202, US-4800159, US-4889818, US-4965188, US-5008182, US-5038852, US-5079352, US-5176995, US-5310652, US-5310893, US-5322770, US-5333675, US-5352600, US-5374553, US-5386022, US-5405774, US-5407800, US-5418149, US-5420029, among others.

To carry out the PCR technique, basically at least one pair of oligonucleotide initiators or primers for each of the pathogens to be identified as well as a labeled probe for each of said pathogens are required. Each pair of initiators must be complementary to the nucleotide sequence to be detected. The first initiator will be complementary to the sequence binding with the 5' end and the second initiator will have a sequence complementary with the 3' end. The nucleotide sequences must have each pair of oligonucleotide initiators and probes specific to the pathogen to be detected, such that they do not react or crosslink with other pathogens.

Since the PCR technique is a quick and sensitive method for detecting pathogens individually, it can also be used for simultaneously detecting several pathogens present in one and the same sample. Examples of applications for the simultaneous multiple detection of pathogens using the PCR methodology are described by John W. Czajka in the publication of international patent application WO-0314704, and by Linxian Wo *et. al.* in the patent family of US patents US-5612473, US-5738995, US-5753444, US-5756701 and US-5846783.

However, the use of the PCR methodology for the simultaneous detection of several pathogens in a sample has its problems. The main obstacle of said methodology lies in the cross reactions that may occur due to the use of multiple nucleotide sequences for obtaining the preferred amplification of certain target sequences present in the sample at the expense of other sequences also present in the same sample. This is made worse if short probe sequences are used due to, a *priori,* the high inspecificity of said sequences and therefore, to the high risk of cross reactions and of low specificity in diagnosis.

### Brief description of the invention

The present invention provides a new PCR technique-based method for simultaneously detecting several pathogens present in one and the same sample. The present invention therefore relates to a method for the detection, quantification and identification of pathogens of two or more different strains or species of microorganisms in one and the same biological sample isolated from a subject through polymerase chain reaction (PCR), preferably performed in a quantitative manner in real time, which comprises detecting, identifying and quantifying the presence or absence of said microorganisms in said biological sample through the determination and quantification of the presence or absence of amplicons from the polymerase chain reaction (PCR), preferably performed in a quantitative manner in real time;
wherein said polymerase chain reaction (PCR) uses DNA, complementary DNA (cDNA) or single-stranded DNA obtained by the reverse transcription of ribonucleic acid (RNA) of the biological sample, at least one pair of oligonucleotide initiators or primers for each of the pathogens to be identified as well as at least one labeled probe for each of the pathogens to be identified, dNTPs, a suitable reaction buffer and a heat-stable DNA polymerase, wherein each of said labeled probes consist of a fluorophore-labeled oligonucleotide with a length of 8 to 9 nucleotides, and wherein each of said probes is labeled with a different fluorophore for each microorganism detected in the same reaction.

### Detailed description of the invention

### Definitions

In the context of the present invention, **"real-time quantitative PCR"** is understood as a variant of polymerase chain reaction (PCR) used for simultaneously amplifying and quantifying in an absolute manner the amplification product of deoxyribonucleic acid (DNA). To that end, it uses, like the conventional PCR, a DNA template, at least one pair of specific primers, dNTPs, a suitable reaction buffer and a heat-stable DNA polymerase; there is added to said mixture a fluorophore-labeled substance which, in a thermal cycler housing sensors for measuring fluorescence after the excitation of the fluorophore at the suitable wavelength, allows measuring the rate at which one or more specific products are generated. Said measurement is taken after each amplification cycle and for this reason it is also referred to as real-time PCR (i.e., simultaneous, immediate PCR). In many cases, the template used for quantitative PCR is not DNA from the outset, but can be a single-stranded complementary DNA (cDNA) obtained by the reverse transcription of ribonucleic acid (RNA).

In the context of the present invention, **"probe/probes"** are understood as those fluorescently labeled oligonucleotides used in quantitative PCR for detecting the appearance of the desired product. The underlying principle of this technique is based on using FRET or resonance energy transfer, which is a mechanism for transferring energy between chromophores. FRET is based on the ability to transfer the excitation from one chromophore to another nearby chromophore, generally when both are located in the same molecule, by means of a dipole-dipole coupling mechanism. If the chromophores are fluorescent chromophores (i.e., fluorochromes), the underlying mechanism is still the same: the energy is transferred, which leads to the appearance of fluorescence.

In the context of the present invention, **"Taqman probe/probes"** are understood as those probes used in real-time PCR which allow measuring the production of PCR products by means of a system consisting of probes labeled by means of two fluorochromes. The usefulness thereof lies in the fact that they have a fluorophore at their 3' end and a molecule at the 5' end blocking fluorescence emission (known as a «quencher»); this labeled probe specifically hybridizes in the central part of the PCR product to be obtained. In this manner, when PCR is performed (with the probe plus the pair of specific primers), the probe hybridizes in the amplicon, but due to how close the fluorophore is to the quencher, no fluorescence is emitted; when the polymerase runs into the probe, it hydrolyzes the probe by means of its 5'-3' exonuclease activity, which causes the separation of the quencher from the fluorochrome and, therefore, fluorescence emission. Fluorescence is related to the amount of amplicon produced.

In the context of the present invention, a **"short probe"** is understood as those fluorescently labeled oligonucleotides used in the quantitative PCR for detecting the appearance of the desired product, said oligonucleotides having a length of 8 or 9 nucleotides, preferably 8 nucleotides.

In the context of the present invention, **"amplicon/amplicons"** are understood as an amplified DNA (deoxyribonucleic acid) fragment detected by real-time PCR. They are also known as PCR products.

In the context of the present invention, **"amplification efficiency"** is understood as the capacity of the reaction to duplicate the number of copies of DNA strands in each cycle (Bustin, S. A. & Nolan, T., 2004, Analysis of mRNA Expression by Real-Time PCR, In: Real-Time PCR; An Essential Guide, K. J. Edwards et al., editors. Horizon Bioscience, Wymondham, p. 125-184). The efficiencies of real-time PCR are calculated from the standard curve slopes (included in the software) obtained after performing serial dilutions with the real-time PCR reactions (Pfaffl, 2004) according to the following formula: E=10[-1/slope]-1.

In the context of the present invention, the **Cp** (crossing point/cycle threshold) for each sample is understood as the number of cycles required to intercept the threshold value (the signal reaching a specific fluorescence level) called "threshold cycle" (Ct). The Ct is inversely proportional to the number of initial copies of the sample DNA.

In the context of the present invention, the UNG of Atlantic cod *(Gadus morhua)* is understood as the uracil-DNA-glycosylase with the amino acid sequence of SEQ ID No 1:

MEFFGESWKKHLSGEFGKPYFIKLMGFVAEERKHYTVYPPPHQVFTWTQMCDIKDVKVVILGQ
DPYHGPNQAHGLCFSVQRPVPPPPSLVNIYKELSTDIEDFVHPGHGDLSGWAKQGVLLLNAVL
TVRAHQANSHKERGWEQFTDAVVSWLNQNSNGLVFLLWGSYAQKKGSAIDRKRHHVLQTAH
PSPLSVYRGFFGCRHFSKTNELLQKSGKKPIDWKEL

It must be noted that in the context of the present invention, variants of the UNG of Atlantic cod (*Gadus morhua)* are understood as those UNGs with a sequence identity of 95% or more, 96% or more, 97% or more, 98% or more, 99% or more with SEQ ID No 1.

### Description of the invention

In the present invention, more than two, three, four, five, six, seven and up to eight different amplicons corresponding to different pathogens and genes have been simultaneously detected and quantified (see examples) using the real-time PCR technique with short probes. The verification of qPCR functionality and of the existence of interferences between the primers and probes in the multiplex reaction was tested by carrying out several assays, based on the hypothesis that there was a high probability of cross reactions occurring which substantially reduce the specificity of the methodology due to the size of the probes used.

In this sense, Example 1 relates to a real-time PCR kit indicated for the detection of M. *tuberculosis* complex. Particularly, this example shows the usefulness of the present invention for carrying out the simultaneous detection, quantification and identification of a pathogen and a plasma analyte in one and the same biological sample using short probes. The Cts obtained both for the pathogen, *M*. *tuberculosis,* and for the analyte, the *Bglob* gene, were those expected (detection limit of 10 copies of DNA and an amplification efficiency of 98.4-101.5%) at the concentrations and number of copies used in this example, which indicated the inexistence, a *priori,* of interferences between the primers and probes that may partially inhibit the simultaneous amplification of one of the targets.

On the other hand, the fact that the negative controls do not show amplification for the 2 targets, in addition to indicating that there has been no contamination in the qPCR amplification process, again showed that there are no interactions between the probes which may result in false positives.

Furthermore, surprisingly the Cts obtained for the detection of *M*. *tuberculosis* and the *Bglo* gene in multiplex and singleplex format were maintained. In other words, the simultaneous use of two pairs of primers and two short probes did not reduce the specificity of the method compared with the singleplex format.

Example 2 of the present invention relates to a real-time PCR kit indicated for the detection of *E. coli, S. agalactiae,* plasmid DNA *B-glob* gene and *L. monocytogenes.* Particularly, this example shows the usefulness of the present invention for carrying out the simultaneous detection, quantification and identification of three different pathogens and a plasma analyte in one and the same biological sample using short probes. Again, the Cts obtained both for *E. coli, S. agalactiae,* plasmid DNA *B-glob* gene and *L. monocytogenes* were those expected (detection limit of 10 copies of DNA and amplification efficiency between 85-96%) at the concentrations and number of copies used in this example, which indicated the inexistence, *a priori,* of interferences between the primers and probes that may partially inhibit the simultaneous amplification of one of the targets.

On the other hand, the negative controls for the 4 targets did not show amplification, which again indicates that there had been no contamination in the qPCR amplification process, and there were no interactions between the probes which may result in false positives.

Example 3 of the present invention relates to a real-time PCR kit indicated for the detection of herpes simplex virus 1, herpes simplex virus 2 and varicella zoster virus. Like in Example 2, this example again proves the usefulness of the present invention for carrying out the simultaneous detection, quantification and identification of three different pathogens and a plasma analyte in one and the same biological sample using short probes. Again, the Cts obtained for herpes simplex virus 1, herpes simplex virus 2, varicella zoster virus and Plasmid DNA *Bglob* gene were those expected (detection limit of 10 to 100 copies of DNA and amplification efficiency between 75-106.8%) at the concentrations and number of copies used in this example, which indicated the inexistence, a *priori,* of interferences between the primers and probes that may partially inhibit the simultaneous amplification of one of the targets.

On the other hand, the negative controls for the 4 targets did not show amplification, which again indicates that there had been no contamination in the qPCR amplification process, and there were no interactions between the probes which may result in false positives.

Additionally, like in Example 1, in the case of the kits set forth in Examples 2 and 3 the simultaneous use of the pairs of primers and short probes did not reduce the specificity of the method compared with the singleplex format.

Example 4 of the present invention relates to a real-time PCR kit indicated for the simultaneous detection of, on one hand, S. *pneumoniae, S. pyogenes* and the endogenous *Bglo* gene, and on the other, *Haemophilus influenzae, Staphylococcus aureus, Kingella kingae* and the endogenous *Bglo* gene. Like in Examples 2 and 3, this example again proves the usefulness of the present invention for carrying out the simultaneous detection, quantification and identification of up to three different pathogens and a plasma analyte in one and the same biological sample using short probes. Again, the Cts obtained for, on one hand, *S*. *pneumoniae, S. pyogenes* and the endogenous *Bglo* gene, and on the other, *Haemophilus influenzae, Staphylococcus aureus, Kingella kingae* and the endogenous *Bglo* gene, were those expected (detection limit of 10 copies of DNA and amplification efficiency between 86-94%) at the concentrations and number of copies used in this example, which indicated the inexistence, a *priori,* of interferences between the primers and probes that may partially inhibit the simultaneous amplification of one of the targets.

On the other hand, the negative controls for the six targets did not show amplification, which again indicates that there had been no contamination in the qPCR amplification process, and there were no interactions between the probes which may result in false positives.

Example 5 of the present invention relates to a real-time PCR kit indicated for the detection of *Candida albicans, C. parapsilopsis, C. glabrata, C. krusei, C. tropicalis, C. guilliermondii, Aspergillus flavus, A. fumigatus* and the endogenous *Bglo* gene. Like in Examples 2, 3 and 4, this example again proves the usefulness of the present invention for carrying out the simultaneous detection, quantification and identification of up to three different pathogens and a plasma analyte in one and the same biological sample using short probes. Again, the Cts obtained for *Candida albicans, C. parapsilopsis, C. glabrata, C. krusei, C. tropicalis, C. guilliermondii, Aspergillus flavus, A. fumigatus* and the endogenous *Bglo* gene were those expected (detection limit of 10 copies of DNA and amplification efficiency between 83.2-101.9%) at the concentrations and number of copies used in this example, which indicated the inexistence, a *priori,* of interferences between the primers and probes that may partially inhibit the simultaneous amplification of one of the targets.

On the other hand, the negative controls for the ten targets did not show amplification, which again indicates that there had been no contamination in the qPCR amplification process, and there were no interactions between the probes which may result in false positives.

Example 6 of the present invention relates to a real-time PCR kit indicated for the detection of VHS-8, herpesvirus 4 (Epstein Barr), cytomegalovirus, adenovirus and the endogenous *Bglo* gene. Like in Examples 2, 3, 4 and 5, this example again proves the usefulness of the present invention for carrying out the simultaneous detection, quantification and identification of up to three different pathogens and a plasma analyte in one and the same biological sample using short probes. Again, the Cts obtained for VHS-8, herpesvirus 4 (Epstein Barr), cytomegalovirus, adenovirus and the endogenous *Bglo* gene were those expected (detection limit of 10-100 copies of DNA and amplification efficiency between 86.2-100%) at the concentrations and number of copies used in this example, which indicated the inexistence, a *priori,* of interferences between the primers and probes that may partially inhibit the simultaneous amplification of one of the targets.

On the other hand, the negative controls for the six targets did not show amplification, which again indicates that there had been no contamination in the qPCR amplification process, and there were no interactions between the probes which may result in false positives.

Example 7 of the present invention relates to a real-time PCR kit indicated for the detection of *Bordetella pertussis, B. parapertussis, B. holmesii* and the endogenous *Bglo* gene. Like in Examples 2-6, this example again proves the usefulness of the present invention for carrying out the simultaneous detection, quantification and identification of up to three different pathogens and a plasma analyte in one and the same biological sample using short probes. Again, the Cts obtained for *Bordetella pertussis, B. parapertussis, B. holmesii* and the endogenous *Bglo* gene were those expected (detection limit of 10 copies of DNA and amplification efficiency between 87-99.2%) at the concentrations and number of copies used in this example, which indicated the inexistence, a *priori,* of interferences between the primers and probes that may partially inhibit the simultaneous amplification of one of the targets.

On the other hand, the negative controls for the three targets did not show amplification, which again indicates that there had been no contamination in the qPCR amplification process, and there were no interactions between the probes which may result in false positives.

Example 8 of the present invention relates to a real-time PCR kit indicated for the simultaneous detection of, on one hand, *Mycoplasma pneumoniae, Coxiella burnetti, Chlamydia pneumoniae* and the endogenous *Bglo* gene, and on the other, *Legionella pneumophila, Pneumocystis jirovecii* and the endogenous *Bglo* gene. Like in Examples 2-7, this example again proves the usefulness of the present invention for carrying out the simultaneous detection, quantification and identification of up to three different pathogens and a plasma analyte in one and the same biological sample using short probes. Again, the Cts obtained for, on one hand, *Mycoplasma pneumoniae, Coxiella burnetti, Chlamydia pneumoniae* and the endogenous *Bglo* gene, and on the other, *Legionella pneumophila, Pneumocystis jirovecii* and the endogenous *Bglo* gene, were those expected (detection limit of 10 copies of DNA and amplification efficiency between 75%-102.3%) at the concentrations and number of copies used in this example, which indicated the inexistence, a *priori,* of interferences between the primers and probes that may partially inhibit the simultaneous amplification of one of the targets.

On the other hand, the negative controls for the five targets did not show amplification, which again indicates that there had been no contamination in the qPCR amplification process, and there were no interactions between the probes which may result in false positives.

Example 9 of the present invention relates to a real-time PCR kit indicated for the simultaneous detection of, on one hand, *Giardia lamblia* B, *Dientamoeba fragilis, Crystosporidium parvum*/*hominis* and *Giardia lamblia* A in stools and, on the other, *Plasmodiun falciparum* and *Plasmodium ovale* in blood on one hand, and *Plasmodium vivax* on the other. Like in Examples 2-8, this example again proves the usefulness of the present invention for carrying out the simultaneous detection, quantification and identification of up to seven different pathogens in one and the same biological sample using short probes. Again, the Cts obtained for, on one hand, *Giardia lamblia* B, *Dientamoeba fragilis, Crystosporidium parvum*/*hominis* and *Giardia lamblia* A in stools and, on the other, *Plasmodiun falciparum* and *Plasmodium ovale* in blood on one hand, and *Plasmodium vivax* on the other, were those expected (detection limit of 10 to 100 copies of DNA and amplification efficiency between 70-101.9%) at the concentrations and number of copies used in this example, which indicated the inexistence, a *priori,* of interferences between the primers and probes that may partially inhibit the simultaneous amplification of one of the targets.

On the other hand, the negative controls for the seven targets did not show amplification, which again indicates that there had been no contamination in the qPCR amplification process, and there were no interactions between the probes which may result in false positives.

Example 10 of the present invention relates to a real-time PCR kit indicated for the detection of *M. tuberculosis* complex MT Complex-VK and the endogenous *Bglo* gene using two M. *tuberculosis* complex targets, i.e., whiB3 and pstS11. Like in Examples 2-9, this example again proves the usefulness of the present invention for carrying out the simultaneous detection, quantification and identification of up to two different targets and a plasma analyte in one and the same biological sample using short probes. Again, the Cts obtained for M. *tuberculosis* complex whiB3 and pstS11 targets and the endogenous *Bglo* gene were those expected (detection limit of 10 copies of DNA and amplification efficiency between 97.9-98.7%) at the concentrations and number of copies used in this example, which indicated the inexistence, a *priori,* of interferences between the primers and probes that may partially inhibit the simultaneous amplification of one of the targets.

On the other hand, the negative controls for the two targets did not show amplification, which again indicates that there had been no contamination in the qPCR amplification process, and there were no interactions between the probes which may result in false positives.

These results, as a whole, are certainly unexpected and they prove the until now unknown possibility of using the PCR technique for simultaneously detecting several pathogens present in one and the same sample using short probes between 8 and 9 nucleotides. In this sense, it must be pointed out that the present invention represents a progress as it allows efficiently amplifying at least four target genes (from pathogens and/or Bglob) simultaneously using short probes between 8 and 9 nucleotides (many being shared probes), representing a competitive advantage in reducing the cost of analysis, a significant reduction in assay time and using one and the same mixture for the detection assay. In addition to achieving improved sensitivity, false positives are kept at bay at all times since the sample preparation has allowed to obtain better results because it has been adapted such that there are factors which may negatively influence amplification.

The method provided by this invention allows simultaneously detecting, identifying and quantifying several pathogens that can be transmitted by any contaminated media, surfaces or environments, such as *E. coli, S. agalactiae, L. monocytogenes,* varicella zoster virus, *Mycobacterium tuberculosis* complex, herpes simplex virus 1, herpes simplex virus 2, *Streptococcus pneumoniae, S. pyogenes, Haemophilus influenzae, Staphylococcus aureus, Kingella kingae, Candida albicans, C. parapsilopsis, C. glabrata, C. krusei, C. tropicalis, C. guilliermondii, Aspergillus flavus, A. fumigatus,* VHS-8 (human herpesvirus 8), herpesvirus 4 (Epstein Barr), cytomegalovirus, adenovirus, *Bordetella pertussis, B. parapertussis, B. holmesii, Mycoplasma pneumoniae, Coxiella burnetti, Chlamydia pneumoniae, Legionella pneumophila, Pneumocystis jirovecii, Dientamoeba fragilis, Giardia lamblia A, Giardia lamblia* B, *Crystosporidium parvum*/*hominis, Plasmodiun falciparum, P. ovale* and/or *P. vivax,* in one and the same test sample by means of multiplex amplification reaction using real-time polymerase chain reaction. The test sample can be any sample containing DNA in which the possible contamination or infection by said pathogens is to be known. In a particular embodiment, said test sample is a biological sample from a subject, preferably human, or a sample from a food product, for example, meat and dairy products, or a sample from contaminated surfaces or environments.

The oligonucleotides of the invention were designed for the purpose of identifying specifically *Escherichia coli, Streptococcus agalactiae, Listeria monocytogenes,* varicella zoster virus, *Mycobacterium tuberculosis* complex, herpes simplex virus 1, herpes simplex virus 2, *Streptococcus pneumoniae, S. pyogenes, Haemophilus influenzae, Staphylococcus aureus, Kingella kingae, Candida albicans, C. parapsilopsis, C. glabrata, C. krusei, C. tropicalis, C. guilliermondii, Aspergillus flavus, A. fumigatus,* VHS-8 (human herpesvirus 8), herpesvirus 4 (Epstein Barr), cytomegalovirus, adenovirus, *Bordetella pertussis, B. parapertussis, B. holmesii, Mycoplasma pneumoniae, Coxiella burnetti, Chlamydia pneumoniae, Legionella pneumophila, Pneumocystis jirovecii, Dientamoeba fragilis, Giardia lamblia A, Giardia lamblia* B, *Crystosporidium parvum*/*hominis, Plasmodiun falciparum, P. ovale* and/or *P. vivax* that may be present in a test sample without giving false positives due to the presence of other pathogens present therein.

There is provided, for each of the pathogens to be identified, a pair of oligonucleotide primers as well as a short probe, such that each pair of primers comprises a first "upstream" synthetic nucleotide sequence complementary to an "upstream" nucleotide sequence binding with the 5' end of a target nucleic acid sequence and a second "downstream" synthetic nucleotide sequence complementary to a "downstream" sequence binding with the 3' end of the target nucleic acid sequence. The nucleotide sequences must have each pair of oligonucleotide primers specific to the pathogen to be detected, such that they do not react or crosslink with other pathogens. Likewise, a specific short probe was developed for each of the pathogens to be identified.

Therefore, the first aspect of the present invention provides a new PCR technique-based method for simultaneously detecting several pathogens present in one and the same sample, preferably in a biological sample from a human being. Particularly, this aspect of the present invention relates to a method for the detection, quantification and identification of pathogens of two or more different strains or species of microorganisms in one and the same sample, preferably biological sample, more preferably isolated from a human subject, through polymerase chain reaction (PCR), preferably performed in a quantitative manner in real time, which comprises detecting, identifying and quantifying the presence or absence of said microorganisms in the biological sample through the determination and quantification of the presence or absence of amplicons from the polymerase chain reaction (PCR), preferably performed in a quantitative manner in real time;
wherein said polymerase chain reaction (PCR) uses DNA, complementary DNA (cDNA) or single-stranded DNA obtained by the reverse transcription of ribonucleic acid (RNA) of the biological sample, at least one pair of oligonucleotide initiators or primers for each of the pathogens to be identified as well as at least one labeled probe for each of the pathogens to be identified, dNTPs, a suitable reaction buffer and a heat-stable DNA polymerase, wherein each of said labeled probes consists of a fluorophore-labeled oligonucleotide with a length of 8 to 9 nucleotides, and wherein each of said probes is labeled with a different fluorophore.

In a preferred embodiment of the first aspect of the invention, said method determines the detection, quantification and identification of three or more different strains or species of microorganisms in one and the same biological sample from a subject and said polymerase chain reaction (PCR) uses at least three pairs of specific primers and at least three fluorescently labeled probes, wherein each of said fluorescently labeled probes consists of a fluorophore-labeled oligonucleotide with a length of 8 to 9 nucleotides, and wherein each of said probes is labeled with a different fluorophore.

In another preferred embodiment of the first aspect of the invention, said method determines the detection, quantification and identification of four or more different strains or species of microorganisms in one and the same biological sample from a subject and said polymerase chain reaction (PCR) uses at least four pairs of specific primers and at least four fluorescently labeled probes, wherein each of said fluorescently labeled probes consists of a fluorophore-labeled oligonucleotide with a length of 8 to 9 nucleotides, and wherein each of said probes is labeled with a different fluorophore.

In another preferred embodiment of the first aspect of the invention, said method determines the detection, quantification and identification of five or more different strains or species of microorganisms in one and the same biological sample from a subject and said polymerase chain reaction (PCR) uses at least five pairs of specific primers and at least four fluorescently labeled probes, wherein each of said fluorescently labeled probes consists of a fluorophore-labeled oligonucleotide with a length of 8 to 9 nucleotides, and wherein each of said probes is labeled with a different fluorophore. In another preferred embodiment of the first aspect of the invention, said method determines the detection, quantification and identification of six or more different strains or species of microorganisms in one and the same biological sample from a subject and said polymerase chain reaction (PCR) uses at least six pairs of specific primers and at least four fluorescently labeled probes, wherein each of said fluorescently labeled probes consists of a fluorophore-labeled oligonucleotide with a length of 8 to 9 nucleotides, and wherein each of said probes is labeled with a different fluorophore.

In another preferred embodiment of the first aspect of the invention, said method determines the detection, quantification and identification of seven or more different strains or species of microorganisms in one and the same biological sample from a subject and said polymerase chain reaction (PCR) uses at least seven pairs of specific primers and at least four fluorescently labeled probes, wherein each of said fluorescently labeled probes consists of a fluorophore-labeled oligonucleotide with a length of 8 to 9 nucleotides, and wherein each of said probes is labeled with a different fluorophore.

In another preferred embodiment of the first aspect of the invention, said method determines the detection, quantification and identification of eight or more different strains or species of microorganisms in one and the same biological sample from a subject and said polymerase chain reaction (PCR) uses at least eight pairs of specific primers and at least four fluorescently labeled probes, wherein each of said fluorescently labeled probes consists of a fluorophore-labeled oligonucleotide with a length of 8 to 9 nucleotides, and wherein each of said probes is labeled with a different fluorophore.

In yet another preferred embodiment of the first aspect of the invention, said probes, which may or may not be Taqman-type probes, consist of synthetic oligonucleotides, preferably LNA (Locked Nucleic Acids)-type synthetic oligonucleotides. Additionally, said probes preferably consist of an oligonucleotide with a length of 8 nucleotides. More preferably, said probes are capable of amplifying amplicons with a length between 60 and 120 nucleotides, preferably amplicons with a length between 70 and 110 nucleotides.

In yet another preferred embodiment of the first aspect of the invention, the pathogen is selected from the list consisting of *Escherichia coli, Streptococcus agalactiae, Listeria monocytogenes,* varicella zoster virus, *Mycobacterium tuberculosis* complex, herpes simplex virus 1, herpes simplex virus 2, *Streptococcus pneumoniae, S. pyogenes, Haemophilus influenzae, Staphylococcus aureus, Kingella kingae, Candida albicans, C. parapsilopsis, C. glabrata, C. krusei, C. tropicalis, C. guilliermondii, Aspergillus flavus, A. fumigatus,* VHS-8 (human herpesvirus 8), herpesvirus 4 (Epstein Barr), cytomegalovirus, adenovirus, *Bordetella pertussis, B. parapertussis, B. holmesii, Mycoplasma pneumoniae, Coxiella burnetti, Chlamydia pneumoniae, Legionella pneumophila, Pneumocystis jirovecii, Dientamoeba fragilis, Giardia lamblia* A, *Giardia lamblia* B, *Crystosporidium parvum*/*hominis, Plasmodiun falciparum, P. ovale* and *P. vivax.*

In another preferred embodiment of the first aspect of the invention, the determination of the presence or absence of the microorganisms, treatment is carried out on the reaction mixture which will be used for carrying out polymerase chain reaction (PCR) performed in a quantitative manner in real time with uracil-DNA-glycosylase (UNG), preferably with the UNG of Atlantic cod (*Gadus morhua*).

In a second aspect of the invention, the method of the first aspect of the invention is for obtaining data useful for the diagnosis of a pathology caused by a microorganism based on the presence or absence of said microorganism in a biological sample from a subject.

In a particular embodiment of the first or second aspect of the invention, the microorganisms are herpes simplex virus 1, herpes simplex virus 2 and/or varicella zoster virus and said method is characterized by comprising probes 63, 157 and 142. Preferably, this method is useful for obtaining data useful for the diagnosis of viral meningitis.

In another particular embodiment of the first or second aspect of the invention, said microorganisms are *Escherichia coli, Streptococcus agalactiae* and/or *Listeria monocytogenes* and said method is characterized by comprising probes 9, 56 and 142. Preferably, this method is useful for obtaining data useful for the diagnosis of neonatal sepsis.

In another particular embodiment of the first or second aspect of the invention, said microorganisms are M. *(Mycobacterium) tuberculosis, M. bovis, M. africanum, M. microtti, M. caprae, M. pinnipedii* and/or *M.canetti.* Preferably, this method is useful for obtaining data useful for the diagnosis of tuberculosis.

In another particular embodiment of the first or second aspect of the invention, said microorganisms are *Streptococcus pneumoniae, S. pyogenes, Haemophilus influenzae, Staphylococcus aureus* and *Kingella kingae* and said method is characterized by comprising probes 129, 38, 110 and 84. Preferably, this method is useful for obtaining data useful for the diagnosis of infectious arthritis.

In another particular embodiment of the first or second aspect of the invention, said microorganisms are *Candida albicans, C. parapsilopsis, C. glabrata, C. krusei, C. tropicalis, C. guilliermondii, Aspergillus flavus* and *A. fumigatus* and said method is characterized by comprising probes 131, 67, 155, 4, 147, 129, 84 and 70. Preferably, this method is useful for obtaining data useful for the diagnosis of invasive fungal infections.

In another particular embodiment of the first or second aspect of the invention, said microorganisms are VHS-8, herpesvirus 4 (Epstein Barr), cytomegalovirus and adenovirus and said method is characterized by comprising probes 150, 40 and 108. Preferably, this method is useful for obtaining data useful for the diagnosis of viral infections in immunosuppressed individuals.

In another particular embodiment of the first or second aspect of the invention, said microorganisms are *Bordetella pertussis, B. parapertussis* and *B. holmesii* and said method is characterized by comprising probes 86, 40 and 81. Preferably, this method is useful for obtaining data useful for the diagnosis of whooping cough.

In another particular embodiment of the first or second aspect of the invention, said microorganisms are *Mycoplasma pneumoniae, Coxiella burnetti, Chlamydia pneumoniae, Legionella pneumophila* and *Pneumocystis jirovecii* and said method is characterized by comprising probes 150, 59, 67, 60 and 141. Preferably, this method is useful for obtaining data useful for the diagnosis of atypical pneumoniae.

In another particular embodiment of the first or second aspect of the invention, said microorganisms are *Dientamoeba fragilis, Giardia lamblia A, Giardia lamblia* B, *Crystosporidium parvum*/*hominis, Plasmodiun falciparum, P. ovale* and *P. vivax* and said method is characterized by comprising probes 129, 6, 70, 4, 67, 119 and 88. Preferably, this method is useful for obtaining data useful for the diagnosis of the presence of parasites in blood and/or stools.

In another particular embodiment of the first or second aspect of the invention, said microorganism is M. *(Mycobacterium) tuberculosis* complex and said method is characterized by comprising probes 152 and 70. Preferably, this method is useful for obtaining data useful for the diagnosis of tuberculosis. A third aspect of the invention relates to a kit or device suitable for the detection, quantification and identification of pathogens of two, three, four or more, such as five, six, seven, eight or more, different strains or species of microorganisms in one and the same sample, comprising at least two, three or four, such as five, six, seven eight or more, pairs of oligonucleotide primers for each of the pathogens to be identified as well as at least two, three or four labeled probes for each of the pathogens to be identified;
wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

A fourth aspect of the invention relates to a kit or device comprising at least three fluorescently labeled probes, wherein each of said fluorescently labeled probes consists of a fluorophore-labeled oligonucleotide with a length of 8 to 9 nucleotides selected from the list consisting of probe 63, probe 157 and probe 142, and wherein each of said probes is labeled with a different fluorophore. Preferably, this kit is used for the detection, quantification and identification of pathogens in a sample consisting of herpes simplex virus 1, herpes simplex virus 2 and varicella zoster virus, more preferably for the diagnosis of viral meningitis.

A fifth aspect of the invention relates to a kit or device comprising at least three fluorescently labeled probes, wherein each of said fluorescently labeled probes consists of a fluorophore-labeled oligonucleotide with a length of 8 to 9 nucleotides selected from the list consisting of probe 9, probe 56 and probe 142, and wherein each of said probes is labeled with a different fluorophore. Preferably, this kit is used for the detection, quantification and identification of pathogens in a sample consisting of *Escherichia coli, Streptococcus agalactiae and Listeria monocytogenes,* more preferably for the diagnosis of neonatal sepsis.

A sixth aspect of the invention relates to a kit or device comprising at least four fluorescently labeled probes, wherein each of said fluorescently labeled probes consists of a fluorophore-labeled oligonucleotide with a length of 8 to 9 nucleotides selected from the list consisting of probe 129, probe 38, probe 110 and probe 84, and wherein each of said probes is labeled with a different fluorophore. Preferably, this kit is used for the detection, quantification and identification of pathogens in a sample consisting of *Streptococcus pneumoniae, S. pyogenes, Haemophilus influenzae, Staphylococcus aureus* and *Kingella kingae,* more preferably for the diagnosis of infectious arthritis.

In a preferred embodiment of the sixth aspect of the invention, the kit or device comprises at least two mixes. Mix 1 comprises at least two fluorescently labeled probes, wherein each of said fluorescently labeled probes consists of a fluorophore-labeled oligonucleotide with a length of 8 to 9 nucleotides selected from the list consisting of probe 129 and probe 38. Preferably, this kit is used for the detection, quantification and identification of pathogens in a sample consisting of *Streptococcus pneumoniae* and *S*. *pyogenes.* Mix 2 comprises at least three fluorescently labeled probes, wherein each of said fluorescently labeled probes consists of a fluorophore-labeled oligonucleotide with a length of 8 to 9 nucleotides selected from the list consisting of probe 84, probe 129 and probe 110. Preferably, this kit is used for the detection, quantification and identification of pathogens in a sample consisting of *Haemophilus influenzae, Staphylococcus aureus* and *Kingella kingae.*

A seventh aspect of the invention relates to a kit or device comprising at least eight fluorescently labeled probes, wherein each of said fluorescently labeled probes consists of a fluorophore-labeled oligonucleotide with a length of 8 to 9 nucleotides selected from the list consisting of probe 131, probe 67, probe 155, probe 147, probe 129, probe 4, probe 84 and probe 70, and wherein each of said probes is labeled with a different fluorophore. Preferably, this kit is used for the detection, quantification and identification of pathogens in a sample consisting of *Candida albicans, C. parapsilopsis, C. glabrata, C. krusei, C. tropicalis, C. guilliermondii, Aspergillus flavus* and *A. fumigatus,* more preferably for the diagnosis of invasive fungal infections.

In a preferred embodiment of the seventh aspect of the invention, the kit or device comprises at least four mixes. Mix 1 comprises at least one fluorescently labeled probe, wherein said fluorescently labeled probe consists of a fluorophore-labeled oligonucleotide with a length of 8 to 9 nucleotides which is probe 131. Preferably, this kit is used for the detection, quantification and identification of pathogens in a sample consisting of *Candida albicans.* Mix 2.1 comprises at least two fluorescently labeled probes, wherein each of said fluorescently labeled probes consists of a fluorophore-labeled oligonucleotide with a length of 8 to 9 nucleotides selected from the list consisting of probe 67 and probe 155. Preferably, this kit is used for the detection, quantification and identification of pathogens in a sample consisting of *C*. *parapsilopsis,* and *C*. *glabrata.* Mix 2.2 comprises at least three fluorescently labeled probes, wherein each of said fluorescently labeled probes consists of a fluorophore-labeled oligonucleotide with a length of 8 to 9 nucleotides selected from the list consisting of probe 147, probe 129 and probe 4. Preferably, this kit is used for the detection, quantification and identification of pathogens in a sample consisting of *C*. *tropicalis, C. guilliermondii* and *C*. *krusei.* Mix 3 comprises at least two fluorescently labeled probes, wherein each of said fluorescently labeled probes consists of a fluorophore-labeled oligonucleotide with a length of 8 to 9 nucleotides selected from the list consisting of probe 84 and probe 70. Preferably, this kit is used for the detection, quantification and identification of pathogens in a sample consisting of *Aspergillus flavus* and *A. fumigatus.*

An eighth aspect of the invention relates to a kit or device comprising at least three fluorescently labeled probes, wherein each of said fluorescently labeled probes consists of a fluorophore-labeled oligonucleotide with a length of 8 to 9 nucleotides selected from the list consisting of probe 150, probe 40 and probe 108, and wherein each of said probes is labeled with a different fluorophore. Preferably, this kit is used for the detection, quantification and identification of pathogens in a sample consisting of VHS-8, herpesvirus 4 (Epstein Barr), cytomegalovirus and adenovirus, more preferably for the diagnosis of viral infections in immunosuppressed individuals.

In a preferred embodiment of the eighth aspect of the invention, the kit or device comprises at least two mixes. Mix 1 comprises at least two fluorescently labeled probes, wherein each of said fluorescently labeled probes consists of a fluorophore-labeled oligonucleotide with a length of 8 to 9 nucleotides selected from the list consisting of probe 150 and probe 40. Preferably, this kit is used for the detection, quantification and identification of pathogens in a sample consisting of VHS-8 and herpesvirus 4 (Epstein Barr). Mix 2 comprises at least two fluorescently labeled probes, wherein each of said fluorescently labeled probes consists of a fluorophore-labeled oligonucleotide with a length of 8 to 9 nucleotides selected from the list consisting of the probe 108 and probe 40. Preferably, this kit is used for the detection, quantification and identification of pathogens in a sample consisting of adenovirus and cytomegalovirus.

A ninth aspect of the invention relates to a kit or device comprising at least three fluorescently labeled probes, wherein each of said fluorescently labeled probes consists of a fluorophore-labeled oligonucleotide with a length of 8 to 9 nucleotides selected from the list consisting of probe 86, probe 40 and probe 81, and wherein each of said probes is labeled with a different fluorophore. Preferably, this kit is used for the detection, quantification and identification of pathogens in a sample consisting of *Bordetella pertussis, B. parapertussis* and *B. holmesii,* more preferably for the diagnosis of whooping cough.

A tenth aspect of the invention relates to a kit or device comprising at least three fluorescently labeled probes, wherein each of said fluorescently labeled probes consists of a fluorophore-labeled oligonucleotide with a length of 8 to 9 nucleotides selected from the list consisting of probe 150, probe 59, probe 67, probe 60 and probe 141, and wherein each of said probes is labeled with a different fluorophore. Preferably, this kit is used for the detection, quantification and identification of pathogens in a sample consisting of *Mycoplasma pneumoniae, Coxiella burnetti, Chlamydia pneumoniae, Legionella pneumophila* and *Pneumocystis jirovecii,* more preferably for the diagnosis of atypical pneumoniae.

In a preferred embodiment of the tenth aspect of the invention, the kit or device comprises at least two mixes. Mix 1 comprises at least three fluorescently labeled probes, wherein each of said fluorescently labeled probes consists of a fluorophore-labeled oligonucleotide with a length of 8 to 9 nucleotides selected from the list consisting of probe 150, probe 59 and probe 67. Preferably, this kit is used for the detection, quantification and identification of pathogens in a sample consisting of *Mycoplasma pneumoniae, Coxiella burnetti* and *Chlamydia pneumoniae.* Mix 2 comprises at least two fluorescently labeled probes, wherein each of said fluorescently labeled probes consists of a fluorophore-labeled oligonucleotide with a length of 8 to 9 nucleotides selected from the list consisting of probe 60 and probe 141. Preferably, this kit is used for the detection, quantification and identification of pathogens in a sample consisting of *Legionella pneumophila* and *Pneumocystis jirovecii.*

An eleventh aspect of the invention relates to a kit or device comprising at least seven fluorescently labeled probes, wherein each of said fluorescently labeled probes consists of a fluorophore-labeled oligonucleotide with a length of 8 to 9 nucleotides selected from the list consisting of probe 129, probe 6, probe 67, probe 70, probe 4, probe 119 and probe 88, and wherein each of said probes is labeled with a different fluorophore. Preferably, this kit is used for the detection, quantification and identification of pathogens in a sample consisting of *Dientamoeba fragilis, Giardia lamblia A, Giardia lamblia* B, *Crystosporidium parvum*/*hominis, Plasmodiun falciparum, P. ovale* and *P. vivax,* more preferably for the diagnosis of the presence of parasites both in stools and in blood.

In a preferred embodiment of the eleventh aspect of the invention, the kit or device comprises at least three mixes. The mix used for the diagnosis of parasites in stools comprises at least four fluorescently labeled probes, wherein each of said fluorescently labeled probes consists of a fluorophore-labeled oligonucleotide with a length of 8 to 9 nucleotides selected from the list consisting of probe 70, probe 129, probe 4 and probe 6. Preferably, this kit is used for the detection, quantification and identification of pathogens in a sample consisting of *Dientamoeba fragilis, Giardia lamblia A, Giardia lamblia* B and *Crystosporidium parvum*/*hominis.* Mix 1, used for the diagnosis of parasites in blood, comprises at least two fluorescently labeled probes, wherein each of said fluorescently labeled probes consists of a fluorophore-labeled oligonucleotide with a length of 8 to 9 nucleotides selected from the list consisting of probe 67 and probe 119. Preferably, this kit is used for the detection, quantification and identification of pathogens in a sample consisting of *Plasmodiun falciparum* and *P. ovale.* Mix 2, used for the diagnosis of parasites in blood, comprises at least one fluorescently labeled probe, wherein said fluorescently labeled probe consists of a fluorophore-labeled oligonucleotide with a length of 8 to 9 nucleotides, wherein said probe consists of probe 88. Preferably, this kit is used for the detection, quantification and identification of pathogens in a sample consisting of P. *vivax.*

A twelfth aspect of the invention relates to a kit or device comprising at least two fluorescently labeled probes, wherein each of said fluorescently labeled probes consists of a fluorophore-labeled oligonucleotide with a length of 8 to 9 nucleotides selected from the list consisting of probe 152 and probe 70, and wherein each of said probes is labeled with a different fluorophore. Preferably, this kit is used for the detection, quantification and identification in a sample of at least two M. *(Mycobacterium) tuberculosis* complex targets (preferably the whiB3 target and the pstS11 target), more preferably for the diagnosis of tuberculosis.

The kit or device of the third, fourth, fifth, sixth, seventh, eighth, ninth, tenth, eleventh and/or twelfth aspect of the invention can additionally comprise at least one of the following elements: dNTPs, a suitable reaction buffer, a heat-stable DNA polymerase, a UNG, preferably the UNG of Atlantic cod *(Gadus morhua)* and/or a positive control, preferably B-globin (the *Bglo* gene). Preferably, the kit or device of the third, fourth, fifth, sixth, seventh, eighth, ninth, tenth, eleventh and/or twelfth aspect of the invention can additionally comprise a fluorescently labeled probe, wherein said fluorescently labeled probe consist of a fluorophore-labeled oligonucleotide with a length of 8 to 9 nucleotides, wherein said probe consists of probe 144. Preferably, the kit or device of the third, fourth, fifth, sixth, seventh, eighth, ninth, tenth, eleventh and/or twelfth aspect of the invention can additionally comprise a fluorescently labeled probe, wherein said fluorescently labeled probe consists of a fluorophore-labeled oligonucleotide with a length of 8 to 9 nucleotides, wherein said probe consists of probe 6. Preferably, the kit or device of the third, fourth, fifth, sixth, seventh, eighth, ninth, tenth, eleventh and/or twelfth aspect of the invention can additionally comprise at least two fluorescently labeled probes, wherein said fluorescently labeled probes consist of a fluorophore-labeled oligonucleotide with a length of 8 to 9 nucleotides selected from the list consisting of probe 144 and probe 6, wherein each of said probes is labeled with a different fluorophore.

It goes without saying that the present invention expressly includes the use of any of the kits or devices mentioned in this specification for the detection, quantification and identification of pathogens of two, three, four or more different strains or species of microorganisms in one and the same sample, particularly in any type of sample, preferably in a biological sample from a human subject.

The present invention is illustrated by the following examples which in no case limit the invention.

### Examples

### Example 1. Kit for the detection of M. tuberculosis Complex

The present example refers to a real-time PCR kit indicated for the detection of M. *tuberculosis* complex. Particularly, this example shows the usefulness of the present invention for carrying out the detection, quantification and identification of two or more different strains or species of microorganisms in one and the same biological sample using short probes.

### 1. Assay description

To carry out the present assay, a real-time PCR kit indicated for the detection of M. *tuberculosis* complex was prepared. The kit contained the required reagents and enzymes mixed at ideal concentrations and designed to make diagnostic analysis easier. This kit used a FAM-(Roche quencher)-labeled UPL probe for the detection of M. *tuberculosis* complex. In addition, the kit provided a DNA extraction control/PCR inhibition control which allowed verifying that the extraction process has been developed correctly and detecting false negatives due to PCR inhibition. This control was detected through a short HEX-BHQ1®-labeled LNA probe (in-house design).

### 2. Singleplex assay (Comparative example)

### 2.1. Protocol validation by means of standards

### 2.1.1. Singleplex qPCR for the detection of M. tuberculosis complex

### 2.1.1.1. qPCR reaction

| Indicate no. of reactions | | 31 | 1 |
|---|---|---|---|
| | | | |

| Reagent (concentration)) | Volume (µl) | Volume (µl) | Check |
|---|---|---|---|
| Probe UPL149 (10 µm) | 15.5 | 0.5 | |
| MT 149-F (20 µM) | 23.25 | 0.75 | |
| MT 149-R (100 µM) | 23.25 | 0.75 | |
| Master Probes | 310 | 10 | |
| H20 | 86.8 | 2.8 | |
| UNG | 6.2 | 0.2 | |
| DNA | | 5 | |
| Final volume | 465 | 20 | |

UPL probe149 as well as primers MT149-F and MT149-R have the sequences provided in detail below:

| **Syndrome** | **Pathogen** | **Forward primer seq.** | **Reverse primer seq.** |
|---|---|---|---|
| **MTBC (Tuberculosis)** | TBC complex | 5'- CGTAGTAGGCAGCCTCGAGTT- 3' | 5’- GCTGGGTCGACTGGTTCA- 3’ |

| **Syndrome** | **Pathogen** | **Probe No.** | **Amplicon seq**. | **Amplicon** | **Probe seq**. |
|---|---|---|---|---|---|
| **MTBC (Tuberculo sis)** | TBC complex | 149 | CGTAGTAGGCAGCCTCGAGTT CGACCGGCGGGACGTCGCCG CAGTACTGGTAGAGGCGGCG ATGGTTGAACCAGTCGACCCA GC | 84 | GGCGGC GA |

The probe as well as primers for the Bglob gene

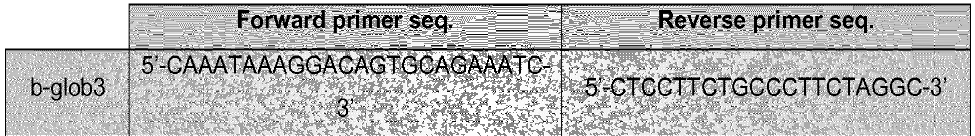

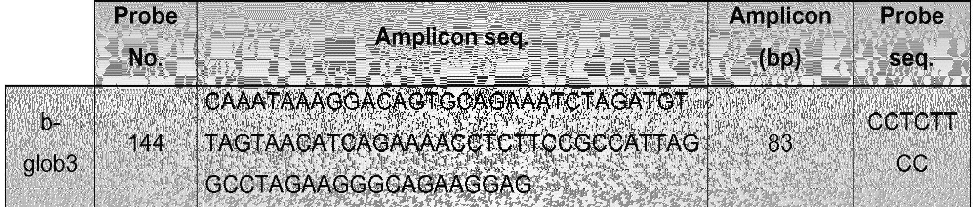

### 2.1.1.2. Thermal cycler program

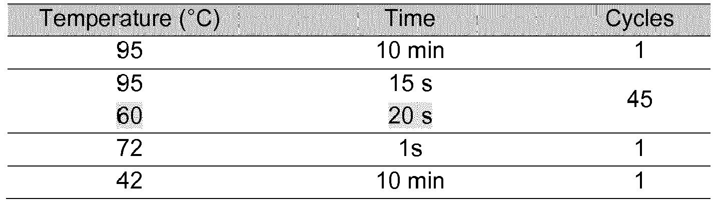

### 2.1.1.3. qPCR functionality

Verification of qPCR functionality was tested by means of analyzing the genomic DNA of *M*. *tuberculosis.* Several qPCRs with different DNA concentrations were performed.

### 2.1.1.4. Detection limit of the target and amplification efficiency

To determine the detection limit of the target, 10-fold serial dilutions of plasmid DNA (standard DNA) were tested in a range of concentration usually varying between 10⁷ copies and 1 copy per well using 2 replicates per dilution.

Calibration curve parameters were analyzed to determine qPCR amplification efficiency. Efficiency: 10 (-1/m) -1, m being the slope of the calibration line equation.

**2.1.1.4.1.1. Results**

| Standard plasmid DNA | Mean ct obtained |
|---|---|
| 10⁷ copies | 15.60 |
| 10⁶ copies | 19 |
| 10⁵ copies | 22.4 |
| 10⁴ copies | 25.97 |
| 10³ copies | 29.21 |
| 10² copies | 32.39 |
| 10 copies | 37.04 |
| 1 copy | No Ct |
| CN | No Ct |

| **Standard curve parameters obtained** | |
|---|---|
| Slope of the line | 3.45 |
| Correlation coefficient (R²) | 0.998 |
| Efficiency | 94.8% |
| Detection limit | 10 copies |

### 2.1.2. Singleplex qPCR for the detection of DNA extraction control/PCR inhibition control: Bglob gene (Comparative example)

### 2.1.2.1. qPCR reaction

| Indicate no. of reactions | | 31 | 1 |
|---|---|---|---|
| | | | |

| Reagent (concentration) | Volume (µl) | Volume (µl) | Check |
|---|---|---|---|
| Probe LNA 144 (10 µm) HEX | 15.5 | 0.5 | |
| Bglo 144F (100 µM) | 4.65 | 0.15 | |
| Bglo 144R (100 µM) | 4.65 | 0.15 | |
| Master Probes | 310 | 10 | |
| H20 | 124 | 4 | |
| UNG | 6.2 | 0.2 | |
| DNA | | 5 | |
| Final volume | 465 | 20 | |

### 2.1.2.2. Thermal cycler program

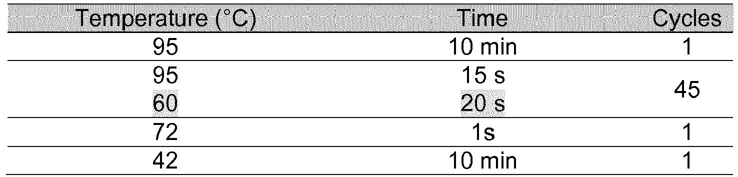

### 2.1.2.3. qPCR functionality

The verification of qPCR functionality was tested by means of analyzing plasmid DNA with a concentration of 10⁵ copies per well in which the amplified product of the target has previously been cloned. Several qPCR with different DNA concentrations were performed. The results are shown in the following section: Detection limit of the target.

### 2.1.2.4. Detection limit of the target and amplification efficiency

To determine the detection limit of the target, 10-fold serial dilutions of plasmid DNA (standard DNA) were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, m being the slope of the calibration line equation.

**2.1.2.4.1.1. Results**

| Standard plasmid DNA | Mean Ct obtained |
|---|---|
| 10⁷ copies | 16.18 |
| 10⁶ copies | 19.46 |
| 10⁵ copies | 22.87 |
| 10⁴ copies | 26.24 |
| 10³ copies | 29.70 |
| 10² copies | 33.05 |
| 10 copies | 35.83 |
| 1 copy | 38.31* |
| CN | No Ct |

| | |
|---|---|
| *Not detected in any replicates | |

| **Standard curve parameters obtained** | |
|---|---|
| Slope of the line | -3.26 |
| Correlation coefficient (R²) | 0.997 |
| Efficiency | 102.7% |
| Detection limit | 10 copies |

### 2.1.3. Multiplex qPCR for the detection of M. tuberculosis complex/Bglo gene

### 2.1.3.1. qPCR reaction

| Indicate no. of reactions | | 31 | 1 |
|---|---|---|---|
| | | | |

| Reagent (concentration)) | Volume (µl) | Volume (µl) | Check |
|---|---|---|---|
| Probe HEX 144 (10 µM) | 15.5 | 0.5 | |
| *Bglo* 144F (100 µM) | 4.65 | 0.15 | |
| *Bglo* 144R (100 µM) | 4.65 | 0.15 | |
| Probe UPL149 (10 µM) | 15.5 | 0.5 | |
| MT149-F (20 µM) | 23.25 | 0.75 | |
| MT149-R (20 µM) | 23.25 | 0.75 | |
| Master Probes | 310 | 10 | |
| H20 | 62 | 2 | |
| UNG | 6.2 | 0.2 | |
| DNA | | 5 | |
| Final volume | 465 | 20 | |

### 2.1.3.2. Thermal cycler program

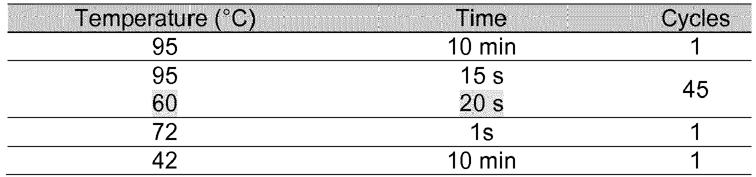

### 2.1.3.3. Multiplex qPCR functionality and interference analysis

The verification of qPCR functionality and the existence of interferences between the primers and probes in the multiplex reaction was tested by means of analyzing the positive control made up of plasmid DNA with a concentration of 10⁵ copies of the *Bglo* gene and the genomic DNA of *M. tuberculosis* (2.5 ng).

### 2.1.3.3.1.1. Results

The results of the Ct values obtained in the singleplex reaction and in the multiplex reaction are described below:

| Mean Ct obtained | Multiplex | Singleplex |
|---|---|---|
| *M. tuberculosis* | 24.41 (2.5 ng) | 30.5 (0.5 ng) |
| | | 34.3 (0.05 ng) |
| *Bglo* | 22.96 | 22.57 |
| CN | No ct | No ct |

The Cts obtained both for M. *tuberculosis* and for the *Bglo* gene are those expected (Ct of about 22-25), which, in principle, indicates that there are no interferences between the primers and probes that may partially inhibit the simultaneous amplification of one of the targets.

In addition, the fact that the negative controls for the 2 targets did not show amplification, in addition to indicating that there has not been contamination in the qPCR amplification process, again shows that there are no interactions between the probes which may result in false positives.

Furthermore, the Cts obtained for the detection of M. *tuberculosis* and the Bglob gene in multiplex and singleplex format are maintained.

### 2.1.3.4. Detection limit of the target and multiplex amplification efficiency

To determine the detection limit of the target, 10-fold serial dilutions of plasmid DNA (standard DNA) were tested in a range of concentration usually varying between 10⁷ copies and 1 copy per well using 2 replicates per dilution.

Calibration curve parameters were analyzed to determine qPCR amplification efficiency. Efficiency: 10(-1/m) -1, m being the slope of the calibration line equation.

### 2.1.3.4.1.1. Results

**2.1.3.4.1.1.1. Mycobacterium tuberculosis complex (probe 149)**

| | Singleplex | Multiplex |
|---|---|---|
| 10⁷ copies | 15.60 | 15.56 |
| 10⁶ copies | 19 | 18.92 |
| 10⁵ copies | 22.4 | 22.41 |
| 10⁴ copies | 25.97 | 25.88 |
| 10³ copies | 29.21 | 29.19 |
| 10² copies | 32.39 | 32.35 |
| 10 copies | 37.04 | 37.19 |
| 1 copy | No Ct | 38.57* |
| CN | No Ct | No Ct |

| **Standard curve parameters** | **Singloplex** | **Multiplex** |
|---|---|---|
| Slope of the line | -3.45 | -3.36 |
| Correlation coefficient (R²) | 0.998 | 0.996 |
| Efficiency | 94.8% | 98.4% |
| Detection limit | 10 copies | 10 copies |

| | | |
|---|---|---|
| *Not detected in any replicates | | |

**2.1.3.4.1.1.2. Bglo gene (probe 144)**

| | Singleplex | Multiplex |
|---|---|---|
| 10⁷ copies | 16.18 | 16.08 |
| 10⁶ copies | 19.46 | 19.56 |
| 10⁵ copies | 22.87 | 22.68 |
| 10⁴ copies | 26.24 | 26.05 |
| 10³ copies | 29.70 | 29.47 |
| 10² copies | 33.05 | 32.54 |
| 10 copies | 35.83 | 35.79 |
| 1 copy | 38.31* | No Ct |
| CN | No Ct | No Ct |

| **Standard curve parameters** | **Singleplex** | **Multiplex** |
|---|---|---|
| Slope of the line | -3.26 | -3.28 |
| Correlation coefficient (R²) | 0.997 | 1 |
| Efficiency | 102.7% | 101.5% |
| Detection limit | 10 copies | 10 copies |

### Example 2. Kit for the detection of Escherichia coli, Streptococcus agalactiae and Listeria monocytogenes

The present example refers to a real-time PCR kit indicated for the detection of *Escherichia coli, Streptococcus agalactiae, Listeria monocytogenes* and the *Bglo* gene. Particularly, this example shows the usefulness of the present invention for carrying out the detection, quantification and identification of four or more different strains or species of microorganisms in one and the same biological sample using short probes.

### 1. Assay description

To carry out the present assay, a real-time PCR kit indicated for the simultaneous detection of *Escherichia coli, Streptococcus agalactiae, Listeria monocytogenes* and the *Bglo* gene as a DNA extraction control/PCR inhibition control is prepared. This control allows verifying that the extraction process has been developed correctly and detecting false negatives due to PCR inhibition.

The kit contains the required reagents and enzymes mixed at ideal concentrations and designed to make diagnostic analysis easier.

Each Master Mix provides a HEX-labeled UPL probe for the detection of *Escherichia coli,* a FAM-labeled UPL probe for the detection of *Streptococcus agalactiae,* a TexTM615-labeled UPL probe for the detection of *Listeria monocytogenes* and a Cy5-BHQ3®-labeled LNA probe for the detection of the *Bglo* gene (DNA extraction control/PCR inhibition control gene). The channels through which fluorescence-related data is collected are described in detail in the following table.

**Table 1: Reporters and Quenchers**

| | Reporter | Quencher |
|---|---|---|
| *Escherichia coli* (probe 9) | HEX/JOE | Exiqon |
| *Streptococcus agalactiae* (probe 56) | FAM | Roche |
| *Listeria monocytogenes* (probe 142) | ROX* | Exiqon |
| *Bglo* gene (probe 144) | Cy5 | BHQ3® |

| | | |
|---|---|---|
| *The fluorophores ROX and Tex™615 have the same emission spectra | | |

The primers and probes used in this assay in particular are described below:

| **Syndrome** | **Pathogen** | **Forward primer seq**. **(F)** | **Reverse primer seq. (R)** | | |
|---|---|---|---|---|---|
| ***Neonatal sepsis*** | *Escherichia coli* | 5'- AACCACGCATCTGTTGAC TG-3' | 5’- ATCCGCATCACGCAGTTC- 3’ | | |
| ***Neonatal sepsis*** | *Streptococcus agalactiae* | 5’- TCAGTCGCAAGTGTTCAA GC-3’ | 5'- TCAGCCTTTACCTCTGAA ACAGT-3' | | |
| ***Neonatal sepsis*** | *Listeria monocytogenes* | 5’- CGGAGATGCAGTGACAA ATG-3’ | 5’- TTCTTCTTTTTCTCCACAA CGAT-3’ | | |

| **Syndrome** | **Pathogen** | **Probe No.** | **Amplicon seq.** | **Amplicon (bp)** | **Probe seq.** |
|---|---|---|---|---|---|
| ***Neonatal sepsis*** | *Escherichia coli* | 9 | AACCACGCATCTGTTGACTGG CAGGTGGGGGCAAATGGTGAT GTCAGCGTTGAACTGCGTGAT GCGGAT | 69 | TGGTGA TG |
| ***Neonatal sepsis*** | *Streptococc* us *agalactiae* | 56 | TCAGTCGCAAGTGTTCAAGCA CAAGAAACAGATACGACGTGG ACAGCACGTACTGTTTCAGAG GTAAAGGCTGA | 74 | GGACAG CA |
| ***Neonatal sepsis*** | *Listeria monocytoge nes* | 142 | CGGAGATGCAGTGACAAATGT GCCGCCAAGAAAAGGTTACAA AGATGGAAATGAATATATCGTT GTGGAGAAAAGAAGAA | 81 | GCCAAG AA |

### 2. Singleplex assay (Comparative example)

### 2.1. Protocol validation by means of standards

### 2.1.1. Singleplex qPCR for the individual detection of E. coli (probe 9)/Str. agalactiae (probe 56)/L. monocytogenes (probe 142)/Bglo gene (probe 144)

### 2.1.1.1. Singleplex qPCR reaction

| Indicate no. of reactions | | 1 | 1 |
|---|---|---|---|
| | | | |

| Reagents (concentration) | Volume (µl) | Volume (µl) | Check |
|---|---|---|---|
| UPL/LNA probe (10 µM) | 0.5 | 0.5 | |
| Primer-F (100 µM) | 0.15 | 0.15 | |
| Primer-R (100 µM) | 0.15 | 0.15 | |
| Master Probes | 10 | 10 | |
| H20 | 4 | 4 | |
| UNG | 0.2 | 0.2 | |
| DNA | | 5 | |
| Final volume | 15 | 20 | |

### 2.1.1.2 Thermal cycler program

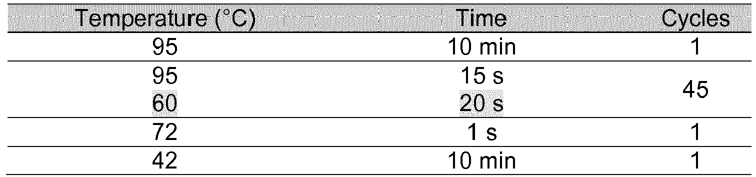

### 2.1.1.3. Detection limit of the target and amplification efficiency of each target in simplex format

To determine the detection limit of the target, 10-fold serial dilutions of plasmid DNA (standard DNA) were tested in a range of concentration usually varying between 10⁸ copies and 1 copy per well using 2 replicates per dilution.

Calibration curve parameters were analyzed to determine qPCR amplification efficiency. Efficiency: 10(-1/m) -1, m being the slope of the calibration line equation.

**2.1.1.3.1. Results**

| | Mean Ct obtained | | | |
|---|---|---|---|---|
| Standard plasmid DNA | *E. coli* (probe 9) | *Str. agalactiae* (probe 56) | *L. monocytogenes* (probe 142) | *Bglo* gene (probe 144) |
| 10⁸ copies | 12.39 | ND | 11.55 | 11.04 |
| 10⁷ copies | 16.39 | 15.34 | 15.8 | 15.35 |
| 10⁶ copies | ND | 19.08 | ND | ND |
| 10⁵ copies | ND | 22.62 | 23.11 | 21.73 |
| 10⁴ copies | 26.39 | 26.25 | 26.72 | 24.76 |
| 10³ copies | 29.86 | 29.78 | 29.99 | 28.22 |
| 10² copies | 33.62 | 33.52 | 33.23 | 31.92 |
| 10 copies | 37.02 | 38.06 | 36.37 | 34.32 |
| 1 copy | 40.15* | 39.6* | No Ct | *37.06 |
| CN | No Ct | No Ct | No Ct | No Ct |

| | | | | |
|---|---|---|---|---|
| *Not detected in any replicates ND: Not determined | | | | |

**2.1.1.3.1.1. E. coli (probe 9)**

| **Standard curve parameters obtained** | |
|---|---|
| Slope of the line | -3.46 |
| Correlation coefficient (R²) | 0.999 |
| Efficiency | 94.2% |
| Detection limit: 10 copies | |

**2.1.1.3.1.2. Str. agalactiae (probe 56)**

| **Standard curve parameters obtained** | |
|---|---|
| Slope of the line | -3.58 |
| Correlation coefficient (R²) | 0.992 |
| Efficiency | 90.2% |
| Detection limit: 10 copies | |

**2.1.1.3.1.3. L. monocytogenes (probe 142)**

| **Standard curve parameters obtained** | |
|---|---|
| Slope of the line | -3.53 |
| Correlation coefficient (R²) | 0.998 |
| Efficiency | 91.9% |
| Detection limit: 10 copies | |

**2.1.1.3.1.4. Bglo gene Cy5 (probe 144)**

| **Standard curve parameters obtained** | |
|---|---|
| Slope of the line | -3,27 |
| Correlation coefficient (R²) | 0.997 |
| Efficiency | 102% |
| Detection limit: 10 copies | |

### 2.1.2. Multiplex qPCR for the simultaneous detection of E.coli (probe 9)lStr. agalactiae (probe 56)/L. monocytogenes (probe 142)/Bglo gene (probe 144)

### 2.1.2.1. Multiplex qPCR reaction

| Indicate no. of reactions | | 1 | 1 |
|---|---|---|---|
| | | | |

| Reagent (concentration) | Volume (µl) | Volume (µl) | Check |
|---|---|---|---|
| Probe 9 (10 µm) | 0.5 | 0.5 | |
| Probe 56 (10 µM) | 0.5 | 0.5 | |
| Probe 142 (10 µM) | 0.5 | 0.5 | |
| Probe 144Cy5 (10 µM) | 0.5 | 0.5 | |
| uidA9F (100 µM) | 0.15 | 0.15 | |
| uidA9R (100 µM) | 0.15 | 0.15 | |
| sip56F (100 µM) | 0.15 | 0.15 | |
| sip56R (100 µM) | 0.15 | 0.15 | |
| hly142F (100 µM) | 0.15 | 0.15 | |
| hly142R (100 µM) | 0.15 | 0.15 | |
| Bglo144F (100 µM) | 0.15 | 0.15 | |
| Bglo144R (100 µM) | 0.15 | 0.15 | |
| Quantifast Qiagen | 10 | 10 | |
| H20 | 1.6 | 1.6 | |
| UNG | 0.2 | 0.2 | |
| DNA | | 5 | |
| Final volume | 15 | 20 | |

### 2.1.2.2. Thermal cycler program

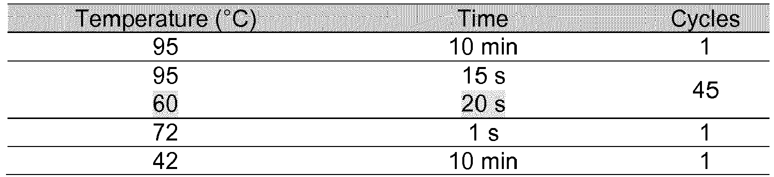

### 2.1.2.3. Multiplex qPCR functionality

The verification of qPCR functionality was tested by means of analyzing a small-scaled assay with 2 replicates of negative control (water) and 2 replicates of positive control made up of:
- *E.coli* plasmid DNA 4*10⁵ copies
- *Listeria monocytogenes* plasmid DNA 4*10⁵ copies
- *S. agalactiae* genomic DNA 6.8 ng
- *Bglo* gene plasmid DNA 4*10⁵ copies

| Multiplex | *St*. *agalactiae* FAM | *E. coli* HEX | *Listeria* Tex615 | *Bglo* Cy5 |
|---|---|---|---|---|
| | Ct | Ct | Ct | Ct |
| CP multiplex | 24.09 | 24.53 | 23.98 | 25.06 |
| CP multiplex | 24.31 | 25.49 | 25.09 | 24.54 |
| CN | No Ct | No Ct | No Ct | No Ct |
| CN | No Ct | No Ct | No Ct | No Ct |

### 2.1.2.4 Detection limit of the target and multiplex amplification efficiency

To determine the detection limit of the target, 10-fold serial dilutions of plasmid DNA (standard DNA) were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, m being the slope of the calibration line equation.

### 2.1.2.4.1. Results

**2.1.2.4.1.1. E. coli (probe 9)**

| | Singleplex | Multiplex |
|---|---|---|
| Standard plasmid DNA | *E. coli* (probe 9) | Mean Ct |
| 10⁸ copies | 12.39 | ND |
| 10⁷ copies | 16.39 | 14.86 |
| 10⁶ copies | ND | 18.73 |
| 10⁵ copies | ND | 21.95 |
| 10⁴ copies | 26.39 | 25.44 |
| 10³ copies | 29.86 | 29.28 |
| 10² copies | 33.62 | 32.805 |
| 10 copies | 37.02 | 38.85 |
| 1 copy | 40.15* | No Ct |
| CN | No Ct | No Ct |

| **Standard curve parameters** | **Singleplex** | **Multiplex** |
|---|---|---|
| Slope of the line | -3.46 | -3.49 |
| Correlation coefficient (R²) | 0.999 | 0.997 |
| Efficiency | 94.2% | 93.2% |
| Detection limit | 10 copies | 10 copies |

| | | |
|---|---|---|
| *Not detected in any replicas ND: Not determined | | |

**2.1.2.4.1.2. Streptococcus agalactiae (probe 56)**

| | Singleplex | Multiplex |
|---|---|---|
| Standard plasmid DNA | | |
| 10⁸ copies | ND | ND |
| 10⁷ copies | 15.34 | 15.71 |
| 10⁵ copies | 19.08 | 18.77 |
| 10⁴ copies | 22.62 | 23.34 |
| 10³ copies | 26.25 | 27.15 |
| 10² copies | 29.78 | 30.75 |
| 10 copies | 33.52 | 34.66 |
| 1 copy | 38.06* | 39.2 |
| CN | No Ct | No Ct |

| **Standard curve parameters** | **Singleplex** | **Multiplex** |
|---|---|---|
| Slope of the line | -3.58 | -3.74 |
| Correlation coefficient (R²) | 0.992 | 0.998 |
| Efficiency | 90.2% | 85% |
| Detection limit | 10 copies | 10 copies |

| | | |
|---|---|---|
| *Not detected in any replicas ND: Not determined | | |

**2.1.2.4.1.3. Listeria monocytogenes (probe 142)**

| | Singleplex | Multiplex |
|---|---|---|
| Standard plasmid DNA | | |
| 10⁸ copies | 11.55 | ND |
| 10⁷ copies | 15.8 | 14.50 |
| 10⁶ copies | ND | 18.52 |
| 10⁵ copies | 23.11 | 22.22 |
| 10⁴ copies | 26.72 | 25.63 |
| 10³ copies | 29.99 | 29.29 |
| 10² copies | 33.23 | 32.47 |
| 10 copies | 36.37 | 36.33 |
| 1 copy | No Ct | No Ct |
| CN | No Ct | No Ct |

| **Standard curve parameters** | **Singleplex** | **Multiplex** |
|---|---|---|
| Slope of the line | -3.53 | -3.58 |
| Correlation coefficient (R²) | 0.998 | 0.999 |
| Efficiency | 91.9% | 90% |
| Detection limit | 10 copies | 10 copies |

| | | |
|---|---|---|
| ND: Not determined | | |

**2.1.2.4.1.4. Bglo gene (probe 144)**

| | Singleplex | Multiplex |
|---|---|---|
| Standard plasmid DNAs | *Bglo* gene (probe 144) | |
| 10⁸ copies | 11.04 | ND |
| 10⁷ copies | 15.35 | 15.25 |
| 10⁶ copies | ND | 18.24 |
| 10⁵ copies | 21.73 | 22.24 |
| 10⁴ copies | 24.76 | 24.99 |
| 10³ copies | 28.22 | 29.27 |
| 10² copies | 31.92 | 32.51 |
| 10 copies | 34.32 | 35.92 |
| 1 copy | *37.06 | 38.28* |
| CN | No Ct | No Ct |

| **Standard curve parameters** | **Singleplex** | **Multiplex** |
|---|---|---|
| Slope of the line | -3.27 | -3.42 |
| Correlation coefficient (R²) | 0.997 | 0.997 |
| Efficiency | 102% | 96.1% |
| Detection limit | 10 copies | 10 copies |

| | | |
|---|---|---|
| *Not detected in any replicates. ND: Not determined | | |

### Example 3. Kit for the detection of herpes simplex virus 1, herpes simplex virus 2 and varicella zoster virus

The present example refers to a real-time PCR kit indicated for the detection of herpes simplex virus 1, herpes simplex virus 2, varicella zoster virus and the Bglob gene. Particularly, this example shows the usefulness of the present invention for carrying out the detection, quantification and identification of four or more different strains or species of microorganisms in one and the same biological sample using short probes.

### 1. Assay description

The present assay relates to a kit indicated for the simultaneous detection of herpes simplex virus 1, herpes simplex virus 2, varicella zoster virus and the *Bglo* gene as a DNA extraction control/PCR inhibition control. This control allows verifying that the extraction process has been developed correctly and detecting false negatives due to PCR inhibition.

The kit contains the required reagents and enzymes mixed at ideal concentrations and designed to make diagnostic analysis easier.

Each Master Mix provides a HEX-(quencher Exiqon)-labeled UPL probe for the detection of herpes simplex virus 1, a short FAM-BHQ1®-labeled LNA probe for the detection of herpes simplex virus 2, a TexTM615-(quencher Exiqon)-labeled UPL probe for the detection of varicella zoster virus and a Cy5-BHQ3®-labeled probe for the detection of the *Bglo* gene (DNA extraction control/PCR inhibition control gene). The channels through which fluorescence-related data is collected are described in detail in the following table.

**Table 1: Reporters and Quenchers**

| | Reporter | Quencher |
|---|---|---|
| Herpes simplex virus 1 (probe 63) | HEX/JOE | Exiqon |
| Herpes simplex virus 2 (probe 157) | FAM | BHQ1® |
| Varicella zoster virus (probe 142) | ROX* | Exiqon |
| *Bglo* gene (probe 144) | Cy5 | BHQ3® |

| | | |
|---|---|---|
| *The fluorophores ROX and Tex™-615 have the same emission spectra | | |

The probes and primers used for carrying out this assay are described in detail below:

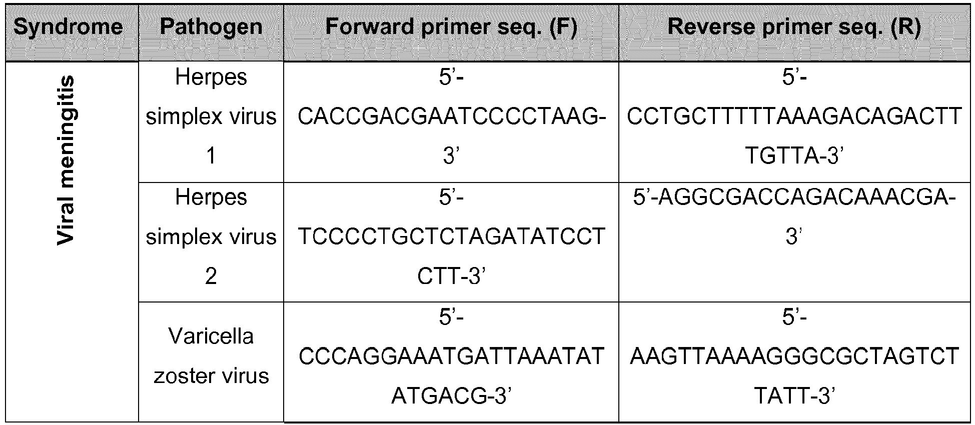

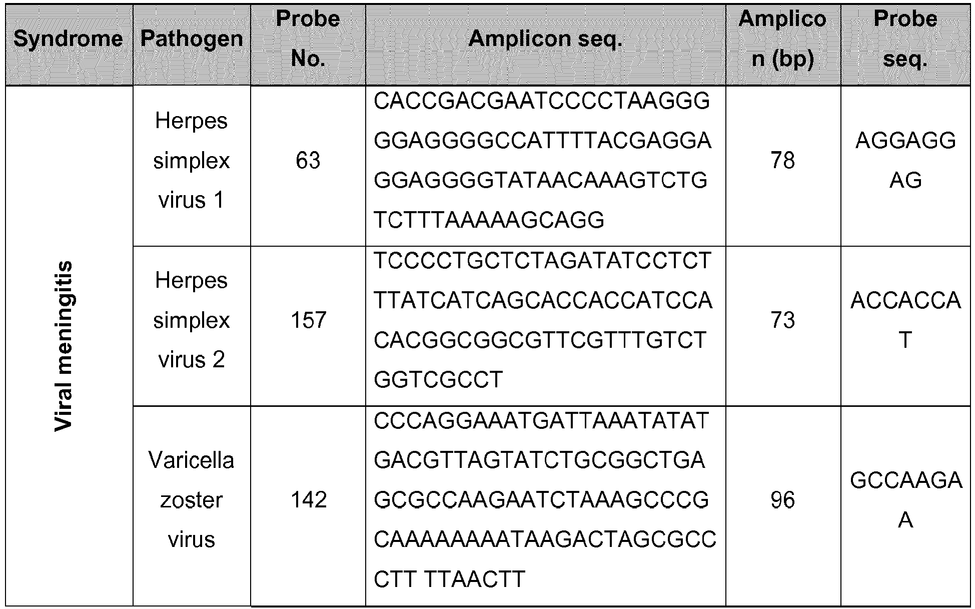

### 2. Singleplex assay (Comparative example)

### 2.1. Protocol validation by means of standards

### 2.1.1. Singleplex qPCR for the individual detection of herpes simplex virus 1/herpes simplex virus 2/varicella zoster virus/Bglo gene

### 2.1.1.1. Singleplex qPCR reaction

| Indicate no. of reactions | | 1 | 1 |
|---|---|---|---|
| | | | |

| Reagent (concentration)) | Volume (µl) | Volume (µl) | Check |
|---|---|---|---|
| UPL/LNA probe (10 µM) | 0.5 | 0.5 | |
| Primer-F (100 µM) | 0.15 | 0.15 | |
| Primer-R (100 µM) | 0.15 | 0.15 | |
| Master Probes | 10 | 10 | |
| H20 | 4 | 4 | |
| UNG | 0.2 | 0.2 | |
| DNA | | 5 | |
| Final volume | 15 | 20 | |

### 2.1.1.2. Thermal cycler program

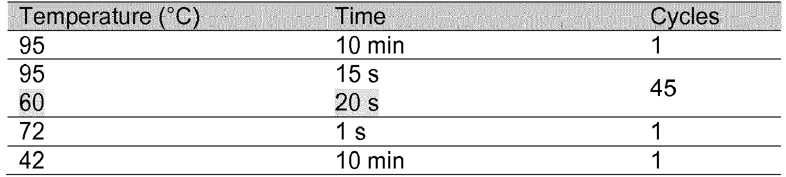

### 2.1.1.3. Detection limit of the target and amplification efficiency of each target in simplex format

To determine the detection limit of the target, 10-fold serial dilutions of plasmid DNA (standard DNA) were tested in a range of concentration usually varying between 10⁸ copies and 1 copy per well using 2 replicates per dilution.

Calibration curve parameters were analyzed to determine qPCR amplification efficiency. Efficiency: 10(-1/m) -1, m being the slope of the calibration line equation.

**2.1.1.3.1. Results**

| | Mean C obtained | | | |
|---|---|---|---|---|
| Standard plasmid DNA | Herpes simplex virus 1 (probe 63) | Herpes simplex virus 1 (probe 157) | Herpes simplex virus 1 (probe 142) | *Bglo* gene (probe 144) |
| 10⁸ copies | 12.77 | 12.87 | 12.23 | 11.04 |
| 10⁷ copies | 16.28 | 16.76 | 15.47 | 15.35 |
| 10⁵ copies | 26.05 | 24.32 | 22.38 | 21.73 |
| 10⁴ copies | 29.88 | 28.38 | 25.88 | 24.76 |
| 10³ copies | 33.17 | 31.49 | 29.26 | 28.22 |
| 10² copies | 36.89 | 35.34 | 32.61 | 31.92 |
| 10 copies | 39.83 | 39.335 | 35.95 | 34.32 |
| 1 copy | No Ct | No Ct | No Ct | *37.06 |
| CN | No Ct | No Ct | No Ct | No Ct |

| | | | | |
|---|---|---|---|---|
| *Not detected in any replicates | | | | |

**2.1.1.3.1.1. Herpes simplex virus 1 (probe 63)**

| **Standard curve parameters obtained** | |
|---|---|
| Slope of the line | -3.96 |
| Correlation coefficient (R²) | 0.994 |
| Efficiency | 78.9% |
| Detection limit: 10 copies | |

**2.1.1.3.1.2. Herpes simplex virus 2 (probe 157)**

| **Standard curve parameters obtained** | |
|---|---|
| Slope of the line | -3.75 |
| Correlation coefficient (R²) | 0.998 |
| Efficiency | 84.7% |
| Detection limit: 10 copies | |

**2.1.1.3.1.3. Varicella zoster virus (probe 142)**

| **Standard curve parameters obtained** | |
|---|---|
| Slope of the line | -3.40 |
| Correlation coefficient (R2) | 0.998 |
| Efficiency | 96.6% |
| Detection limit: 10 copies | |

**2.1.1.3.1.4. Bglo gene Cy5 (probe 144)**

| **Standard curve parameters obtained** | |
|---|---|
| Slope of the line | -3,27 |
| Correlation coefficient (R²) | 0.997 |
| Efficiency | 102% |
| Detection limit: 10 copies | |

### 2.1.2. Multiplex qPCR for the simultaneous detection of herpes simplex virus 1/herpes simplex virus 2/varicella zoster virus /Bglo gene

### 2.1.2.1. Multiplex qPCR reaction

| Indicate no. of reactions | | 1 | 1 |
|---|---|---|---|
| | | | |

| Reagent (concentration) | Volume (µl) | Volume (µl) | Check |
|---|---|---|---|
| Probe 63HEX (10 µM) | 0.5 | 0.5 | |
| Probe 142Tex615 (10 µM) | 0.5 | 0.5 | |
| Probe 157FAM (10 µM) | 0.5 | 0.5 | |
| Probe 144Cy5 (10 µM) | 0.5 | 0.5 | |
| Her1S63F (100 µM) | 0.15 | 0.15 | |
| Her1S63R (100 µM) | 0.15 | 0.15 | |
| Her2S157F (100 µM) | 0.15 | 0.15 | |
| Her2S157R (100 µM) | 0.15 | 0.15 | |
| WZ142F (100 µM) | 0.15 | 0.15 | |
| WZ142R (100 µM) | 0.15 | 0.15 | |
| Bglo144F (100 µM) | 0.15 | 0.15 | |
| Bglo144R (100 µM) | 0.15 | 0.15 | |
| Probes Master | 10 | 10 | |
| H20 | 1.6 | 1.6 | |
| UNG | 0.2 | 0.2 | |
| DNA | | 5 | |
| Final volume | 15 | 20 | |

### 2.1.2.2. Thermal cycler program

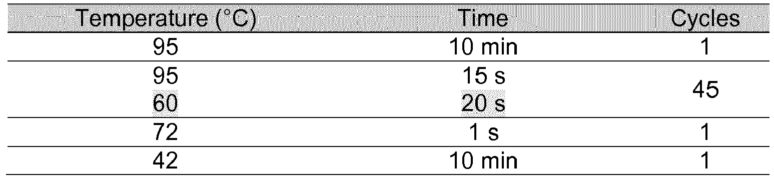

### 2.1.2.3. Detection limit of the target and multiplex amplification efficiency

To determine the detection limit of the target, 10-fold serial dilutions of plasmid DNA (standard DNA) were tested in a range of concentration usually varying between 10⁷ copies and 1 copy per well using 2 replicates per dilution.

Calibration curve parameters were analyzed to determine qPCR amplification efficiency. Efficiency: 10(-1/m) -1, m being the slope of the calibration line equation.

The starting standard for analyzing the detection limit and amplification efficiency is made up of:
- 2.5*10⁷ copies of plasmid DNA with the cloned amplicon of herpes simplex virus 1 (probe 63).
- 2.5*10⁷ copies of plasmid DNA with the cloned amplicon of herpes simplex virus 2 (probe 157).
- 2.5*10⁷ copies of plasmid DNA with the cloned amplicon of varicella zoster virus (probe 142).

### 2.1.2.3.1.1. Results

To compare the results, a piece of multiplex qPCR amplification efficiency data was used (see the section below) and the possible Cts obtained if it had been based on a non-diluted standard concentration, were found with this piece of data.

**2.1.2.3.1.1.1. Herpes simplex virus 1 (probe 63)**

| | Singleplex | Multiplex |
|---|---|---|
| Standard plasmid DNA | Herpes simplex virus 1 (probe 63) | Mean Ct applied for correction * |
| 10⁸ copies | 12.77 | 12.44 |
| 10⁷ copies | 16.28 | |
| 10⁵ copies | 26.05 | 26.12 |
| 10⁴ copies | 29.88 | |
| 10³ copies | 33.17 | |
| 10² copies | 36.89 | 37.23 |
| 25 copies | | 39.29 |
| 10 copies | 39.83 | |
| 1 copy | No Ct | No Ct |
| CN | No Ct | No Ct |

| **Standard curve parameters** | **Singloplex** | **Multiplex** |
|---|---|---|
| Slope of the line | -3.96 | -4.13 |
| Correlation coefficient (R²) | 0.994 | 0.996 |
| Efficiency | 78.9% | 74.6% |
| Detection limit | 10 copies | 10 copies |

| | | |
|---|---|---|
| *Slope value applied for correction: -4.13 | | |

**2.1.2.3.1.1.2. Herpes simplex virus 2 (probe 157)**

| | Singleplex | Multiplex |
|---|---|---|
| Standard plasmid DNA | Herpes simplex virus 2 (probe 157) | Mean Ct applied for correction |
| 10⁸ copies | 12.87 | 13.40 |
| 10⁷ copies | 16.76 | |
| 10⁵ copies | 24.32 | 23.76 |
| 10⁴ copies | 28.38 | |
| 10³ copies | 31.49 | |
| 10² copies | 35.34 | 35.10 |
| 25 copies | | 36.91 |
| 10 copy | 39.33 | 38.72 |
| 1 copy | No Ct | No Ct |
| CN | No Ct | No Ct |

| **Standard curve parameters** | **Singleplex** | **Multiplex** |
|---|---|---|
| Slope of the line | -3.75 | -3.61 |
| Correlation coefficient (R2) | 0.998 | 0.998 |
| Efficiency | 84.7% | 89% |
| Detection limit | 10 copies | 10 copies |

**2.1.2.3.1.1.3. Varicella zoster virus (probe 142)**

| | Singleplex | Multiplex |
|---|---|---|
| Standard plasmid DNA | Varicella zoster virus (probe 143) | Mean Ct applied for correction |
| 10⁸ copies | 12.23 | 11.61 |
| 10⁷ copies | 15.47 | |
| 10⁵ copies | 22.38 | 21.78 |
| 10⁴ copies | 25.88 | |
| 10³ copies | 29.26 | |
| 10² copies | 32.61 | 31.58 |
| 25 copies | | 36.26 |
| 10 copy | 35.95 | 34.6 |
| 1 copy | No Ct | No Ct |
| CN | No Ct | No Ct |

| **Standard curve parameters** | **Singleplex** | **Multiplex** |
|---|---|---|
| Slope of the line | -3.40 | -3.32 |
| Correlation coefficient (R2) | 0.998 | 0.985 |
| Efficiency | 96.6% | 99.7% |
| Detection limit | 10 copies | 10 copies |

**2.1.2.3.1.1.4. Bglo gene (probe 144)**

| | Singleplex | Multiplex |
|---|---|---|
| Standard plasmid DNA | *Bglo* gene (probe 144) | Mean Ct applied for correction |
| 10⁸ copies | 11.04 | 11.63 |
| 10⁷ copies | 15.35 | |
| 10⁵ copies | 21.73 | 22.38 |
| 10⁴ copies | 24.76 | |
| 10³ copies | 28.22 | |
| 10² copies | 31.92 | 32.98 |
| 10 copies | 34.32 | 36.14 |
| 1 copy | *37.06 | 36.49* |
| CN | No Ct | |

| **Standard curve parameters** | **Singloplex** | **Multiplex** |
|---|---|---|
| Slope of the line | -3.27 | -3.16 |
| Correlation coefficient (R²) | 0.997 | 0.975 |
| Efficiency | 102% | 106.8% |
| Detection limit | 10 copies | 10 copies |

| | | |
|---|---|---|
| *Not detected in any replicates | | |

### Example 4. Real-time multiplex PCR kit indicated for the simultaneous detection of, on one hand, Streptococcus pneumoniae, S. pyogenes and the endogenous Bglo gene, and on the other, Haemophilus influenzae, Staphylococcus aureus, Kingelia kingae and the endogenous Bglo gene

The present example refers to a real-time PCR kit indicated for the simultaneous detection of, on one hand, *Streptococcus pneumoniae, S. pyogenes* and the endogenous *Bglo* gene, and on the other, *Haemophilus influenzae, Staphylococcus aureus, Kingella kingae* and the endogenous *Bglo* gene. Particularly, this example shows the usefulness of the present invention for carrying out the detection, quantification and identification of two or more different strains or species of microorganisms in one and the same biological sample using short probes.

### 1. Assay description

To carry out the present assay, a real-time PCR kit indicated for the simultaneous detection of, on one hand, *Streptococcus pneumoniae, S. pyogenes* and the endogenous *Bglo* gene, and on the other, *Haemophilus influenzae, Staphylococcus aureus, Kingella kingae* and the endogenous *Bglo* gene, is prepared. The kit contained the required reagents and enzymes mixed at ideal concentrations and designed to make diagnostic analysis easier.

The endogenous gene works as a DNA extraction control and PCR inhibition control. It allows verifying that the extraction process has been developed correctly and detecting false negatives due to PCR inhibition.

The kit contains 2 mixes:
- **Mix 1** provides a HEX-labeled UPL probe for the detection of *S. pneumoniae,* a Tex 615-labeled UPL probe for the detection of *S*. *pyogenes* and a Cy5-BHQ3®-labeled LNA probe for the detection of the *Bglo* gene (DNA extraction control/PCR inhibition control gene).
- **Mix 2** provides a FAM-labeled UPL probe for the detection of *K*. *kingae,* a HEX-labeled UPL probe for the detection of *S*. *aureus,* a Tex 615-labeled UPL probe for the detection of *H*. *influenzae* and a Cy5-BHQ3®-labeled LNA probe for the detection of the *Bglo* gene (DNA extraction control/PCR inhibition control gene).

The channels through which fluorescence-related data is collected are described in detail in Table 1.

**Table 1: Reporters and Quenchers**

| Mix 1 | Reporter | Quencher |
|---|---|---|
| *S*. *pneumoniae* (probe 129) | HEX | Exiqon |
| *S*. *pyogenes* (probe 38) | Tex615/ROX | Exiqon |
| *Bglo* gene (probe 144) | Cy5 | BHQ3® |

| Mix 2 | Reporter | Quencher |
|---|---|---|
| *K. kingae* (probe 84) | FAM | Roche |
| *S*. *aureus* (probe 129) | HEX | Exiqon |
| *H. influenzae* (probe 110) | Tex615/ROX | Exiqon |
| *Bglo* gene (probe 144) | Cy5 | BHQ3® |

The UPL/LNA probe as well as the forward (F) and reverse (R) primers have the sequences provided in detail below:

| **Syndrome** | **Pathogen** | **Forward primer seq. (F)** | **Reverse primer seq**. (**R**) |
|---|---|---|---|
| ***Infectious arthritis*** | *S*. *pneumoniae* | 5'- TCAAACCAGACGGAACACT G-3' | 5’- TCAAGCCATCTGGCTCTACTG -3’ |
| ***Infectious arthritis*** | *S. pyogenes* | 5'- GAGCTGAGATTAGCATTTCC TCA-3' | 5'- TGTCATCGACTTGGTCTAAAC TG-3' |
| ***Infectious arthritis*** | *K. kingae* | 5’- AAGGAGGCAGTCTGAACGA A-3’ | 5'- GCATCTTCTTGTAATCGAGCA A-3' |
| ***Infectious arthritis*** | *S. aureus* | 5'- TCACTTGGTACTTCTGGTGT CG-3 | 5'-TACCGAGTGAGCCGGAAA- 3 |
| ***Infectious arthritis*** | *H. influenzae* | 5’- GGAAATATCAGTGTTGCAG GTAAG-3’ | 5’- TTACGGCTAAGGCTTGCTACA -3’ |

| **Syndrome** | **Pathogen** | **Probe No.** | **Amplicon seq.** | **Amplicon (bp)** | **Probe seq.** |
|---|---|---|---|---|---|
| ***Infectious arthritis*** | *S*. *pneumoniae* | 129 | TCAAACCAGACGGAACACTG GCAGACAAGCCAGAATTCAC AGTAGAGCCAGATGGCTTGA | 60 | AGCCAG AA |
| ***Infectious arthritis*** | *S. pyogenes* | 38 | GAGCTGAGATTAGCATTTCCT CAATTAAGCTATAATCAAGAG CTAATTCCTCAGGAAGCAGG ATTTTTAGTCAGTTTAGACCA AGTCGA TGACA | 94 | GGAAGC AG |
| ***Infectious arthritis*** | *K. kingae* | 84 | AAGGAGGCAGTCTGAACGAA CTTATAAAAGCAGCAGATGAA CTAGGGATTGCTCGATTAC AAGAAGATGC | 70 | GCAGCA GA |
| ***Infectious arthritis*** | *S. aureus* | 129 | TCACTTGGTACTTCTGGTGTC GGTGGCGTTGGTGTTTCTGG CTCACTCGGTACTTCCGGCG TCGGTGGTGTTGGTGTTTCC GGCTCACTCGGTA | 94 | AGCCAG AA |
| ***Infectious arthritis*** | *H. influenzae* | 110 | GGAAATATCAGTGTTGCAGGT AAGACACCAGAGGCTATTCAA GCTCAAATTGTAGCAAGCCTT AGCCGTAA | 71 | CAGAGG CT |

The probe as well as the primers for the Bglob gene have the sequences provided in detail below:

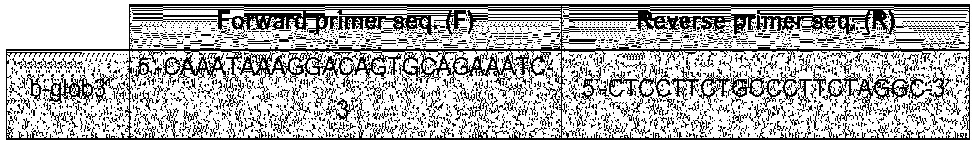

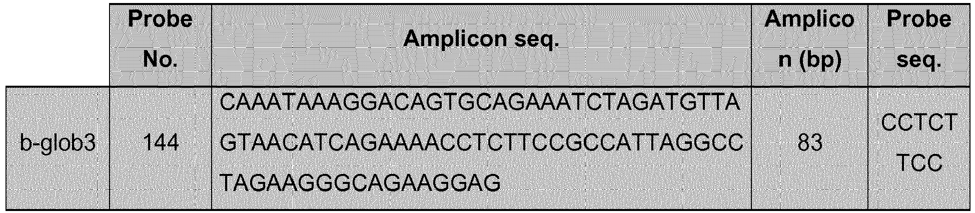

### 2. Singleplex assay (Comparative example)

### 2.1 Protocol validation by means of standards

### 2.1.1. Singleplex qPCR for the individual detection of S. pneumoniae (probe 129)/S. pyogenes (probe 38)/K. kingae (probe 84)/S. aureus (probe 129)/H. influenzae (probe 110)/Bglo gene (probe 144)

### 2.1.1.1. Singleplex qPCR reaction

| **Indicate no. of reactions** | **1** | **1** |
|---|---|---|
| | | |

| Reagent (concentration)) | Volume (µl) | Volume (µl) |
|---|---|---|
| UPL/LNA probe (10 µM) | 0.5 | 0.5 |
| Primer-F (100 µM) | 0.15 | 0.15 |
| Primer-R (100 µM) | 0.15 | 0.15 |
| Master Probes | 10 | 10 |
| H20 | 4 | 4 |
| UNG | 0.2 | 0.2 |
| DNA | | 5 |
| Final volume | 15 | 15 |

### 2.1.1.2. Thermal cycler program

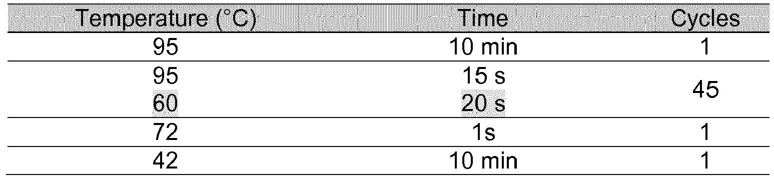

### 2.1.1.3. Detection limit of the target and amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, "m" being the slope of the calibration line equation.

**2.1.1.3.1. Results**

| | Mean Ct obtained | | |
|---|---|---|---|
| Standard plasmid DNA | *S*. *pneumoniae* (probes 129) | *S. pyogenes* (probe 38) | ***Bglo* gene (probe 144)** |
| 10⁷ copies | 17.75 | 15.28 | 15.35 |
| 10⁶ copies | 21.84 | 19.23 | ND |
| 10⁵ copies | 26.70 | 24.02 | 21.73 |
| 10⁴ copies | 30.41 | 27.45 | 24.76 |
| 10³ copies | 33.42 | 31.00 | 28.22 |
| 10² copies | 37.07 | 33.89 | 31.92 |
| 10 copies | 39.305 | 37.11 | 34.32 |
| 1 copy | 41.96 | 38.82 | 37.06* |
| CN | No Ct | No Ct | No Ct |

| | Mean Ct obtained | | |
|---|---|---|---|
| Standard plasmid DNA | *K. kingae* (probe 84) | *S. aureus* (probe 129) | *H. influenzae* (probe 110) |
| 10⁷ copies | 16.34 | 19.15 | 14.83 |
| 10⁶ copies | 19.82 | 22.29 | 18.81 |
| 10⁵ copies | 24.32 | 26.36 | 23.82 |
| 10⁴ copies | 27.36 | 29.92 | 26.80 |
| 10³ copies | 30.53 | 33.32 | 29.76 |
| 10² copies | 34.38 | 35.60 | 34.43 |
| 10 copies | 37.05 | 40.18 | 36.64 |
| 1 copy | 41.05* | No Ct | No Ct |
| CN | No Ct | No Ct | No Ct |

| | | | |
|---|---|---|---|
| *Not detected in any replicates. ND:Not determined | | | |

**2.1.1.3.1.1. S. pneumoniae (probe 129)**

| **Standard curve parameters obtained** | |
|---|---|
| Slope of the line | -3.32 |
| Correlation coefficient (R²) | 0.971 |
| Efficiency | 99.9% |
| Detection limit: 1 copy | |

**2.1.1.3.1.2. S. pyogenes (probe 38)**

| **Standard curve parameters obtained** | |
|---|---|
| Slope of the line | -3.54 |
| Correlation coefficient (R²) | 0.989 |
| Efficiency | 91.5% |
| Detection limit: 1 copy | |

**2.1.1.3.1.3. K. kingae (probe 84)**

| **Standard curve parameters obtained** | |
|---|---|
| Slope of the line | -3.48 |
| Correlation coefficient (R²) | 0.995 |
| Efficiency | 93.7% |
| Detection limit: 10 copies | |

**2.1.1.3.1.4. S. aureus (probe 129)**

| **Standard curve parameters obtained** | |
|---|---|
| Slope of the line | -3.43 |
| Correlation coefficient (R²) | 0.995 |
| Efficiency | 95.6% |
| Detection limit: 10 copies | |

**2.1.1.3.1.5. H. influenzae (probe 110)**

| **Standard curve parameters obtained** | |
|---|---|
| Slope of the line | -3.69 |
| Correlation coefficient (R²) | 0.992 |
| Efficiency | 86.4% |
| Detection limit: 10 copies | |

**2.1.1.3.1.6. Bglo gene Cy5 (probe 144)**

| **Standard curve parameters obtained** | |
|---|---|
| Slope of the line | -3.27 |
| Correlation coefficient (R²) | 0.997 |
| Efficiency | 102% |
| Detection limit: 10 copies | |

### 2.1.2. Mix 1: multiplex qPCR for the simultaneous detection of S. pneumoniae (probe 129)/S. agalactiae (probe 56)/S. pyogenes (probe 38)/Bglo gene (probe 144)

### 2.1.2.1. Multiplex qPCR reaction

| Indicate no. of reactions | | 1 |
|---|---|---|
| | | |

| Reagent (concentration) | Volume (µl) | Check |
|---|---|---|
| Probe SP129HEX (10 µM) | 0.5 | 0.5 |
| Probe SP38tex615 (10 µM) | 0.5 | 0.5 |
| Probe Bglo144Cy5 (10 µM) | 0.5 | 0.5 |
| SP129F (100 µM) | 0.15 | 0.15 |
| SP129R (100 µM) | 0.15 | 0.15 |
| SP38F (100 µM) | 0.15 | 0.15 |
| SP38R (100 µM) | 0.15 | 0.15 |
| Bglo144F (100 µM) | 0.15 | 0.15 |
| Bglo144R (100 µM) | 0.15 | 0.15 |
| Master Mix Roche | 10 | 10 |
| H20 | 2.4 | 2.4 |
| UNG | 0.2 | 0.2 |
| DNA | | |
| Final volume | 15 | 15 |

### 2.1.2.2. Thermal cycler program

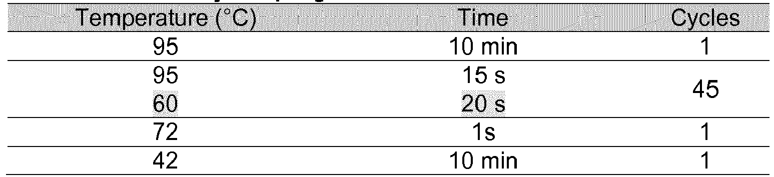

### 2.1.2.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well using 3 replicates per dilution.

Calibration curve parameters were analyzed to determine qPCR amplification efficiency. Efficiency: 10(-1/m)-1, m being the slope of the calibration line equation.

### 2.1.2.3.1. Results

**2.1.2.3.1.1. S. pneumoniae (probe 129)**

| **Standard plasmid DNA** | ***S*. *pneumoniae* (probe129)** | |
|---|---|---|
| **Singleplex** | **Singleplex** | **Multiplex** |
| **Mean Ct** | **Mean Ct** | **Mean Ct** |
| 10⁷ copies | 17.75 | 15.18 |
| 10⁶ copies | 21.84 | 18.38 |
| 10⁵ copies | 26.70 | 21.41 |
| 10⁴ copies | 30.41 | 24.69 |
| 10³ copies | 33.42 | 27.96 |
| 10² copies | 37.07 | 31.78 |
| 10 copies | 39.305 | 35.19 |
| 1 copy | 41.96 | 38.86 |
| CN | No Ct | No Ct |

| **Standard curve parameters** | **Singleplex** | **Multiplex** |
|---|---|---|
| Slope of the line | -3.32 | -3.35 |
| Correlation coefficient (R²) | 0.971 | 0.997 |
| Efficiency | 99.9% | 98.8% |
| Detection limit | 1 copy | 1 copy |

**2.1.2.3.1.2. S. pyogenes (probe 38)**

| **Standard plasmid DNA** | ***S. pneumoniae* (probe 38)** | |
|---|---|---|
| | **singleplex** | **Multiplex** |
| | **Mean Ct** | **Mean Ct** |
| 10⁷ copies | 15.28 | 13.25 |
| 10⁶ copies | 19.23 | 16.09 |
| 10⁵ copies | 24.02 | 19.69 |
| 10⁴ copies | 27.45 | 23.4 |
| 10³ copies | 31.00 | 26.96 |
| 10² copies | 33.89 | 30.63 |
| 10 copies | 37.11 | 33.69 |
| 1 copy | 38.82 | No Ct |
| CN | No Ct | No Ct |

| **Standard curve parameters** | **Singleplex** | **Multiplex** |
|---|---|---|
| Slope of the line | -3.54 | -3.49 |
| Correlation coefficient (R²) | 0.989 | 0.998 |
| Efficiency | 91.5% | 93.3% |
| Detection limit | 1 copy | 1 copy |

| | | |
|---|---|---|
| *Not detected in any replicas | | |

**2.1.2.3.1.3. Bglo gene (probe 144)**

| **Standard plasmid DNA** | ***Bglo* gene (probe 144)** | |
|---|---|---|
| | **singleplex** | **Multiplex** |
| | **Mean Ct** | **Mean Ct** |
| 10⁷ copies | 15.35 | 12.71 |
| 10⁶ copies | ND | 16.12 |
| 10⁵ copies | 21.73 | 19.07 |
| 10⁴ copies | 24.76 | 23.25 |
| 10³ copies | 28.22 | 25.81 |
| 10² copies | 31.92 | 30.24 |
| 10 copies | 34.32 | 34.19 |
| 1 copy | 37.06* | No Ct |
| CN | No Ct | No Ct |

| **Standard curve parameters** | **Singleplex** | **Multiplex** |
|---|---|---|
| Slope of the line | -3.27 | -3.54 |
| Correlation coefficient (R²) | 0.997 | 0.994 |
| Efficiency | 102% | 91.6% |
| Detection limit | 10 copy | 10 copy |

| | | |
|---|---|---|
| *Not detected in any replicas | | |

### 2.1.3. Mix 2: multiplex qPCR for the simultaneous detection of H. influenzae (probe 110)/S. aureus (probe 129)/Kingella kingae (probe 84)/Bglo gene (probe 144)

**2.1.3.1. Multiplex qPCR reaction**

| Indicate no. of reactions | | 1 |
|---|---|---|
| | | |

| Reagent (concentration) | Volume (µl) | Check |
|---|---|---|
| Probe SAU129HEX (10 µM) | 0.5 | 0.5 |
| Probe KK84FAM (10 µM) | 0.5 | 0.5 |
| Probe Bex110tex615 (10 µM) | 1 | 1 |
| Probe Bglo144Cy5 (10 µM) | 0.5 | 0.5 |
| SAU129F (100 µM) | 0.15 | 0.15 |
| SAU129R (100 µM) | 0.15 | 0.15 |
| KK84F (100 µM) | 0.15 | 0.15 |
| KK84R (100 µM) | 0.15 | 0.15 |
| Bex110x (100 µM) | 0.3 | 0.3 |
| Bex110R (100 µM) | 0.3 | 0.3 |
| Bglo144F (100 µM) | 0.15 | 0.15 |
| Bglo144R (100 µM) | 0.15 | 0.15 |
| Master Mix roche | 10 | 10 |
| H20 | 0.8 | 0.8 |
| UNG | 0.2 | 0.2 |
| DNA | 5 | 5 |
| Final volume | 14.8 | 14.8 |

**2.1.3.1.1.1. H. influenzae (probe 110)**

| **Standard plasmid DNA** | ***H. influenzae* (probe 110)** | |
|---|---|---|
| | **Singleplex** | **Multiplex** |
| **Mean Ct** | **Mean Ct** | **Mean Ct** |
| 10⁷ copies | 14.83 | 15.57 |
| 10⁶ copies | 18.81 | 18.63 |
| 10⁵ copies | 23.82 | 21.93 |
| 10⁴ copies | 26.80 | 26.38 |
| 10³ copies | 29.76 | 29.95 |
| 10² copies | 34.43 | 30.24 |
| 10 copies | 36.64 | 34.36 |
| 1 copy | No Ct | 36.5* |
| CN | No Ct | No Ct |

| **Standard curve parameters** | **Singleplex** | **Multiplex** |
|---|---|---|
| Slope of the line | -3.69 | -3.68 |
| Correlation coefficient (R²) | 0.992 | 0.995 |
| Efficiency | 86.4% | 86.8% |
| Detection limit | 10 copies | 10 copies |

| | | |
|---|---|---|
| *Not detected in any replicas | | |

**2.1.3.1.1.2. S. aureus (probe 129)**

| **Standard plasmid DNA** | ***S. aureus* (probe *129*)** | |
|---|---|---|
| | **Singleplex** | **Multiplex** |
| | **Mean Ct** | **Mean Ct** |
| 10⁷ copies | 19.15 | 16.15 |
| 10⁶ copies | 22.29 | 19.39 |
| 10⁵ copies | 26.36 | 23.86 |
| 10⁴ copies | 29.92 | 26.49 |
| 10³ copies | 33.32 | 30.14 |
| 10² copies | 35.60 | Outlier |
| 10 copies | 40.18 | 36.77 |
| 1 copy | No Ct | No Ct |
| CN | No Ct | No Ct |

| **Standard curve parameters** | **Singleplex** | **Multiplex** |
|---|---|---|
| Slope of the line | -3.43 | -3.46 |
| Correlation coefficient (R²) | 0.995 | 0.994 |
| Efficiency | 95.6% | 94.2% |
| Detection limit | 10 copies | 10 copies |

**2.1.3.1.1.3. K. kingae (probe 84)**

| **Standard plasmid DNA** | ***H. kingae* (probe 84)** | |
|---|---|---|
| | **Singleplex** | **Multiplex** |
| | **Mean Ct** | **Mean Ct** |
| 10⁷ copies | 16.34 | 15.35 |
| 10⁶ copies | 19.82 | 18.99 |
| 10⁵ copies | 24.32 | 23.63 |
| 10⁴ copies | 27.36 | 26.91 |
| 10³ copies | 30.53 | 30.12 |
| 10² copies | 34.38 | 33.77 |
| 10 copies | 37.05 | 36.57 |
| 1 copy | 41.05* | 39.85* |
| CN | No Ct | No Ct |

| **Standard curve parameters** | **Singleplex** | **Multiplex** |
|---|---|---|
| Slope of the line | -3.48 | -3.55 |
| Correlation coefficient (R²) | 0.995 | 0.995 |
| Efficiency | 93.7% | 91% |
| Detection limit | 10 copies | 10 copies |

| | | |
|---|---|---|
| *Not detected in any replicas | | |

**2.1.3.1.1.4. Bglo gene (probe 144)**

| **Standard plasmid DNA** | ***Bglo* gene (probe 144)** | |
|---|---|---|
| | **Singleplex** | **Multiplex** |
| | **Mean Ct** | **Mean Ct** |
| 10⁷ copies | 15.35 | 13.37 |
| 10⁶ copies | ND | 17.94 |
| 10⁵ copies | 21.73 | 21.19 |
| 10⁴ copies | 24.76 | 25.23 |
| 10³ copies | 28.22 | 27.89 |
| 10² copies | 31.92 | 31.58 |
| 10 copies | 34.32 | 33.54 |
| 1 copy | 37.06* | No Ct |
| CN | No Ct | No Ct |

| **Standard curve parameters** | **Singleplex** | **Multiplex** |
|---|---|---|
| Slope of the line | -3,27 | 0,987 |
| Correlation coefficient (R²) | 0,997 | -3,46 |
| Efficiency | 102% | 94,3% |
| Detection limit | 10 copies | 10 copies |

| | | |
|---|---|---|
| *Not detected in any replicates ND: Not determined | | |

### Example 5. Multiplex qPCR kit for the detection of Candida albicans, C. parapsilopsis, C. glabrata, C. krusei, C. tropicalis, C. guilliermondii, Aspergillus flavus, A. fumigatus and the endogenous Bglo gene

### 1. Product description

The present example refers to a real-time multiplex PCR kit indicated for the detection of *Candida albicans, C. parapsilopsis, C. glabrata, C. krusei, C. tropicalis*, *C. guilliermondii*, *Aspergillus flavus*, *A. fumigatus* and the endogenous *Bglo* gene. Particularly, this example shows the usefulness of the present invention for carrying out the detection, quantification and identification of two or more different strains or species of microorganisms in one and the same biological sample using short probes.

The endogenous gene works as a DNA extraction control and PCR inhibition control. It allows verifying that the extraction process has been developed correctly and detecting false negatives due to PCR inhibition.

The kit contains the required reagents and enzymes mixed at ideal concentrations and designed to make diagnostic analysis easier.

The kit contains the following mixes:
- **Mix 1** provides a HEX-labeled LNA probe for the detection of C. *albicans,* and a Cy5-BHQ3®-labeled LNA probe for the detection of the *Bglo* gene (DNA extraction control/PCR inhibition control gene).
- **Mix 2.1** provides a FAM-labeled UPL probe for the detection of C. *parapsilopsis,* a Tex615/ROX-labeled LNA probe for the detection of *C*. *glabrata,* and a Cy5-BHQ3®-labeled LNA probe for the detection of the *Bglo* gene (DNA extraction control/PCR inhibition control gene).
- **Mix 2.2** provides a FAM-labeled UPL probe for the detection of *C*. *tropicalis*, a HEX-labeled LNA probe for the detection of *C*. *guilliermondii,* a Tex615/ROX-labeled LNA probe for the detection of *C*. *krusei and* a Cy5-BHQ3®-labeled LNA probe for the detection of the *Bglo* gene (DNA extraction control/PCR inhibition control gene).
- **Mix 3** provides a FAM-labeled UPL probe for the detection of *A. flavus*, a Tex615/ROX-labeled LNA probe for the detection of *A. fumigatus and* a Cy5-BHQ3®-labeled LNA probe for the detection of the *Bglo* gene (DNA extraction control/PCR inhibition control gene).

The channels through which fluorescence-related data is collected are described in detail in Table 1.

**Table 1: Reporters and Quenchers**

| Mix 1 | Reporter | Quencher |
|---|---|---|
| *Candida albicans* (probe 131) | HEX | Exiqon |
| *Bglo* gene (probe 144) | Cy5 | BHQ3® |

| Mix 2.1 | Reporter | Quencher |
|---|---|---|
| *C*. *parapsilopsis* (probe 67) | FAM | Roche |
| *C. glabrata* (probe 155) | Tex615/ROX | Exiqon |
| *Bglo* gene (probe 6) | Cy5 | BHQ3® |

| Mix 2.2 | Reporter | Quencher |
|---|---|---|
| *C*. *tropicalis* (probe 147) | FAM | Roche |
| *C*. *guilliermondii* (probe 129) | HEX | Exiqon |
| *C. krusei* (probe 4) | Tex 615/ROX | Exiqon |
| *Bglo* gene (probe 6) | Cy5 | BHQ3® |

| Mix 3 | Reporter | Quencher |
|---|---|---|
| *Aspergillus flavus* (probe 84) | FAM | Roche |
| *A. fumigatus* (probe 70) | Tex615/ROX | Exiqon |
| *Bglo* gene (probe 144) | Cy5 | BHQ3® |

The UPL/LNA probe as well as the forward (F) and reverse (R) primers have the sequences provided in detail below:

| **Syndrome** | **pathogen** | **Forward primer seq. (F)** | **Reverse primer seq. (R)** |
|---|---|---|---|
| **Fungal infection** | *C. albicans* | 5’- CGAGCTCAAGTAGACGTG TTGA-3’ | 5’- GGTAAATTCAAATCCAGAAA ATCG-3’ |
| **Fungal infection** | *C*. *parapsilopsis* | 5’- TGGTGTCAATTGTGGGAA AG-3’ | 5'- AACAGTTCCACCGCCAAC-3' |
| **Fungal infection** | *C. glabrata* | 5'- TTCACCTTCACCATCTTTA GGAA-3' | 5'- TGGTGTATTTTGTAGCGATT TCA-3' |
| **Fungal infection** | *C. tropicalis* | 5'- TCGTTATATTGTAAATACT GCTCATCC-3' | 5’- CATGTTGACTTATCAATGGA ATGTAAT-3’ |
| **Fungal infection** | *C*. *guilliermondii* | 5'- GCGAGTACTGGACCCAAC C-3' | 5’- AAACACCACAAGGGCGAAT- 3’ |
| **Fungal infection** | *C*. *krusei* | 5'- GCCCTATACCATCCATCA GC-3' | 5'- GAAACCAAGTGATGTCGCT ATCT-3' |
| **Fungal infection** | *A. flavus* | 5’- GGTGCCTACTTTTCCGACT TC-3’ | 5'- GGTGAAGGCACATTCCAAC T-3' |
| **Fungal infection** | *A. fumigatus* | 5’- CGGTCTACTCGGCTCAAT CT-3’ | 5'AGTGTCACTTCGAGCATA GGG-3' |

| Syndrome | pathogen | probe No. | Amplicon seq. | Amplicon (bp) | probe seq. |
|---|---|---|---|---|---|
| **Fungal infection** | *C*. *albicans* | 131 | CGAGCTCAAGTAGAC GTGTTGAAAAATGAG TTGGCTAAATATACT GGTGGTAGCGATTTT CTGGATTTGAATTTAC C | 77 | CTGGTGG T |
| **Fungal infection** | *C*. *parapsilopsis* | 67 | TGGTGTCAATTGTGG GAAAGACGATAATAC TCCAGCACTGCCGTT GGCGGTGGAACTGTT | 60 | CTCCAGC A |
| **Fungal infection** | *C. glabrata* | 155 | TTCACCTTCACCATCT TTAGGAATACCAATA AAAAATAGCACAATG AGCCCTTCATTGCCT TCCATGAAATCGCTA CAAAATACACCA | 88 | TTGCCTT C |
| **Fungal infection** | *C. tropicalis* | 147 | TCGTTATATTGTAAAT ACTGCTCATCCTGTT GATGGCGGTTGGGG ATTACATTCCATTGA TAAGTCAACATG | 72 | TTGATGG C |
| **Fungal infection** | *C*. *guilliermondii* | 129 | GCGAGTACTGGACCC AACCGAGCCTTTCCT TCTGGCTAACCATTC GCCCTTGTGGTGTTT | 60 | TTCTGGC T |
| **Fungal infection** | *C*. *krusei* | 4 | GCCCTATACCATCCA TCAGCGGATGCATTT GCTTCCATCTTCACT GAGCTTCCTGCAAAG ATAGCGACATCACTT GGTTTC | 81 | CTTCCTG C |
| **Fungal infection** | *A. flavus* | 84 | GGTGCCTACTTTTCC GACTTCTTGCAGCAG ACGAAAGGCAAACCT CCGAGTTGGAATGTG CCTTCACC | 68 | GCAGCA GA |
| **Fungal infection** | *A. fumigatus* | 70 | CGGTCTACTCGGCTC AATCTGCGGAGAATC CGGCGGCGGCTTTT GGTGTCCCTGGACGT GTCGCGTCCCCCTAT GCTCGAAGTGACACT | 89 | GGCGGC GG |

The probe as well as the primers for the Bglob gene have the sequences provided in detail below:

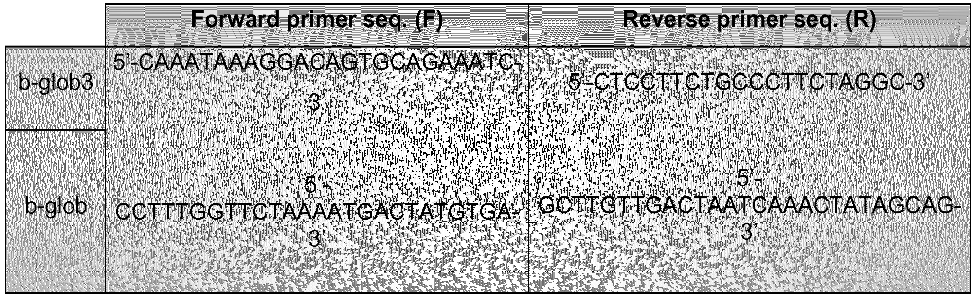

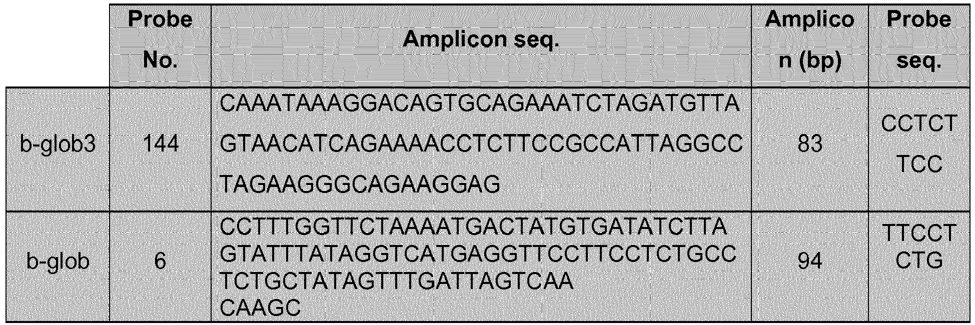

### 2. Singleplex assay (Comparative example)

### 2.1. Protocol validation by means of standards

### 2.1.1. Singleplex qPCR for the individual detection of Candida albicans (probe 131)/C. parapsilopsis (probe 67)/C. glabrata (probe 155)/C. tropicalis (probe 147)/C. guilliermondii (probe 129)/C. krusei (probe 4)lAspergillus flavus (probe 84)/A. fumigatus (probe 70)/Bglo gene (probe 144)/Bglo gene (probe 6)

### 2.1.1.1 Singleplex qPCR reaction

| Indicate no. of reactions | 1 | 1 |
|---|---|---|
| | | |

| Reagent (concentration)) | Volume (µl) | Volume (µl) |
|---|---|---|
| UPL/LNA probe (10 µM) | 0.5 | 0.5 |
| Primer-F (100 µM) | 0.15 | 0.15 |
| Primer-R (100 µM) | 0.15 | 0.15 |
| Master Probes | 10 | 10 |
| H20 | 4 | 4 |
| UNG | 0.2 | 0.2 |
| DNA | | 5 |

### 2.1.1.2. Thermal cycler program

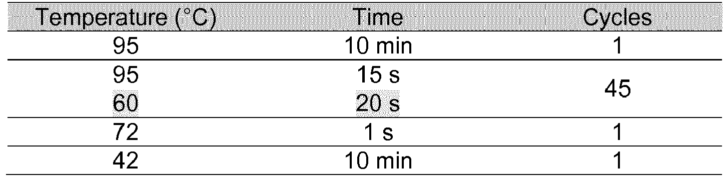

### 2.1.1.3. Detection limit of the target and amplification efficiency of each target in simplex format

To determine the detection limit of the target, 10-fold serial dilutions of a mixture of plasmid DNA (standard DNA) of the targets to be detected were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, "m" being the slope of the calibration line equation.

**2.1.1.3.1. Results**

| | Mean Ct obtained | | |
|---|---|---|---|
| Standard plasmid DNA | *Candida albicans* (probe 131) | *C. parapsilopsis* (probe 67) | *C. glabrata* (probe 155) |
| 10⁷ copies | 16.07 | 16.82 | 14.17 |
| 10⁶ copies | 19.63 | 20.57 | 18.14 |
| 10⁵ copies | 23.04 | 24.16 | 21.71 |
| 10⁴ copies | 26.51 | 27.29 | 25.15 |
| 10³ copies | 30.01 | 31.04 | 28.51 |
| 10² copies | 33.35 | 34.19 | 32.04 |
| 10 copies | 36.71 | 37.27 | 35.27 |
| 1 copy | No Ct | 39.81 | No Ct |
| CN | No Ct | No Ct | No Ct |

| | Mean Ct obtained | | |
|---|---|---|---|
| *C. tropicalis* | *C. tropicalis* (probe 147) | *C. guilliermondii* (probe 129) | |
| 10⁷ copies | 15.38 | 17 | |
| 10⁶ copies | 18.88 | 20.47 | |
| 10⁵ copies | 22.27 | 23.8 | |
| 10⁴ copies | 25.63 | 26.29 | |
| 10³ copies | 29.19 | 30.51 | |
| 10² copies | 32.58 | 34.89 | |
| 10 copies | 34.98 | 36.91 | |
| 1 copy | 38.77* | 37.86* | |
| CN | No Ct | No Ct | |

| | Mean Ct obtained | | |
|---|---|---|---|
| | *C. krusei* (probe 4) | *Aspergillus flavus* (probe 84) | *A. fumigatus* (probe 70) |
| 10⁷ copies | 16.42 | 16.59 | 16.89 |
| 10⁶ copies | 20.03 | 20.27 | 20.29 |
| 10⁵ copies | 23.53 | 23.59 | 23.86 |
| 10⁴ copies | 26.82 | 27.19 | 27.20 |
| 10³ copies | 30.46 | 30.37 | 30.58 |
| 10² copies | 33.44 | 33.68 | 33.73 |
| 10 copies | 37.50 | 36.53 | 35.77 |
| 1 copy | 40.73* | 37.95* | No Ct |
| CN | No Ct | No Ct | No Ct |

| | Mean Ct obtained | | |
|---|---|---|---|
| | *Bglo gene* (probe 6) | *Bglo* gene (probe 144) | |
| 10⁷ copies | 24.1 | 15.35 | |
| 10⁶ copies | 28.15 | ND | |
| 10⁷ copiers | 31.96 | 21.73 | |
| 10⁴ copies | 34.88 | 24.76 | |
| 10³ copies | 37.48 | 28.22 | |
| 10² copies | 42.485 | 31.92 | |
| 10 copies | No Ct | 34.32 | |
| 1 copy | No Ct | 37.06* | |
| CN | No Ct | No Ct | |

| | | | |
|---|---|---|---|
| Obs. High variability observed between replicates (*Bglo* gene (probe 6) *Not detected in any replicates ND: Not determined | | | |

**2.1.1.3.1.1. Candida albicans (probe 131)**

| Standard curve parameters obtained | |
|---|---|
| Slope of the line | -3.44 |
| Correlation coefficient (R²) | 0.999 |
| Efficiency | 95.3% |
| Detection limit: 10 copies | |

**2.1.1.3.1.2. C. parapsilopsis (probe 67)**

| Standard curve parameters obtained | |
|---|---|
| Slope of the line | -3.33 |
| Correlation coefficient (R²) | 0.997 |
| Efficiency | 99.5% |
| Detection limit: 1 copy | |

**2.1.1.3.1.3. C. glabrata (probe 155)**

| Standard curve parameters obtained | |
|---|---|
| Slope of the line | -3.49 |
| Correlation coefficient (R²) | 0.999 |
| Efficiency | 93.2% |
| Detection limit: 10 copies | |

**2.1.1.3.1.4. C. tropicalis (probe 147)**

| Standard curve parameters obtained | |
|---|---|
| Slope of the line | -3.34 |
| Correlation coefficient (R²) | 0.998 |
| Efficiency | 98.9% |
| Detection limit: 10 copies | |

**2.1.1.3.1.5. C. guilliermondii (probe 129)**

| Standard curve parameters obtained | |
|---|---|
| Slope of the line | -3.45 |
| Correlation coefficient (R²) | 0.999 |
| Efficiency | 99.0% |
| Detection limit: 10 copies | |

**2.1.1.3.1.6. C. krusei (probe 4)**

| Standard curve parameters obtained | |
|---|---|
| Slope of the line | -3.46 |
| Correlation coefficient (R²) | 0.999 |
| Efficiency | 94.4% |
| Detection limit: 10 copies | |

**2.1.1.3.1.7. Aspergillus flavus (probe 84)**

| Standard curve parameters Obtained | |
|---|---|
| Slope of the line | -3.35 |
| Correlation coefficient (R²) | 0.998 |
| Efficiency | 98.8% |
| Detection limit: 10 copies | |

**2.1.1.3.1.8. A. fumigatus (probe 70)**

| Standard curve parameters Obtained | |
|---|---|
| Slope of the line | -3.25 |
| Correlation coefficient (R²) | 0.996 |
| Efficiency | 102.8% |
| Detection limit: 10 copies | |

**2.1.1.3.1.9. Bglo gene Cy5 (probe 144)**

| Standard curve parameters Obtained | |
|---|---|
| Slope of the line | -3.27 |
| Correlation coefficient (R²) | 0.997 |
| Efficiency | 102% |
| Detection limit: 10 copies | |

**2.1.1.3.1.10. Bglo gene Cy5 (probe 6)**

| Standard curve parameters Obtained | |
|---|---|
| Slope of the line | -3.56 |
| Correlation coefficient (R²) | 0.994 |
| Efficiency | 90.9% |
| Detection limit: 100 copies | |

### 2.1.2. Mix 1: multiplex qPCR for the simultaneous detection of Candida albicans and the endogenous Bglo gene

**2.1.2.1. Multiplex qPCR reaction**

| Indicate no. of reactions | 1 | 1 |
|---|---|---|
| | | |

| Reagent (concentration) | Volume (µl) | Volume (µl) |
|---|---|---|
| Probe 144CY5 (10 µM) | 0.5 | 0.5 |
| Bglo144F (100 µM) | 0.15 | 0.15 |
| Bglo144R (100 µM) | 0.15 | 0.15 |
| Probe UPL131 (100 µM) HEX | 0.5 | 0.5 |
| Calbicans131-F (20 µM) | 0.15 | 0.15 |
| Calbicans131-R (20 µM) | 0.15 | 0.15 |
| Master Probes | 10 | 10 |
| H20 | 3.2 | 3.2 |
| UNG | 0.2 | 0.2 |
| DNA | | 5 |
| Final volume | | 20 |

### 2.1.2.2. Thermal cycler program

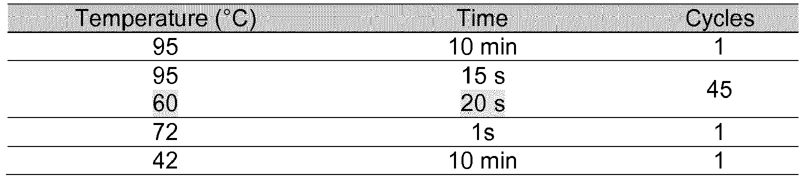

### 2.1.2.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m)-1, m being the slope of the calibration line equation.

### 2.1.2.3.1. Results

**2.1.2.3.1.1. Candida albicans (probe 131)**

| **Standard plasmid DNA** | ***Candida albicans* (probe 131)** | |
|---|---|---|
| | **Singleplex** | **Multiplex** |
| | **Mean Ct** | **Mean Ct** |
| 10⁷ copies | 16.07 | 15.18 |
| 10⁶ copies | 19.63 | 18.69 |
| 10⁵ copies | 23.04 | 22.4 |
| 10⁴ copies | 26.51 | 25.65 |
| 10³ copies | 30.01 | 29.32 |
| 10² copies | 33.35 | 32.53 |
| 10 copies | 36.71 | 36.90 |
| 1 copy | No Ct | 38.54* |
| CN | No Ct | No Ct |

| **Standard curve parameters** | **Singleplex** | **Multiplex** |
|---|---|---|
| Slope of the line | -3.44 | -3.56 |
| Correlation coefficient (R²) | 0.999 | 0.998 |
| Efficiency | 102% | 99.2% |
| Detection limit | 10 copies | 10 copies |

| | | |
|---|---|---|
| *Not detected in any replicas | | |

**2.1.2.3.1.2. Endogenous Bglo gene (144)**

| **Standard plasmid DNA singleplex** | **Endogenous *Bglo* gene (probe 131)** | |
|---|---|---|
| | **Singleplex** | **Multiplex** |
| | **Mean Ct** | **Mean Ct** |
| 10⁷ copies | 15.35 | 15.7 |
| 10⁶ copies | ND | 19.14 |
| 10⁵ copies | 21.73 | 22.86 |
| 10⁴ copies | 24.76 | 26.04 |
| 10³ copies | 28.22 | 29.70 |
| 10² copies | 31.92 | 32.67 |
| 10 copies | 34.32 | 36.5 |
| 1 copy | 37.06* | 38.33* |
| CN | No Ct | No Ct |

| **Standard curve parameters** | **Singleplex** | **Multiplex** |
|---|---|---|
| Slope of the line | -3.27 | -3.34 |
| Correlation coefficient (R²) | 0.997 | 0.993 |
| Efficiency | 102% | 99.2% |
| Detection limit | 10 copies | 10 copies |

| | | |
|---|---|---|
| *Not detected in any replicas | | |

### 2.1.3. Mix 2.1: multiplex qPCR for the simultaneous detection of C. parapsilopsis (probe 67)/C. glabrata (probe 155)/Bglo (probe 6)

### 2.1.3.1. Multiplex qPCR reaction

| Indicate no. of reactions | 1 | 1 |
|---|---|---|
| | | |

| Reagent (concentration)) | Volume (µl) | Volume (µl) |
|---|---|---|
| Probe 144CY5 (10 µM) | 0.5 | 0.5 |
| Bglo144F (100 µM) | 0.15 | 0.15 |
| Bglo144R (100 µM) | 0.15 | 0.15 |
| Probe UPL131 (10 µM) HEX | 0.5 | 0.5 |
| Calbicans131-F (20 µM) | 0.15 | 0.15 |
| Calbicans131-R (20 µM) | 0.15 | 0.15 |
| Master Probes | 10 | 10 |
| H20 | 3.2 | 3.2 |
| UNG | 0.2 | 0.2 |
| DNA | | 5 |
| Final volume | | 20 |

### 2.1.3.2. Thermal cycler program

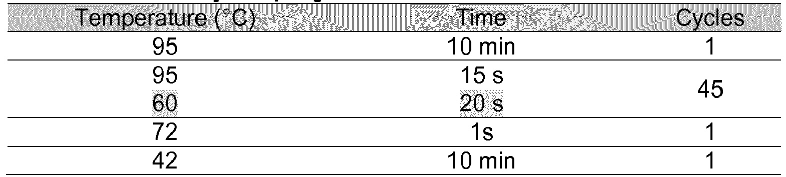

### 2.1.3.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m)-1, m being the slope of the calibration line equation.

### 2.1.1.3.1.3. C. parapsilopsis (probe 67)

| **Standard plasmid DNA** | ***C. parapsilopsis* (probe 67)** | |
|---|---|---|
| | **Singleplex** | **Multiplex** |
| | **Mean Ct** | **Mean Ct** |
| 10⁷ copies | 16.82 | 15.72 |
| 10⁶ copies | 20.57 | 18.37 |
| 10⁵ copies | 24.16 | 21.54 |
| 10⁴ copies | 27.29 | 25.44 |
| 10³ copies | 31.04 | 28.55 |
| 10² copies | 34.19 | 31.98 |
| 10 copies | 37.27 | 35.66 |
| 1 copy | 39.81 | 38.12* |
| CN | No Ct | No Ct |

| **Standard curve parameters** | **Singleplex** | **Multiplex** |
|---|---|---|
| Slope of the line | -3,33 | -3,32 |
| Correlation coefficient (R²) | 0,997 | 0,997 |
| Efficiency | 99,5% | 99,7% |
| Detection limit | 1 copy | 10 copies |

| | | |
|---|---|---|
| * Not detected in any replicas. | | |

**2.1.3.3.1.1. C. glabrata (probe 155)**

| **Standard plasmid DNA** | ***C. glabrata* (probe 155)** | |
|---|---|---|
| | **singleplex** | **Multiplex** |
| | **Mean Ct** | **Mean Ct** |
| 10⁷ copies | 14.17 | 12.61 |
| 10⁶ copies | 18.14 | 16.25 |
| 10⁵ copies | 21.71 | 19.57 |
| 10⁴ copies | 25.15 | 23.14 |
| 10³ copies | 28.51 | 26.34 |
| 10² copies | 32.04 | 30.02 |
| 10 copies | 35.27 | 32.97 |
| 1 copy | No Ct | 25.89 |
| CN | No Ct | No Ct |

| **Standard curve parameters** | **Singleplex** | **Multiplex** |
|---|---|---|
| Slope of the line | -3,49 | -3,38 |
| Correlation coefficient (R²) | 0,999 | 0,998 |
| Efficiency | 93,2% | 97,4% |
| Detection limit | 10 copies | 1 copy |

| | | |
|---|---|---|
| * Not detected in any replicas. | | |

**2.1.3.3.1.2. Bglo (probe 6)**

| **Standard plasmid DNA** | **Endogenous gene (probe 6)** | |
|---|---|---|
| | **Singleplex** | **Multiplex** |
| | **Mean Ct** | **Mean Ct** |
| 10⁷ copies | 24.1 | 14.08 |
| 10⁶ copies | 28.15 | 18.24 |
| 10⁵ copies | 31.96 | 22.21 |
| 10⁴ copies | 34.88 | 25.36 |
| 10³ copies | 37.48 | 28.86 |
| 10² copies | 42.485 | 32.66 |
| 10 copies | No Ct | 35.89 |
| 1 copy | No Ct | 39.66* |
| CN | No Ct | No Ct |

| **Standard curve parameter** | **Singleplex** | **Multiplex** |
|---|---|---|
| Slope of the line | -3,56 | -3,60 |
| Correlation coefficient (R²) | 0,994 | 0,997 |
| Efficiency | 90,9% | 89,6% |
| Detection limit | 100 copies | 10 copy |

| | | |
|---|---|---|
| * Not detected in any replicas. | | |

### 2.1.4. Mix 2.2: multiplex qPCR for the simultaneous detection of C. tropicalis (probe 147)/C. guilliermondii (probe 129)/C. krusei (probe 4)/Bglo gene (probe 6)

| Indicate no. of reactions | 1 | |
|---|---|---|
| | | |

| Reagent (concentration)) | Volume (µl) | Check |
|---|---|---|
| Probe C. *tropicalis* 147 FAM (10 µM) | 0.5 | 0.5 |
| Probe *C. guilliermondii* 129 HEX (10 µM) | 0.5 | 0.5 |
| Probe *C krusei* 4 tex615 (10 µM) | 0.5 | 0.5 |
| Probe Bglo6Cy5 (10 µM) | 0.5 | 0.5 |
| C. *tropicalis* 147 F (100 (µM) | 0.15 | 0.15 |
| C. *tropicalis* 147 R (100 µM) | 0.15 | 0.15 |
| C. *guilliermondii* 129F (100 µM) | 0.15 | 0.15 |
| *C .guilliermondii* 129R (100 µM) | 0.15 | 0.15 |
| C. *krusei* 4F (100 µM) | 0.15 | 0.15 |
| C. *krusei* 4R (100 µM) | 0.15 | 0.15 |
| Bglo6F (100 µM) | 0.15 | 0.15 |
| Bglo6R (100 µM) | 0.15 | 0.15 |
| Master Mix Roche | 10 | 10 |
| H20 | 1.6 | 1.6 |
| UNG | 0.2 | 0.2 |
| DNA | 5 | 5 |
| Final volume | 15 | 15 |

### 2.1.4.2. Thermal cycler program

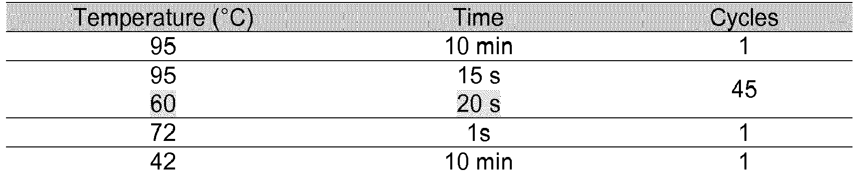

### 2.1.4.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m)-1, m being the slope of the calibration line equation.

### 2.1.4.3.1. Results

**2.1.4.3.1.1. C. tropicalis (probe 147)**

| **Standard plasmid DNA** | ***C. tropicalis* (probe 147)** | |
|---|---|---|
| | **Singleplex** | **Multiplex** |
| | **Mean Ct** | **Mean Ct** |
| 10' copies | 15.38 | 14.81 |
| 10⁶ copies | 18.88 | 17.87 |
| 10⁵ copies | 22.27 | 21.28 |
| 10⁴ copies | 25.63 | 24.85 |
| 10³ copies | 29.19 | 28.52 |
| 10² copies | 32.58 | 31.38 |
| 10 copies | 34.98 | 35.2 |
| 1 copy | 38.77* | 35.80* |
| CN | No Ct | No Ct |

| **Standard curve parameters** | **Singleplex** | **Multiplex** |
|---|---|---|
| Slope of the line | -3,34 | -3,40 |
| Correlation coefficient (R²) | 0,998 | 0,998 |
| Efficiency | 98,9% | 86,6% |
| Detection limit | 10 copies | 10 copy |

| | | |
|---|---|---|
| * Not detected in any replicates. ND: Not determined | | |

### 2.1.4.3.1.2. C. guilliermondii (probe 129)

| **Standard plasmid DNA** | ***C. guilliermondii* (probe 129)** | |
|---|---|---|
| | **Singleplex** | **Multiplex** |
| | **Mean Ct** | **Mean Ct** |
| 10⁷ copies | 17 | 16.39 |
| 10⁶ copies | 20.47 | 19.49 |
| 10⁵ copies | 23.8 | 22.70 |
| 10⁴ copies | 26.99 | 26.26 |
| 10³ copies | 30.51 | 29.48 |
| 10² copies | 34.89 | 32.83 |
| 10 copies | 36.91 | 35.82 |
| 1 copy | 37.86* | 39.49* |
| CN | No Ct | No Ct |

| **Standard curve parameters** | **Singleplex** | **Multiplex** |
|---|---|---|
| Slope of the line | -3,45 | -3,27 |
| Correlation coefficient (R²) | 0,999 | 0,999 |
| Efficiency | 99,0% | 101,9% |
| Detection limit | 10 copies | 10 copies |

| | | |
|---|---|---|
| * Not detected in any replicates. ND: Not determined | | |

**2.1.4.3.1.3. C. krusei (probe 4)**

| **Standard plasmid DNA** | ***C. krusei* (probe 4)** | |
|---|---|---|
| | **Singleplex** | **Multiplex Mean Ct** |
| | **Mean Ct** | |
| 10⁷ copies | 16.42 | 14.39 |
| 10⁶ copies | 20.03 | 17.82 |
| 10⁵ copies | 23.53 | 21.25 |
| 10⁴ copies | 26.82 | 24.57 |
| 10^{d} copies | 30.46 | 27.98 |
| 10² copies | 33.44 | 31.41 |
| 10 copies | 37.50 | 34.49 |
| 1 copy | 40.73* | 36.39* |
| CN | No Ct | No Ct |

| **Standard curve parameters** | **Singleplex** | **Multiplex** |
|---|---|---|
| Slope of the line | -3,46 | -3,36 |
| Correlation coefficient (R²) | 0,999 | 0,999 |
| Efficiency | 94,4% | 98,2% |
| Detection limit | 10 copies | 10 copies |

| | | |
|---|---|---|
| * Not detected in any replicas. ND: Not determined | | |

**2.1.4.3.1.4. Bglo (probe 6)**

| **Standard plasmid DNA** | **Endogenous gene (probe 6)** | |
|---|---|---|
| | **Singloplex** | **Multiplex** |
| | **Mean Ct** | **Mean Ct** |
| 10⁷ copies | 24.1 | 14.01 |
| 10⁶ copies | 28.15 | 18.07 |
| 10⁵ copies | 31.96 | 22.88 |
| 10⁴ copies | 34.88 | 26.21 |
| 10³ copies | 37.48 | 30.40 |
| 10² copies | 42.485 | 33.1 |
| 10 copies | No Ct | 37 |
| 1 copy | No Ct | 44.03 |
| CN | No Ct | No Ct |

| **Standard curve parameters** | **Singleplex** | **Multiplex** |
|---|---|---|
| Slope of the line | -3,56 | -3,8 |
| Correlation coefficient (R²) | 0,994 | 0,993 |
| Efficiency | 90,9% | 83,2% |
| Detection limit | 100 copies | 10 copies |

| | | |
|---|---|---|
| * Not detected in any replicas. | | |

### 2.1.5. Mix 3: multiplex qPCR for the simultaneous detection of Aspergillus flavus (probe 84)/A. fumigatus (probe 70)/Bglo gene (probe 144)/Bglo gene (probe 6)

**2.1.5.1. Multiplex qPCR reaction**

| Indicate no. of reactions | 1 | |
|---|---|---|
| | | |

| Reagent (concentration) | Volume (µl) | Check |
|---|---|---|
| Probe *A. flavus* 84 FAM (10 µM) | 0.5 | 0.5 |
| Probe *A. fumigatus* 70tex615 (10 µM) | 0.5 | 0.5 |
| Probe Bglo144Cy5 (10 µM) | 0.5 | 0.5 |
| *A. flavus* 84F (100 µM) | 0.15 | 0.15 |
| *A. flavus* 84R (100 µM) | 0.15 | 0.15 |
| *A. fumigatus* 70F (100 µM) | 0.15 | 0.15 |
| *A. fumigatus* 70R (100 µM) | 0.15 | 0.15 |
| Bqlo144F (100 µM) | 0.15 | 0.15 |
| Bglo144R (100 µM) | 0.15 | 0.15 |
| Master Mix Roche | 10 | 10 |
| H2O | 2.4 | 2.4 |
| UNG | 0.2 | 0.2 |
| DNA | 5 | 5 |
| Final volume | 5 | 5 |

### 2.1.5.2. Thermal cycler program

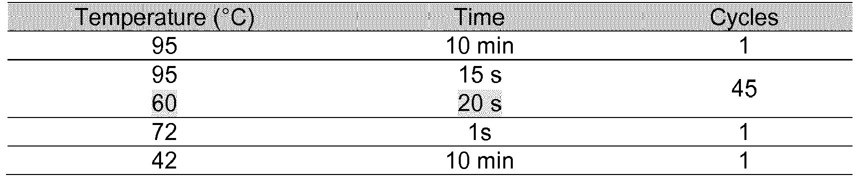

### 2.1.5.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m)-1, m being the slope of the calibration line equation.

### 2.1.5.3.1. Results

**2.1.5.3.1.1. Aspergillus flavus (probe 84)**

| **Standard plasmid DNA** | ***Aspergillus flavus* (probe 84)** | |
|---|---|---|
| | **Singleplex** | **Multiplex** |
| | **Mean Ct** | **Mean Ct** |
| 10⁷ copies | 16.59 | 15.95 |
| 10⁶ copies | 20.27 | 19.05 |
| 10⁵ copies | 23.59 | 22.72 |
| 10⁴ copies | 27.19 | 26.35 |
| 10³ copies | 30.37 | 29.84 |
| 10² copies | 33.68 | 33.14 |
| 10 copies | 36.53 | 36.52 |
| 1 copy | 37.95 | No Ct |
| CN | No Ct | No Ct |

| **Standard curve parameters** | **Singleplex** | **Multiplex** |
|---|---|---|
| Slope of the line | -3,35 | -3,46 |
| Correlation coefficient (R²) | 0,998 | 0,998 |
| Efficiency | 98,8% | 94,4% |
| Detection limit | 10 copies | 10 copies |

| | | |
|---|---|---|
| * Not detected in any replicas. ND: Not determined | | |

**2.1.5.3.1.2. A. fumigatus (probe 70)**

| **Standard plasmid DNA** | ***A. fumigatus* (probe 70)** | |
|---|---|---|
| | **Singleplex** | **Multiplex** |
| | **Mean Ct** | **Mean Ct** |
| 10⁷ copies | 16.89 | 14.24 |
| 10⁶ copies | 20.29 | 17.35 |
| 10⁵ copies | 23.86 | 20.64 |
| 10⁴ copies | 27.20 | 24.05 |
| 10³ copies | 30.58 | 27.48 |
| 10² copies | 33.73 | 30.89 |
| 10 copies | 35.77 | 34.14 |
| 1 copy | No Ct | 35.95* |
| CN | No Ct | No Ct |

| **Standard curve parameters** | **Singleplex** | **Multiplex** |
|---|---|---|
| Slope of the line | -3,25 | -3,34 |
| Correlation coefficient (R²) | 0,996 | 0,999 |
| Efficiency | 102,8% | 99,1% |
| Detection limit | 10 copies | 10 copies |

| | | |
|---|---|---|
| * Not detected in any replicas. ND: Not determined. | | |

**2.1.5.3.1.3. Bglo gene (probe 144)**

| **Standard plasmid DNA** | ***Bglo* gene (probe 144)** | |
|---|---|---|
| | **Singleplex** | **Multiplex** |
| | **Mean Ct** | **Mean Ct** |
| 10⁷ copies | 15,35 | 14.35 |
| 10⁶ copies | ND | 17.44 |
| 10⁵ copies | 21,73 | 20.99 |
| 10⁴ copies | 24,76 | 24.44 |
| 10³ copies | 28,22 | 27.68 |
| 10² copies | 31,92 | 30.59 |
| 10 copies | 34,32 | 34.07 |
| 1 copy | 37,06* | 35.77* |
| CN | No Ct | No Ct |

| **Standard curve parameters** | **Singleplex** | **Multiplex** |
|---|---|---|
| Slope of the line | -3,27 | -3,29 |
| Correlation coefficient (R²) | 0,997 | 0,999 |
| Efficiency | 102% | 101,2% |
| Detection limit | 10 copies | 10 copies |

| | | |
|---|---|---|
| * Not detected in any replicas. ND: Not determined | | |

### Example 6. Multiplex qPCR kit for the detection of VHS-8, herpesvirus 4 (Epstein Barr), cytomegalovirus, adenovirus and the endogenous Bglo gene

The present example refers to a real-time PCR kit indicated for the detection of VHS-8, herpesvirus 4 (Epstein Barr), cytomegalovirus, adenovirus and the endogenous *Bglo* gene. Particularly, this example shows the usefulness of the present invention for carrying out the detection, quantification and identification of two or more different strains or species of microorganisms in one and the same biological sample using short probes.

### 1. Product description

The kit 10 is a real-time multiplex PCR kit indicated for the detection of VHS-8, herpesvirus 4 (Epstein Barr), cytomegalovirus, adenovirus and the endogenous *Bglo* gene. The endogenous gene works as a DNA extraction control and PCR inhibition control. It allows verifying that the extraction process has been developed correctly and detecting false negatives due to PCR inhibition.

The kit contains the required reagents and enzymes mixed at ideal concentrations and designed to make diagnostic analysis easier.

The kit contains the following mixes:
- **Mix 1** provides a FAM-labeled LNA probe for the detection of VHS-8, a HEX-labeled LNA probe for the detection of herpesvirus 4 (Epstein Barr) and a Cy5-BHQ3®-labeled LNA probe for the detection of the *Bglo* gene (DNA extraction control/PCR inhibition control gene).
- **Mix2** provides a FAM-labeled LNA probe for the detection of adenovirus, a HEX-labeled LNA probe for the detection of cytomegalovirus and a Cy5-BHQ3®-labeled LNA probe for the detection of the *Bglo* gene (DNA extraction control/PCR inhibition control gene).

The channels through which fluorescence-related data is collected are described in detail in Table 1.

**Table 1: Reporters and Quenchers**

| Mix 1 | Reporter | Quencher |
|---|---|---|
| VHs-8 | FAM | Roche |
| Herpesvirus 4 (Epstein Barr) | HEX | Exiqon |
| *Bglo* gene | Cy5 | BHQ3® |

| | Reporter | Quencher |
|---|---|---|
| Adenovirus | FAM | Roche |
| Cytomegalovirus | HEX | Exiqon |
| *Bglo* gene | Cy5 | BHQ3® |
| *Bglo* gene (probe 6) | Cy5 | BHQ3® |

The UPL/LNA probe as well as the forward (F) and reverse (R) primers have the sequences provided in detail below:

| **Syndrome** | **pathogen** | **Forward primer seq**. | **Reverse primer seq**. |
|---|---|---|---|
| ***Virosis in transplant recipients*** | VHS-8 | 5'- GACGACCCGTGCAAGATTAT -3' | 5'- AGGAGGTGGAAGAGCAGGA -3' |
| ***Virosis in transplant recipients*** | Herpesvirus 4 | 5'- GCCCAGAGATGGTGTTGC-3' | 5’- GTACTGCTCGCTGGCAAAG- 3’ |
| ***Virosis in transplant recipients*** | Cytomegaloviru s | 5'- CTTGTTCAGCTTCGGTGTCC- 3' | 5'- CTCGAACCCAGGGACGTAT- 3' |
| ***Virosis in transplant recipients*** | Adenovirus | 5'- ACCACCGTCAGTGAAAACG- 3' | 5'- TTACTGACGCCAGACGCC-3' |

| **Syndrome** | **Pathogen** | **Probe No.** | **Amplicon seq.** | **Amplicon (bp)** | **Probe seq.** |
|---|---|---|---|---|---|
| ***Virosis in transplant recipients*** | VHS-8 | 150 | GACGACCCGTGCAAGA TTATGGGCTCTTCCAC CGTCTCCTGCTCCTGC TGTTCCACCCCCTGCT GCTCCTGCTCTTCCAC CTCCT | 85 | TGCTG TTC |
| ***Virosis in transplant recipients*** | Herpesvirus 4 | 40 | GCCCAGAGATGGTGTT GCGCCAGTCGCTGCG CCTGCTGCTGGGAACC TGGTCCTCCTTTG CCAGCGAGCAGTAC | 74 | GCCTG CTG |
| ***Virosis in transplant recipients*** | Cytomegalovirus | 40 | CTTGTTCAGCTTCGGT GTCCAGGTAGCCTGCT GTGTGTACCTGAGGTG GATACGTCCCTGGGTT CGAG | 68 | GCCTG CTG |
| ***Virosis in transplant recipients*** | Adenovirus | 108 | ACCACCGTCAGTGAAA ACGTTCCTGCTCTCAC AGATCACGGACCCTG CCGTTACGCAGCAGTA TCCGGGGAGTCCAGC GCGTGACCGTTACTGA CGCCAGACGCC | 86 | CTGGG CAA |

The probe as well as the primers for the Bglob gene have the sequences provided in detail below:

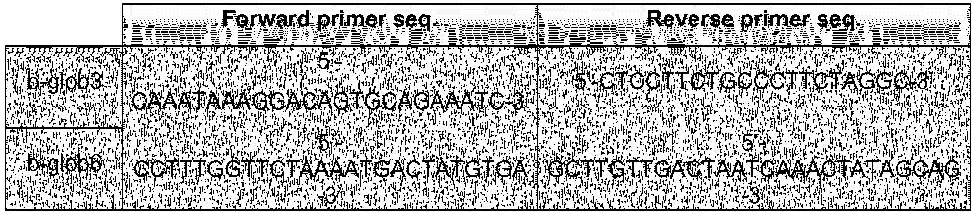

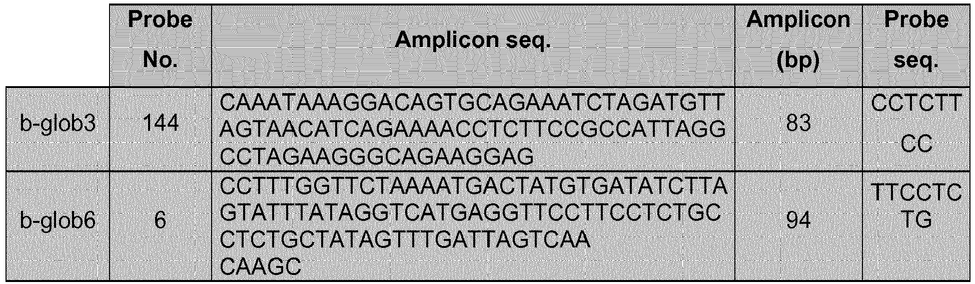

### 2. Singleplex assay (Comparative example)

### 2.1. Protocol validation by means of standards

### 2.1.1. Singleplex qPCR for the individual detection of VHS-8 (probe 150), herpesvirus 4 (Epstein Barr) (probe 40), cytomegalovirus (probe 40), adenovirus (probe 108) and the endogenous Bglo gene (probe 144 in Mix 1 and probe 6 in Mix 2)

**2.1.1.1. Singleplex qPCR reaction**

| Indicate no. of reactions | 1 | 1 |
|---|---|---|
| | | |

| Reagent (concentration) | Volume (µl) | Volume (µl) |
|---|---|---|
| UPL/LNA probe (10 µM) | 0.5 | 0.5 |
| Primer-F (100 µM) | 0.15 | 0.15 |
| Primer-R (100 µM) | 0.15 | 0.15 |
| Master Probes | 10 | 10 |
| H2O | 4 | 4 |
| UNG | 0.2 | 0.2 |
| DNA | | 5 |

### 2.1.1.2. Thermal cycler program

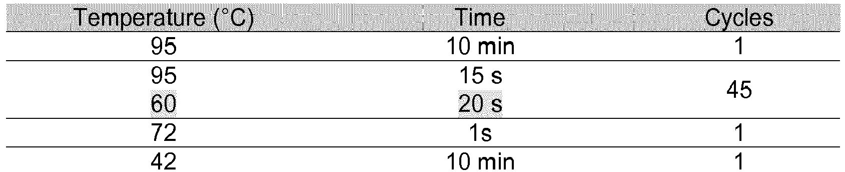

### 2.1.1.3. Detection limit of the target and amplification efficiency of each target in simplex format

To determine the detection limit of the target, 10-fold serial dilutions of a mixture of plasmid DNA (standard DNA) of the targets to be detected were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, "m" being the slope of the calibration line equation.

**2.1.1.3.1. Results**

| | Mean Ct obtained | | |
|---|---|---|---|
| Standard plasmid DNA | VHS-8 (probe 150) | Herpesvirus 4 (Epstein Barr) (probe 40) | Endogenous *Bglo* gene (probe 144) |
| 107 copies | 15.96 | 18.21 | 15,35 |
| 106 copies | 19.07 | 21.02 | ND |
| 105 copies | 22.22 | 24.42 | 21.73 |
| 104 copies | 25.72 | 27.85 | 24,76 |
| 103 copies | 29.12 | 31.21 | 28,22 |
| 102 copies | 32.74 | 34.58 | 31,92 |
| 10 copies | 35.62 | 38.06 | 34,32 |
| 1 copy | No Ct | No Ct | 37,06* |
| CN | No Ct | No Ct | No Ct |

| | Mean Ct obtained | | |
|---|---|---|---|
| | Cytomegalovirus (probe 140) | Adenovirus (probe 108) | Endogenous Bglo gene (probe 6) |
| 107 copies | 14.47 | 14.11 | 24.1 |
| 106 copies | 17.73 | 17.05 | 28.15 |
| 105 copies | 20.97 | 20.46 | 31.96 |
| 104 copies | 24.47 | 24.12 | 34.88 |
| 103 copies | 27.78 | 27.88 | 37.48 |
| 102 copies | 31.18 | 30.86 | 42.485 |
| 10 copies | 34.25 | 34.59 | No Ct |
| 1 copy | No Ct | No Ct | No Ct |
| CN | No Ct | No Ct | No Ct |

| | | | |
|---|---|---|---|
| Obs. High variability observed between replicates (*Bglo* gene (probe 6) *Not detected in any replicates ND: Not determined | | | |

**2.1.1.3.1.1. VHS-8 (probe 150)**

| **Standard curve parameters Obtained** | |
|---|---|
| Slope of the line | -3.32 |
| Correlation coefficient (R²) | 0.997 |
| Efficiency | 99.7% |
| Detection limit: 10 copies | |

**2.1.1.3.1.2. Herpesvirus 4 (Epstein Barr) (probe 40)**

| **Standard curve parameters Obtained** | |
|---|---|
| Slope of the line | -3.33 |
| Correlation coefficient (R²) | 0.998 |
| Efficiency | 99.4% |
| Detection limit: 10 copies | |

**2.1.1.3.1.3. Cytomegalovirus (probe 40)**

| **Standard curve parameters Obtained** | |
|---|---|
| Slope of the line | -3.32 |
| Correlation coefficient (R²) | 0.999 |
| Efficiency | 100% |
| Detection limit: 10 copies | |

**2.1.1.3.1.4. Adenovirus (probe 108)**

| **Standard curve parameters Obtained** | |
|---|---|
| Slope of the line | -3.44 |
| Correlation coefficient (R²) | 0.999 |
| Efficiency | 95.2% |
| Detection limit: 10 copies | |

**2.1.1.3.1.5. Bglo gene Cy5 (probe 144)**

| **Standard curve parameters Obtained** | |
|---|---|
| Slope of the line | -3.27 |
| Correlation coefficient (R²) | 0.997 |
| Efficiency | 102% |
| Detection limit: 10 copies | |

**2.1.1.3.1.6. Bglo gene Cy5 (probe 6)**

| **Standard curve parameters Obtained** | |
|---|---|
| Slope of the line | -3.56 |
| Correlation coefficient (R²) | 0.994 |
| Efficiency | 90,9% |
| Detection limit: 100 copies | |

### 2.1.2. Mix 1: multiplex qPCR for the simultaneous detection of VHS-8 (probe 150), herpesvirus 4 (Epstein Barr) (probe 40) and the endogenous Bglo gene (probe 144)

### 2.1.2.1. Multiplex qPCR reaction

| Indicate no. of reactions | 1 | |
|---|---|---|
| | | |

| Reagent (concentration) | Volume (µl) | Check |
|---|---|---|
| VHS8 probe 150 FAM (10 µM) | 0.5 | 0.5 |
| VHS-4 (Epstein Barr) probe 40 HEX (10 µM) | 0.5 | 0.5 |
| Probe Bglo144Cy5(10 µ) | 0.5 | 0.5 |
| VH8-150 F (100 µM) | 0.15 | 0.15 |
| VH8-150 R (100 µM) | 0.15 | 0.15 |
| EB 40 F (100 µM) | 0.15 | 0.15 |
| EB 40 R (100 µM) | 0.15 | 0.15 |
| Bglo144F (100 µM) | 0.15 | 0.15 |
| Bglo144R (100 µM) | 0.15 | 0.15 |
| Master Mix Roche | 10 | 10 |
| H2O | 2.4 | 2.4 |
| UNG | 0.2 | 0.2 |
| DNA | 5 | 5 |
| Final volume | 15 | 15 |

### 2.1.2.2. Thermal cycler program

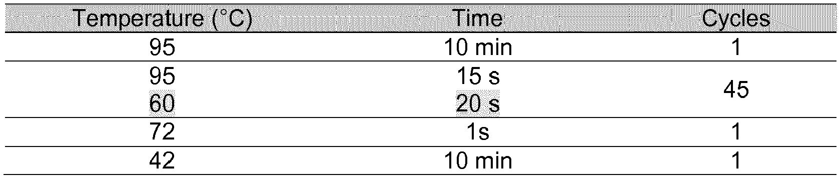

### 2.1.2.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m)-1, m being the slope of the calibration line equation.

### 2.1.2.3.1. Results

### 2.1.2.3.1.1. VHS-8 (probe 150)

| **Standard plasmid DNA** | **VHS-8 (probe 150)** | |
|---|---|---|
| | **Singleplex** | **Multiplex** |
| | **Mean Ct** | **Mean Ct** |
| 10⁷ copies | 15,96 | 18.43 |
| 10⁶ copies | 19.07 | 20.82 |
| 10⁵ copies | 22.22 | 24.70 |
| 10⁴ copies | 25.72 | 28.24 |
| 10³ copies | 29.12 | 31.49 |
| 10² copies | 32.74 | 34.67 |
| 10 copies | 35.62 | 38.84 |
| 1 copy | No Ct | No Ct |
| CN | No Ct | No Ct |

| **Standard curve parameters** | **Singleplex** | **Multiplex** |
|---|---|---|
| Slope of the line | -3,32 | -3,41 |
| Correlation coefficient (R²) | 0,997 | 0,993 |
| Efficiency | 99.7% | 96.1% |
| Detection limit | 10 copies | 10 copies |

| | | |
|---|---|---|
| * Not detected in any replicas. ND: Not determined | | |

**2.1.1.3.1.3. Herpesvirus 4 (Epstein Barr) (probe 40)**

| **Standard plasmid DNA** | **Epstein Barr (probe 40)** | |
|---|---|---|
| | **Singleplex** | **Multiplex** |
| | **Mean Ct** | **Mean Ct** |
| 10⁷ copies | 18.21 | 17.01 |
| 10⁶ copies | 21.02 | 19.50 |
| 10⁵ copies | 24.42 | 22.77 |
| 10⁴ copies | 27.85 | 26.25 |
| 10³ copies | 31.21 | 29.62 |
| 10² copies | 34.58 | 32.98 |
| 10 copies | 38.06 | 37.85 |
| 1 copy | No Ct | No Ct |
| CN | No Ct | No Ct |

| **Standard curve parameters** | **Singleplex** | **Multiplex** |
|---|---|---|
| Slope of the line | -3,33 | -3,44 |
| Correlation coefficient (R²) | 0,998 | 0,993 |
| Efficiency | 99.4% | 95.3% |
| Detection limit | 10 copies | 10 copies |

| | | |
|---|---|---|
| * Not detected in any replicas. | | |

### 2.1.2.3.1.2. Endogenous gene Bglo (144)

| **Standard plasmid DNA** | **Endogenous Bglo gene (144)** | |
|---|---|---|
| | **Singleplex** | **Multiplex** |
| | **Mean Ct** | **Mean Ct** |
| 10⁷ copies | 15,35 | 14.58 |
| 10⁶ copies | ND | 17.28 |
| 10⁵ copies | 21,73 | 20.72 |
| 10⁴ copies | 24,76 | 23.91 |
| 10³ copies | 28,22 | 27.34 |
| 10² copies | 31,92 | 30.69 |
| 10 copies | 34,32 | 34.43 |
| 1 copy | 37,06* | No Ct |
| CN | No Ct | No Ct |

| **Standard curve parameters** | **Singleplex** | **Multiplex** |
|---|---|---|
| Slope of the line | -3,27 | -3,32 |
| Correlation coefficient (R²) | 0,997 | 0,997 |
| Efficiency | 102% | 100% |
| Detection limit | 10 copies | 10 copies |

| | | |
|---|---|---|
| * Not detected in any replicas. ND: Not determined | | |

### 2.1.3. Mix 2: multiplex qPCR for the simultaneous detection of cytomegalovirus (probe 40)/adenovirus (probe 108)/Bglo (probe 6)

### 2.1.3.1. Multiplex qPCR reaction

| Indicate no. of reactions | 1 | |
|---|---|---|
| | | |

| Reagent (concentration) | Volume (µl) | Check |
|---|---|---|
| Cytomegalovirus (herpV5) probe 40 HEX (10 µM) | 0.5 | 0.5 |
| Adenovirus 108 FAM (10 µM) | 0.5 | 0.5 |
| Probe Bglo Cy5 (10 µM) | 0.5 | 0.5 |
| Cytomegalo 40F (100 µM) | 0.15 | 0.15 |
| Cytomegalo 40R (100 µM) | 0.15 | 0.15 |
| Adenovirus 108F (100 µM) | 0.15 | 0.15 |
| Adenovirus 108R (100 µM) | 0.15 | 0.15 |
| Bglo 6F (100 µM) | 0.15 | 0.15 |
| Bglo 6R (100 µM) | 0.15 | 0.15 |
| Parvo F 110 (100 µM) | 0.15 | 0.15 |
| Parvo R 110 (100 µM) | 0.15 | 0.15 |
| Master Mix Qiagen | 10 | 10 |
| H2O | 2.1 | 2.1 |
| UNG | 0.2 | 0.2 |
| DNA | 5 | 5 |
| Final volume | 15 | 15 |

### 2.1.3.2. Thermal cycler program

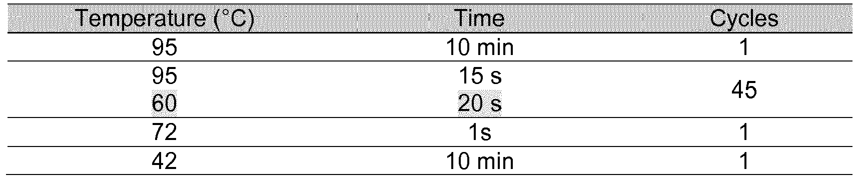

### 2.1.3.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m)-1, m being the slope of the calibration line equation.

**2.1.1.3.1.3. Cytomegalovirus (probe 40)**

| **Standard plasmid DNA** | **Cytomegalovirus (probe 40)** | |
|---|---|---|
| | **Singleplex** | **Multiplex** |
| | **Mean Ct** | **Mean Ct** |
| 10⁷ copies | 14.47 | 22.855 |
| 10⁶ copies | 17.73 | 25.915 |
| 10⁵ copies | 20.97 | 29.475 |
| 10⁴ copies | 24,47 | 33.105 |
| 10³ copies | 27.78 | 36.27 |
| 10² copies | 31,18 | 39.41 |
| 10 copies | 34,25 | 42.74 |
| 1 copy | No Ct | No Ct |
| CN | No Ct | No Ct |

| **Standard curve parameters** | **Singleplex** | **Multiplex** |
|---|---|---|
| Slope of the line | -3,32 | -3,34 |
| Correlation coefficient (R²) | 0,999 | 0,997 |
| Efficiency | 100% | 99.1% |
| Detection limit | 10 copies | 10 copies |

| | | |
|---|---|---|
| * Not detected in any replicas. ND: Not determined | | |

**2.1.3.3.1.1. Adenovirus (probe 108)**

| **Standard plasmid DNA** | **Adenovirus (probe 108)** | |
|---|---|---|
| | **Singleplex** | **Multiplex** |
| | **Mean Ct** | **Mean Ct** |
| 10⁷ copies | 14.11 | 17.65 |
| 10⁶ copies | 17.05 | 20.775 |
| 10⁵ copies | 20.46 | 24.445 |
| 10⁴ copies | 24.12 | 27.515 |
| 10³ copies | 27.88 | 31.175 |
| 10² copies | 30.86 | 34.88 |
| 10 copies | 34,59 | 36.21 |
| 1 copy | No Ct | No Ct |
| CN | No Ct | No Ct |

| **Standard curve parameters** | **Singleplex** | **Multiplex** |
|---|---|---|
| Slope of the line | -3,44 | -3,37 |
| Correlation coefficient (R²) | 0,999 | 0,998 |
| Efficiency | 95.2% | 98.0% |
| Detection limit | 10 copies | 10 copies |

| | | |
|---|---|---|
| * Not detected in any replicas. ND: Not determined | | |

**2.1.3.3.1.2. Bglo (probe 6)**

| Standard plasmid DNA | Endogenous gene (probe 6) | |
|---|---|---|
| | Singleplex | Multiplex |
| | Mean Ct | Mean Ct |
| 10⁷ copies | 24.1 | 18.24 |
| 10⁶ copies | 28.15 | 21.91 |
| 10⁵ copies | 31.96 | 25.28 |
| 10⁴ copies | 34.88 | 28.72 |
| 10³ copies | 37.48 | 32.99 |
| 10² copies | 42.485 | 37.09 |
| 10 copies | No Ct | 42.57* |
| 1 copy | No Ct | No Ct |
| CN | No Ct | No Ct |

| **Standard curve parameters** | **Singleplex** | **Multiplex** |
|---|---|---|
| Slope of the line | -3.56 | -3.70 |
| Correlation coefficient (R²) | 0.994 | 0.997 |
| Efficiency | 90.9% | 86.2% |
| Detection limit | 10 copies | 10 copies |

| | | |
|---|---|---|
| *Not detected in any replicas | | |

### Example 7. Multiplex qPCR kit for the detection of Bordetella pertussis, B. parapertussis, B. holmesii and the endogenous Bglo gene

### 1. Product description

Kit 3 is a real-time multiplex PCR kit indicated for the simultaneous detection of *Bordetella pertussis, B. parapertussis, B. holmessi* and the *Bglo* gene as a DNA extraction control/PCR inhibition control. This control allows verifying that the extraction process has been developed correctly and detecting false negatives due to PCR inhibition.

The kit contains the required reagents and enzymes mixed at ideal concentrations and designed to make diagnostic analysis easier.
Each Master Mix provides a FAM-labeled UPL probe for the detection of *B. pertussis,* a HEX-labeled UPL probe for the detection of *B. parapertussis,* a ROX-labeled UPL probe for the detection of *B. holmesii* and a Cy5-BHQ3®-labeled LNA probe for the detection of the *Bglo* gene (DNA extraction control/PCR inhibition control gene). The channels through which fluorescence-related data is collected are described in detail in Table 1.

**Table 1: Reporters and Quenchers**

| | Reporter | Quencher |
|---|---|---|
| *B. pertussis* (probe 86) | FAM | Roche |
| *B. parapertussis* (probe 40) | HEX | Exiqon |
| *B. holmesii* (probe 81) | ROX | Exiqon |
| *Bglo* gene (probe 144) | Cy5 | BHQ3® |

The UPL/LNA probe as well as the forward (F) and reverse (R) primers have the sequences provided in detail below:

| **Syndrome** | **Pathogen** | **Forward primer seq. (F)** | **Reverse primer seq. (R)** |
|---|---|---|---|
| **Whooping cough** | *B. pertussis* | 5’- TATGCATGTGCGAGCAGAAT- 3’ | 5’- ATCAGGTGGACGCCGTATT- 3’ |
| **Whooping cough** | *B. parapertussis* | 5'- GCAGGATCATCAAATCGAGTC- 3' | 5'- CTTGCAGCAATTCGTCGAG- 3' |
| **Whooping cough** | *B*. *holmesii* | 5'-ATGGACGACAAAACCAGCA- 3' | 5’- GCCAAACTGCTTTTCGATCT- 3’ |

| **Syndrome** | **Pathogen** | **Probe No.** | **Amplicon seq.** | **Amplicon (bp)** | **Probe seq.** |
|---|---|---|---|---|---|
| **Whooping cough** | *B. pertussis* | 86 | TATGCATGTGCGAGCA GAATATATTGCTCCAC TGCGAACGCTTGTCAG CCCAGGGGGAGCGCC CGGTGAATACGGCGTC CACCTGAT | 87 | seq. TCCACT GC |
| **Whooping cough** | *B. parapertussis* | 40 | GCAGGATCATCAAATC GAGTCGCTGGCTGCT GCTGCGCAACCGTGA CAACCTGGATCGGCAG CAGGCCGTCCGGCTC GACGAATTGCTGCAAG | 93 | CAGCAG GC |
| **Whooping cough** | *B*. *holmesii* | 81 | ATGGACGACAAAACCA GCAAGGCCGCATCGG AAAAAGCCAAGGCCCT GGCCGCAGCGCTCTC CCAGATCGAAAAGCAG TTTGGC | 84 | GGCCCT GG |

The probe as well as the primers for the *Bglo* gene have the sequences provided in detail below:

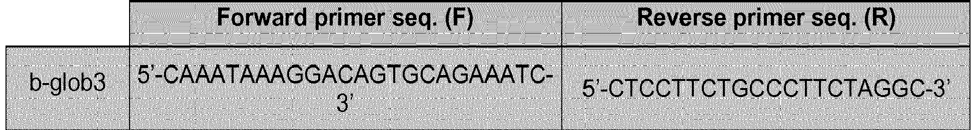

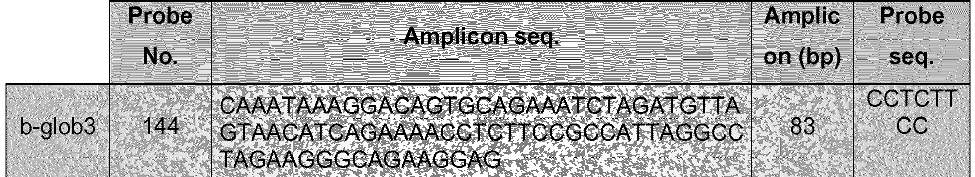

### 2. Singleplex assay (Comparative example)

### 2.1. Protocol validation by means of standards

### 2.1.1. Singleplex qPCR for the individual detection of B. pertussis (probe 86)/B. parapertussis (probe 40)/B. holmesii (probe 81)/Bglo gene (probe 144)

### 2.1.1.1. Singleplex qPCR reaction

| Indicate no. of reactions | 1 | 1 |
|---|---|---|
| | | |

| Reagent (concentration) | Volume (µl) | Volume (µl) |
|---|---|---|
| UPL/LNA probe (10 µM) | 0.5 | 0.5 |
| Primer-F (100 µM) | 0.15 | 0.15 |
| Primer-R (100 µM) | 0.15 | 0.15 |
| Master Probes | 10 | 10 |
| H2O | 4 | 4 |
| UNG | 0.2 | 0.2 |
| DNA | | 5 |
| Final volume | 15 | 20 |

### 2.1.1.2. Thermal cycler program

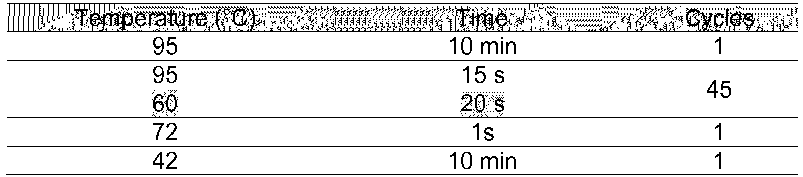

### 2.1.1.3. Detection limit of the target and amplification efficiency of each target in simplex format

To determine the detection limit of the target, 10-fold serial dilutions of plasmid DNA (standard DNA) were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, "m" being the slope of the calibration line equation.

**2.1.1.3.1. Results**

| | Mean Ct obtained | | | |
|---|---|---|---|---|
| Standard plasmid DNA | *B. pertussis* (probe 86) | *B. parapertussis* (probe 40) | *B*. *holmesii* (probe 81) | *Bglo* gene (probe 144) |
| 10⁷ copies | 13.28 | 14.04 | 13.02 | 15.35 |
| 10⁶ copies | 16.64 | 17.55 | 16.27 | ND |
| 10⁵ copies | 20.15 | 20.85 | 19.64 | 21.73 |
| 10⁴ copies | 23.79 | 24.21 | 22.99 | 24.76 |
| 10³ copies | 27.26 | 27.55 | 26.37 | 28.22 |
| 10² copies | 30.86 | 31.30 | 29.78 | 31.92 |
| 10 copies | 34.49 | 34.01 | 33.72 | 34.32 |
| 1 copy | 36.46* | 36.93* | 35.61* | *37.06 |
| CN | No Ct | No Ct | No Ct | No Ct |

| | | | | |
|---|---|---|---|---|
| *Not detected in any replicates ND: Not determined | | | | |

**2.1.1.3.1.1. B. pertussis (probe 86)**

| Standard curve parameters obtained | |
|---|---|
| Slope of the line | -3.47 |
| Correlation coefficient (R²) | 0.997 |
| Efficiency | 93.9% |
| Detection limit: 10 copies | |

**2.1.1.3.1.2. B. parapertussis (probe 40)**

| Standard curve parameters obtained | |
|---|---|
| Slope of the line | -3.33 |
| Correlation coefficient (R²) | 0.998 |
| Efficiency | 99.6% |
| Detection limit: 10 copies | |

**2.1.1.3.1.3. B. holmessi (probe 81)**

| Standard curve parameters obtained | |
|---|---|
| Slope of the line | -3.34 |
| Correlation coefficient (R²) | 0.994 |
| Efficiency | 99.0% |
| Detection limit: 10 copies | |

**2.1.1.3.1.4. Bglo gene Cy5 (probe 144)**

| Standard curve parameters obtained | |
|---|---|
| Slope of the line | -3.27 |
| Correlation coefficient (R²) | 0.997 |
| Efficiency | 102% |
| Detection limit: 10 copies | |

### 2.1.2. Multiplex qPCR for the simultaneous detection of B. pertussis (probe 86)/B. parapertussis (probe 40)/B. holmessi (probe 81)/Bglo gene (probe 144)

### 2.1.2.1. Multiplex qPCR reaction

| Indicate no. of reactions | 1 |
|---|---|
| | |

| Reagent (concentration) | Volume (µl) |
|---|---|
| Probe 86 (10 µM) | 0.5 |
| Probe 40 (10 µM) | 0.5 |
| Probe 81 (10 µM) | 0.5 |
| Probe 144Cy5 (10 µM) | 0.5 |
| BPthio86F (100 µM) | 0.15 |
| BPthio86R (100 µM) | 0.15 |
| Bpara40F (100 µM) | 0.15 |
| Bpara40R (100 µM) | 0.15 |
| BH81 F (100 µM) | 0.25 |
| BH81 R (100 µM) | 0.25 |
| Bglo144F (100 µM) | 0.15 |
| Bglo144R (100 µM) | 0.15 |
| Master Mix Roche | 10 |
| H2O | 1.4 |
| UNG | 0.2 |
| DNA | 5 |
| Final volume | 20 |

### 2.1.2.2. Thermal cycler program

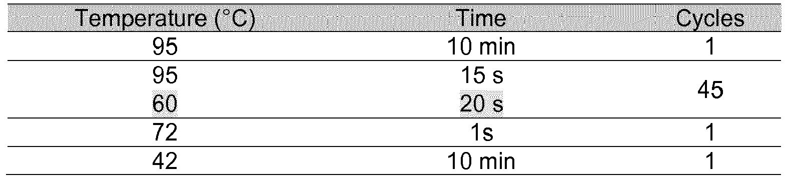

### 2.1.2.3. Detection limit of the target and multiplex amplification efficiency

To determine the detection limit of the target, 10-fold serial dilutions of plasmid DNA (standard DNA) were tested in a range of concentration usually varying between 10⁷ copies and 1 copy per well using 3 replicates per dilution.

Calibration curve parameters were analyzed to determine qPCR amplification efficiency. Efficiency: 10(-1/m)-1, m being the slope of the calibration line equation.

### 2.1.2.3.1. Results

**2.1.2.3.1.1. B. pertussis**

| **Standard plasmid DNA** | ***B*. *pertussis* (probe 86)** | |
|---|---|---|
| | **Singleplex** | **Multiplex** |
| | **Mean Ct** | **Mean Ct** |
| 10⁷ copies | 13.28 | 15.23 |
| 10⁶ copies | 16.64 | 18.40 |
| 10⁵ copies | 20.15 | 22.08 |
| 10⁴ copies | 23.79 | 25.83 |
| 10³ copies | 27.26 | 29.25 |
| 10² copies | 30.86 | 32.79 |
| 10 copies | 34.49 | 36.57 |
| 1 copy | 36.46* | 39.71* |
| CN | No Ct | No Ct |

| **Standard curve parameters** | **Singleplex** | **Multiplex** |
|---|---|---|
| Slope of the line | -3.47 | -3.55 |
| Correlation coefficient (R²) | 0.997 | 0.997 |
| Efficiency | 93.9% | 91.3% |
| Detection limit | 10 copies | 10 copies |

| | | |
|---|---|---|
| * Not detected in any replicas. ND: Not determined | | |

**2.1.2.3.1.2. B. parapertussis (probe 40)**

| **Standard plasmid DNA** | ***B*. *parapertussis* (probe 40)** | |
|---|---|---|
| | **Singleplex** | **Multiplex** |
| | **Mean Ct** | **Mean Ct** |
| 10⁷ copies | 14.04 | 14.92 |
| 10⁶ copies | 17.55 | 17.97 |
| 10⁵ copies | 20.85 | 21.33 |
| 10⁴ copies | 24.21 | 24.82 |
| 10³ copies | 27.55 | 28.32 |
| 10² copies | 31.30 | 31.54 |
| 10 copies | 34.01 | 34.46 |
| 1 copy | 36.93* | 38.47 |
| CN | No Ct | No Ct |

| **Standard curve parameters** | **Singleplex** | **Multiplex** |
|---|---|---|
| Slope of the line | -3.33 | -3.34 |
| Correlation coefficient (R²) | 0.998 | 0.999 |
| Efficiency | 99.6% | 99.2% |
| Detection limit | 10 copies | 10 copies |

| | | |
|---|---|---|
| *Not detected in any replicas. | | |

**2.1.2.3.1.3. B. holmessi (probe 81)**

| **Standard plasmid DNA** | ***B*. *holmesii* (probe 81)** | |
|---|---|---|
| | **Singleplex** | **Multiplex** |
| | **Mean Ct** | **Mean Ct** |
| 10⁷ copies | 13.2 | 13.24 |
| 10⁶ copies | 16.27 | 16.74 |
| 10⁵ copies | 19.64 | 20.16 |
| 10⁴ copies | 22.99 | 23.88 |
| 10³ copies | 26.37 | 28.02 |
| 10² copies | 29.78 | 31.47 |
| 10 copies | 33.72 | 35.04 |
| 1 copy | 35.61* | 43.67* |
| CN | No Ct | No Ct |

| **Standard curve parameters** | **Singleplex** | **Multiplex** |
|---|---|---|
| Slope of the line | -3.34 | -3.67 |
| Correlation coefficient (R²) | 0.994 | 0.999 |
| Efficiency | 99.0% | 87% |
| Detection limit | 10 copies | 10 copies |

| | | |
|---|---|---|
| *Not detected in any replicas. | | |

**2.1.2.3.1.4. Bglo gene (probe 144)**

| **Standard plasmid DNA** | ***Bglo* gene (probe 144)** | |
|---|---|---|
| | **Singleplex** | **Multiplex** |
| | **Mean Ct** | **Mean Ct** |
| 10⁷ copies | 15.35 | 14.09 |
| 10⁶ copies | ND | 16.83 |
| 10⁵ copies | 21.73 | 19.82 |
| 10⁴ copies | 24.76 | 23.66 |
| 10³ copies | 28.22 | 26.97 |
| 10² copies | 31.92 | 30.9 |
| 10 copies | 34.32 | 35.46 |
| 1 copy | 37.06* | 36.38* |
| CN | No Ct | No Ct |

| **Standard curve parameters** | **Singleplex** | **Multiplex** |
|---|---|---|
| Slope of the line | -3.27 | -3.43 |
| Correlation coefficient (R²) | 0.997 | 0.988 |
| Efficiency | 102% | 95.5% |
| Detection limit | 10 copies | 10 copies |

| | | |
|---|---|---|
| *Not detected in any replicas. ND: Not determined | | |

### Example 8. Multiplex qPCR kit for the detection of Mycoplasma pneumoniae, Coxielia burnetti, Chlamydia pneumoniae, Legionella pneumophila, Pneumocystis jirovecii and the endogenous gene Bglo

### 1. Product description

Kit 6 is a real-time multiplex PCR kit indicated for the simultaneous detection of, on one hand, *Mycoplasma pneumoniae, Coxiella burnetti, Chlamydia pneumoniae* and the endogenous *Bglo* gene, and on the other, *Legionella pneumophila, Pneumocystis jirovecii* and the endogenous *Bglo* gene. The endogenous gene works as a DNA extraction control and PCR inhibition control.

It allows verifying that the extraction process has been developed correctly and detecting false negatives due to PCR inhibition. The kit contains the required reagents and enzymes mixed at ideal concentrations and designed to make diagnostic analysis easier.

The kit contains 2 mixes:
- **Mix 1** provides a FAM-labeled UPL probe for the detection of *Mycoplasma pneumoniae,* a HEX-labeled UPL probe for the detection of *Coxiella burnetti,* a Tex 615-labeled UPL probe for the detection of *Chlamydia pneumoniae,* and a Cy5-BHQ3®-labeled LNA probe for the detection of the *Bglo* gene (DNA extraction control/PCR inhibition control gene).
- **Mix 2** provides a FAM-labeled UPL probe for the detection of *Legionella pneumophila,* a HEX-labeled UPL probe for the detection of *Pneumocystis jirovecii,* and a Cy5-BHQ3®-labeled LNA probe for the detection of the *Bglo* gene (DNA extraction control/PCR inhibition control gene).

The channels through which fluorescence-related data is collected are described in detail in Table 1.

**Table 1: Reporters and Quenchers**

| Mix 1 | Reporter | Quencher |
|---|---|---|
| *Mycoplasma pneumoniae* (probe 150) | FAM | Roche |
| *Coxiella burnetti* (probe 59) | HEX | Exiqon |
| *Chlamydia pneumoniae* (probe 67) | Tex615/ROX | Exiqon |
| *Bglo* gene (probe 144) | Cy5 | BHQ3® |

| Mix 2 | Reporter | Quencher |
|---|---|---|
| *Legionella pneumophila* (probe 60) | FAM | Roche |
| *Pneumocystis jirovecii* (probe 141) | HEX | Exiqon |
| *Bglo* gene (probe 144) | Cy5 | BHQ3® |

The UPL/LNA probe as well as the forward (F) and reverse (R) primers have the sequences provided in detail below:

| **Syndrome** | **Pathogen** | **Forward primer seq**. (**F**) | **Reverse primer seq. (R)** |
|---|---|---|---|
| ***Atypical pneumoniae*** | *Mycoplasma pneumoniae* | 5'- GCAGCTAATGTCCGTAGT GCT-3' | 5'- GTGGTGAGCATGACCAGG TT-3' |
| ***Atypical pneumoniae*** | *Coxiella burnetti* | 5'- GAAATTATTTAACGACCCT GCATC-3' | 5’- AATCGAAAAATTCAACCAA TGTAAC-3’ |
| ***Atypical pneumoniae*** | *Chlam ydia pneumoniae* | 5’- CGTTGGCTACGAAAGATT CAG-3’ | 5’- GGGTAAAGAATAACGAGC ACCA-3’ |
| ***Atypical pneumoniae*** | *Legionella pneumophila* | 5'- GCAAACGATGAAAACTTT GCT-3' | 5'- CACAGTGATGGTTTAACTA AAGTGC-3' |
| ***Atypical pneumoniae*** | *Pneumocystis jirovecii* | 5'- TTTTTCGATGGGGGTGTT C-3' | 5'- CGTGTAGACTGCCCACCA AT-3' |

| **Syndrome** | **Pathogen** | **Probe No.** | **Amplicon seq.** | **Amplicon (bp)** | **Probe seq.** |
|---|---|---|---|---|---|
| ***Atypical pneumoniae*** | *Mycoplasma pneumoniae* | 150 | GCAGCTAATGTCCGT AGTGCTTGACTAGTA GATGCTGTTCCCGTT GAACCTGGTCATGCT CACCAC | 66 | TGCTGTT C |
| ***Atypical pneumoniae*** | *Coxiella burnetti* | 59 | GAAATTATTTAACGAC CCTGCATCACCAGTG GCAGGCAATCCTCAT GGCAATGTTACATTG GTTGAATTTTTCGATT | 77 | CAGTGGC A |
| ***Atypical pneumoniae*** | *Chlamydia pneumoniae* | 67 | CGTTGGCTACGAAAG ATTCAGGCTGTAAAG GTTGCTGGAGAGCGT GGTGCTCGTTATTCT TTACCC | 66 | TGCTGGA G |
| ***Atypical pneumoniae*** | *Legionella pneumophila* | 60 | GCAAACGATGAAAAC TTTGCTGGTGGGGAA GCTATCGCTGCCTAA TAAGCACTTTAGTTAA ACCATCACTGTG | 73 | TGGGGAA G |
| ***Atypical pneumoniae*** | *Pneumocystis jirovecii* | 141 | TTTTTCGATGGGGGT GTTCATTCATATGATT CTATATTAATGGATGT GGAGAATTTTATAAAT GCAGGGGCGACGAT AATTGATATTGGTGG GCAGTCTACACG | 104 | GGCGAC GA |

The probe as well as the primers for the *Bglo* gene have the sequences provided in detail below:

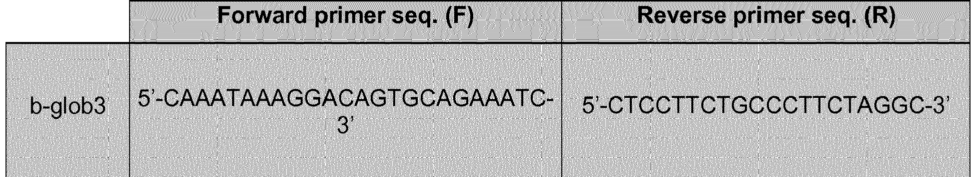

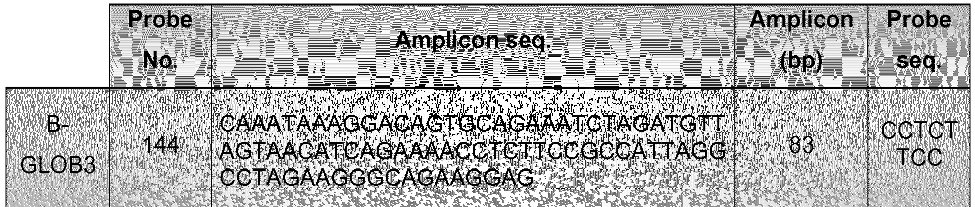

### 2. Singleplex assay (Comparative example)

### 2.1. Protocol validation by means of standards.

### 2.1.1. Singleplex qPCR for the individual detection of Mycoplasma pneumoniae (probe 150)/Coxiella burnetti (probe 59)/Chlamydia pneumoniae (probe 67)/Legionella pneumophila (probe 60)/Pneumocystis jirovecii (probe 141)/Bglo gene (probe 144)

**2.1.1.1. Singleplex qPCR reaction**

| Indicate no. of reactions | 1 | 1 |
|---|---|---|
| | | |

| Reagent (concentration) | Volume (µl) | Volume (µl) |
|---|---|---|
| UPL/LNA probe (10 µM) | 0.5 | 0.5 |
| Primer-F (100 µM) | 0.15 | 0.15 |
| Primer-R (100 µM) | 0.15 | 0.15 |
| Master Probes | 10 | 10 |
| H2O | 4 | 4 |
| UNG | 0.2 | 0.2 |
| DNA | | 5 |
| Final volume | 15 | 20 |

### 2.1.1.2. Thermal cycler program

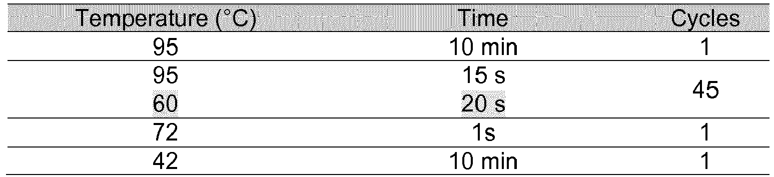

### 2.1.1.3. Detection limit of the target and amplification efficiency of each target in simplex format

To determine the detection limit of the target, 10-fold serial dilutions of a mixture of plasmid DNA (standard DNA) of the targets to be detected were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, "m" being the slope of the calibration line equation.

**2.1.1.3.1. Results**

| | Mean Ct obtained | | |
|---|---|---|---|
| Standard plasmid DNA | *Mycoplasma pneumorriae* (probe 150) | *Coxiella burnetti* (probe 59) | *Chlamydia pneumoniae* (probe 67) |
| 10⁷ copies | 15.21 | 16.01 | 13.53 |
| 10⁶ copies | 18.75 | 19.89 | 16.47 |
| 10⁵ copies | 22.11 | 23 | 19.98 |
| 10⁴ copies | 25.53 | 26.40 | 23.485 |
| 10³ copies | 29.13 | 30.11 | 27.03 |
| 10² copies | 32.38 | 33.35 | 30.27 |
| 10 copies | 36.16 | 36.69 | 33.79 |
| 1 copy | 37.88* | 36.94* | 37.09* |
| CN | No Ct | No Ct | No Ct |

| | Mean Ct obtained | | |
|---|---|---|---|
| | *Legionella pneumophila* (probe 60) | Pneumocystis jirovecii (probe 141) | *Bglo* gene (probe 144) |
| 10⁷ copies | 14.99 | 20.38 | 15.35 |
| 10⁶ copies | 18.77 | 24.7 | ND |
| 10⁵ copies | 22.05 | 29.59 | 21.73 |
| 10⁴ copies | 25.48 | 32.59 | 24.76 |
| 10³ copies | 28.86 | 36.61 | 28.22 |
| 10² copies | 32.17 | 38.38 | 31.92 |
| 10 copies | 36.26 | 41 | 34.32 |
| 1 copy | 36.88* | No Ct | 37.06^{*} |
| CN | No Ct | No Ct | No Ct |

| | | | |
|---|---|---|---|
| ^{*}Not detected in any replicates. ND: Not determined | | | |

**2.1.1.3.1.1. Mycoplasma pneumoniae (probe 150)**

| Standard curve parameters obtained | |
|---|---|
| Slope of the line | -3.40 |
| Correlation coefficient (R²) | 0.998 |
| Efficiency | 96.5% |
| Detection limit: 10 copies | |

**2.1.1.3.1.2. Coxiella burnetti (probe 59)**

| Standard curve parameters obtained | |
|---|---|
| Slope of the line | -3.43 |
| Correlation coefficient (R²) | 0.998 |
| Efficiency | 95.6% |
| Detection limit: 10 copies | |

**2.1.1.3.1.3. Chlamydia pneumoniae (probe 67)**

| Standard curve parameters obtained | |
|---|---|
| Slope of the line | -3.40 |
| Correlation coefficient (R²) | 0.999 |
| Efficiency | 96.7% |
| Detection limit: 10 copies | |

**2.1.1.3.1.4. Legionella pneumophila (probe 60)**

| Standard curve parameters obtained | |
|---|---|
| Slope of the line | -3.31 |
| Correlation coefficient (R²) | 0.988 |
| Efficiency | 100.3% |
| Detection limit: 10 copies | |

**2.1.1.3.1.5. Pneumocystis jirovecii (probe 141)**

| Standard curve parameters obtained | |
|---|---|
| Slope of the line | -3.38 |
| Correlation coefficient (R²) | 0.983 |
| Efficiency | 97.6% |
| Detection limit: 10 copies | |

**2.1.1.3.1.6. Bglo gene Cy5 (probe 144)**

| Standard curve parameters obtained | |
|---|---|
| Slope of the line | -3.27 |
| Correlation coefficient (R²) | 0.997 |
| Efficiency | 102% |
| Detection limit: 10 copies | |

### 2.1.2. Mix 1: multiplex qPCR for the simultaneous detection of Mycoplasma pneumoniae (probe 150)/Coxiella burnetti (probe 59)/Chlamydia pneumoniae (probe 67)/Bglo gene (probe 144)

### 2.1.2.1. Multiplex qPCR reaction

| Indicate no. of reactions | 1 | |
|---|---|---|
| | | |

| Reagent (concentration) | Volume (µl) | Check |
|---|---|---|
| Probe Mp card150 FAM (10 µM) | 0.5 | 0.5 |
| Probe CBcom59 HEX (10 µM) | 0.5 | 0.5 |
| Probe chla67tex615 (10 µM) | 1 | 1 |
| Probe Bglol44Cy5 (10 µM) | 1 | 1 |
| Mp card150F (100 µM) | 0.15 | 0.15 |
| Mp card150R (100 µM) | 0.15 | 0.15 |
| CBcom59F (100 µM) | 0.15 | 0.15 |
| CBcom59R (100 µM) | 0.15 | 0.15 |
| chla67F (100 µM) | 0.3 | 0.3 |
| chla67R (100 µM) | 0.3 | 0.3 |
| Bglo144F (100 µM) | 0.25 | 0.25 |
| Bglo144R (100 µM) | 0.25 | 0.25 |
| Master Mix Roche | 10 | 10 |
| H2O | 0.1 | 0.1 |
| UNG | 0.2 | 0.2 |
| DNA | 5 | 5 |
| Final volume | 5 | 5 |

### 2.1.2.2. Thermal cycler program

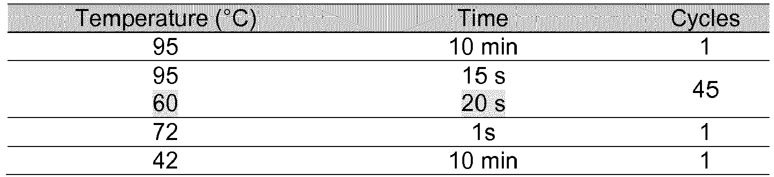

### 2.1.2.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well. Two replicates per dilution were analyzed.

Calibration curve parameters were analyzed to determine qPCR amplification efficiency. Efficiency: 10(-1/m) -1, m being the slope of the calibration line equation.

### 2.1.2.3.1. Results

**2.1.2.3.1.1. Mycoplasma pneumoniae (probe 150)**

| **Standard plasmid DNA** | ***Mycoplasma pneumoniae* (probe 150)** | |
|---|---|---|
| | **Singleplex** | **Multiplex** |
| | **Mean Ct** | **Mean Ct** |
| 10⁷ copies | 15.21 | 14.44 |
| 10⁶ copies | 18.75 | 18.10 |
| 10⁵ copies | 22.11 | 21.29 |
| 10⁴ copies | 25.53 | 24.63 |
| 10³ copies | 29.13 | 28.01 |
| 10² copies | 32.38 | 31.59 |
| 10 copies | 36.16 | 34.85 |
| 1 copy | 37.88 | No Ct |
| CN | No Ct | No Ct |

| **Standard curve parameters** | **Singleplex** | **Multiplex** |
|---|---|---|
| Slope of the line | -3.40 | -3.39 |
| Correlation coefficient (R²) | 0.998 | 0.998 |
| Efficiency | 96.5% | 97.2% |
| Detection limit | 10 copies | 10 copies |

| | | |
|---|---|---|
| ^{*}Not detected in any replicas. ND: Not determined | | |

**2.1.2.3.1.2. Coxiella burnetti (probe 59)**

| **Standard plasmid DNA** | ***Coxiella burnetti* (probe 59)** | |
|---|---|---|
| | **Singleplex** | **Multiplex** |
| | **Mean Ct** | **Mean Ct** |
| 10⁷ copies | 16.01 | 14.31 |
| 10⁶ copies | 19.89 | 18.16 |
| 10⁵ copies | 23 | 20.95 |
| 10⁴ copies | 26.40 | 25.015 |
| 10³ copies | 30.11 | 28.26 |
| 10² copies | 33.35 | 31.725 |
| 10 copies | 36.69 | 35.87 |
| 1 copy | 36.94* | No Ct |
| CN | No Ct | No Ct |

| **Standard curve parameters** | **Singleplex** | **Multiplex** |
|---|---|---|
| Slope of the line | -3.43 | -3.51 |
| Correlation coefficient (R²) | 0.998 | 0.996 |
| Efficiency | 95.6% | 92.5% |
| Detection limit | 10 copies | 10 copies |

| | | |
|---|---|---|
| *Not detected in any replicas. | | |

**2.1.1.3.1.3. Chlamydia pneumoniae (probe 67)**

| **Standard plasmid DNA** | ***Chlamydia pneumoniae* (probe 67)** | |
|---|---|---|
| | **Singleplex** | **Multiplex** |
| | **Mean Ct** | **Mean Ct** |
| 10⁷ copies | 13.53 | 10.04 |
| 10⁶ copies | 16.47 | 13.91 |
| 10⁵ copies | 19.98 | 18.44 |
| 10⁴ copies | 23.48 | 23,28 |
| 10³ copies | 27.03 | 26.64 |
| 10² copies | 30.27 | 28.82 |
| 10 copies | 33.79 | 31.9 |
| 1 copy | 37.09* | 37.29* |
| CN | No Ct | No Ct |

| **Standard curve parameters** | **Singleplex** | **Multiplex** |
|---|---|---|
| Slope of the line | -3.40 | -4.1 |
| Correlation coefficient (R²) | 0.999 | 0.993 |
| Efficiency | 96.7% | 75.2% |
| Detection limit | 10 copies | 10 copies |

| | | |
|---|---|---|
| ^{*}Not detected in any replicas. | | |

**2.1.2.3.1.3. Bglo gene (probe 144)**

| **Standard plasmid DNA** | ***Bglo* gene (probe 144)** | |
|---|---|---|
| | **Singleplex** | **Multiplex** |
| | **Mean Ct** | **Mean Ct** |
| 10⁷ copies | 15.35 | 12.81 |
| 10⁶ copies | ND | 16.06 |
| 10⁵ copies | 21.73 | 19.685 |
| 10⁴ copies | 24.76 | 23.21 |
| 10³ copies | 28.22 | 26.52 |
| 10² copies | 31.92 | 30.41 |
| 10 copies | 34.32 | 35.79 |
| 1 copy | 37.06* | No Ct |
| CN | No Ct | No Ct |

| **Standard curve parameters** | **Singleplex** | **Multiplex**** |
|---|---|---|
| Slope of the line | -3.27 | -3.51 |
| Correlation coefficient (R²) | 0.997 | 0.998 |
| Efficiency | 102% | 92.7% |
| Detection limit | 10 copies | 10 copies |

| | | |
|---|---|---|
| *Not detected in any replicas. **Data obtained from the standard of 10 copies not included in the calculation of standard curve parameters because it takes too long. However, it was detected in all replicates. ND: Not determined | | |

### 2.1.3. Mix 2: multiplex qPCR for the simultaneous detection of Legionella pneumophila (probe 60)/Pneumocystis jirovecii (probe 141)/Bglo gene (probe 144)

**2.1.3.1. Multiplex qPCR reaction**

| Indicate no. of reactions | 31 | 1 |
|---|---|---|
| | | |

| Reagent (concentration) | Volume (µl) | Check |
|---|---|---|
| Probe Legtag60 tex 615 (10 µM) | 0.5 | 0.5 |
| Probe Pjir141 (10 µM) | 0.5 | 0.5 |
| Probe Bglo6Cy5 (10 µM) | 0.5 | 0.5 |
| Legtag60F (100 µM) | 0.15 | 0.15 |
| Legtag60R (100 µM) | 0.15 | 0.15 |
| Pjir141 F (100 µM) | 0.15 | 0.15 |
| Pjir141 R (100 µM) | 0.15 | 0.15 |
| Bglol44F (100 µM) | 0.15 | 0.15 |
| Bglo144R (100 µM) | 0.15 | 0.15 |
| Master Mix Roche | 10 | 10 |
| H2O | 2.4 | 2.4 |
| UNG | 0.2 | 0.2 |
| DNA | 5 | 5 |
| Final volume | 15 | 5 |

### 2.1.3.2. Thermal cycler program

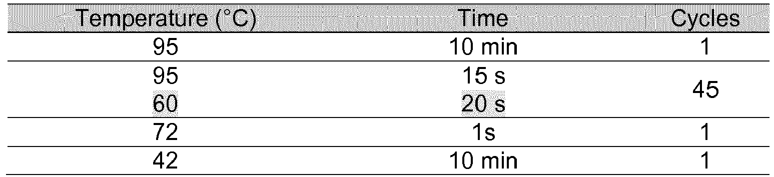

### 2.1.3.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well. Three replicates per dilution were analyzed.

Calibration curve parameters were analyzed to determine qPCR amplification efficiency. Efficiency: 10(-1/m) -1, m being the slope of the calibration line equation.

### 2.1.3.3.1. Results

**2.1.3.3.1.1. Legionella pneumophila (probe 60)**

| **Standard plasmid DNA** | ***Legionella pneumophila* (probe 60)** | |
|---|---|---|
| | **Singleplex** | **Multiplex** |
| | **Mean Ct** | **Mean Ct** |
| 10⁷ copies | 14.99 | 17.54 |
| 10⁶ copies | 18.77 | 20.59 |
| 10⁵ copies | 22.05 | 24.01 |
| 10⁴ copies | 25.48 | 27.46 |
| 10³ copies | 28.86 | 30.59 |
| 10² copies | 32.17 | 34.09 |
| 10 copies | 36.26 | 36.85 |
| 1 copy | 36.88* | No Ct |
| CN | No Ct | No Ct |

| **Standard curve parameters** | **Singleplex** | **Multiplex** |
|---|---|---|
| Slope of the line | -3.31 | -3.26 |
| Correlation coefficient (R²) | 0.988 | 0.999 |
| Efficiency | 100.3% | 102.3% |
| Detection limit | 10 copies | 10 copies |

| | | |
|---|---|---|
| ^{*}Not detected in any replicas. | | |

**2.1.3.3.1.2. Pneumocystis jirovecii (probe 141)**

| **Standard plasmid DNA** | ***Pneumocystis jirovecii* (probe 141)** | |
|---|---|---|
| | **Singleplex** | **Multiplex** |
| | **Mean Ct** | **Mean Ct** |
| 10⁷ copies | 20.38 | 17.13 |
| 10⁶ copies | 24.7 | 21.04 |
| 10⁵ copies | 29.59 | 22.76 |
| 10⁴ copies | 32.59 | 24.71 |
| 10³ copies | 36.61 | 26.38 |
| 10² copies | 38.38 | 28.22 |
| 10 copies | 41 | 32.33 |
| 1 copy | No Ct | No Ct |
| CN | No Ct | No Ct |

| **Standard curve parameters** | **Singleplex** | **Multiplex**** |
|---|---|---|
| Slope of the line | -3.38 | -3.73 |
| Correlation coefficient (R²) | 0.983 | 0.998 |
| Efficiency | 97.6% | 85.4% |
| Detection limit | 10 copies | 10 copies |

| | | |
|---|---|---|
| **Data obtained from the standard of 10 copies not included in the calculation of standard curve parameters because it takes too long. However, it was detected in all replicates. | | |

**2.1.3.3.1.3. Bglo gene (probe 144)**

| **Standard plasmid DNA** | ***Bglo* gene (probe 144)** | |
|---|---|---|
| | **Singleplex** | **Multiplex** |
| | **Mean Ct** | **Mean Ct** |
| 10⁷ copies | 15.35 | 18.01 |
| 10⁶ copies | ND | 21.15 |
| 10⁵ copies | 21.73 | 24.27 |
| 10⁴ copies | 24.76 | 27.67 |
| 10³ copies | 28.22 | 30.99 |
| 10² copies | 31.92 | 33.31 |
| 10 copies | 34.32 | 38.29 |
| 1 copy | 37.06* | No Ct |
| CN | No Ct | No Ct |

| Standard curve parameters | Singleplex | Multiplex |
|---|---|---|
| Slope of the line | -3.27 | -3.28 |
| Correlation coefficient (R2) | 0.997 | 0.994 |
| Efficiency | 102% | 101.7% |
| Detection limit | 10 copies | 10 copies |

| | | |
|---|---|---|
| ^{*}Not detected in any replicas. ND: Not determined | | |

### Example 9. Multiplex qPCR kit for the detection of Dientamoeba fragilis, Giardia lamblia A, Giardia lamblia B, Crystosporidium parvumlhominis, Plasmodiun falciparum, P. ovale and P. vivax

### 1. Product description

The kit of Example 9 is a real-time multiplex PCR kit indicated for the detection of *Dientamoeba fragilis, Giardia lamblia* A, *Giardia lamblia* B, *Crystosporidium parvum*/*hominis, Plasmodiun falciparum, P. ovale* and *P. vivax.* The kit contains the required reagents and enzymes mixed at ideal concentrations and designed to make diagnostic analysis easier.

The kit contains the following mixes:

### Parasites in stools

**-Mix 1:** provides a FAM-labeled LNA probe for the detection *Giardia lamblia* B, a HEX-labeled LNA probe for the detection of *Dientamoeba fragilis,* a Tex615-labeled LNA probe for the detection of *Crystosporidium parvum*/*hominis* and a Cy5-BHQ3®-labeled LNA probe for the detection of *Giardia lamblia* A.

### Parasites in blood:

- **Mix1:** provides a FAM-labeled LNA probe for the detection of *Plasmodiun falciparum,* and a Cy5-labeled LNA probe for the detection of *Plasmodium ovale.*
- **Mix2:** provides a Tex615-labeled LNA probe for the detection of *Plasmodium vivax.*

The channels through which fluorescence-related data is collected are described in detail in Table 1.

**Table 1: Reporters and Quenchers**

| Parasites in stools | Reporter | Quencher |
|---|---|---|
| *Giardia lamblia* B | FAM | Roche |
| *Dientamoeba fragilis* | HEX | Exiqon |
| *Crystosporidium parvum*/*hominis* | Tex 615 | Exiqon |
| *Giardia lamblia* A | Cy5 | BHQ3^{®} |

| Parasites in blood (Mix 1) | Reporter | Quencher |
|---|---|---|
| *Plasmodiun falciparum* | FAM | Roche |
| *Plasmodium ovale* | Cy5 | Exiqon |

| Parasites in blood (Mix 2) | Reporter | Quencher |
|---|---|---|
| *Plasmodium vivax* | Tex615 | Exiqon |

The UPL/LNA probe as well as the forward (F) and reverse (R) primers have the sequences provided in detail below:

| **Syndrome** | **Pathogen** | **Forward primer seq. (F)** | **Reversa primer seq. (R)** |
|---|---|---|---|
| ***PARASITES*** | *Dientamoeba fragilis* | 5'- GACACGTATCCTTACAAAC ATTGG-3' | 5'- GACTTTTCTGTCATGTTAT CACCAG-3' |
| ***PARASITES*** | *Giardia lamblia* A | 5’- AACAAGAGCGGCCTTTCC-3 | 5'- CTTTCCACGGCAGATGAAC -3' |
| ***PARASITES*** | *Giardia lamblia* B | 5’- CTCCCCAAGGACACAAGC- 3’ | 5’- GCAACCGTTGTCCTGAGC- 3’ |
| ***PARASITES*** | *Crystosporidiu m parvum*/*homin is* | 5’- GTGAAAAGTGTCTCTTAGC AAATCATA-3 | 5'- TTGTCTTTGAACACACCTT TCTTT-3' |
| ***PARASITES*** | *Plasmodiun falciparum* | 5’- TGAAAAACAGATAAAATGTA TGTGCAG-3’ | 5’- TCTCGACAAACATTAGGAA CACA-3’ |
| ***PARASITES*** | *P. ovale* | 5’- CAGTTAATGATAAAGAATC GGGTTATC-3’ | 5’- ATTTCTCCTTGGTAAAATTT ATCTTCA-3’ |
| ***PARASITES*** | *P. vivax* | 5’- TGGGGTATGACGTGTGTGA G-3’ | 5'- CGCTGTCTTCACTGGAGAA GTT-3' |

| **Syndrome** | **Pathogen** | **Probe No**. | **Amplicon seq.** | **Amplicon (bp)** | **Probe seq.** |
|---|---|---|---|---|---|
| ***PARASITES*** | *Dientamoeba fragilis* | 129 | GACACGTATCCTT ACAAACATTGGTT TCAAGCCAGAAC AATACAAGTTYGT TCCAATMTCAGGT TTYGCTGGTGATA ACATGACAGAAA AGTC | 93 | [?] AGCCAGA A |
| ***PARASITES*** | *Giardia lamblia* A | 6 | AACAAGAGCGGC CTTTCCACTGGC GCCATCGCGGGG ATCTCCGTCGCA GCCGTCGTCGTC GTGGCCGGCCTT GTGGGCTTCCTC TGCTGGTGGTTC ATCTGCCGTGGA AAG | 111 | TTCCTCT G |
| ***PARASITES*** | *Giardia lamblia* B | 70 | CTCCCCAAGGAC ACAAGCCATGCAT GCCCGCGCACCC GGGAGGCGGCG GACGGCTCAGGA CAACGGTTGC | 70 | GGCGGC GG |
| ***PARASITES*** | *Crystosporidium parvum*/*hominis* | 4 | GTGAAAAGTGTCT CTTAGCAAATCAT AGATTTGAACAAG AATTATATAGACA AAAATTGCAGGAA GAAAAAGAAAGG TGTGTTCAAAGAC AA | 92 | GCAGGAA G |
| ***PARASITES*** | *Plasmodiun falciparum* | 67 | TGAAAAACAGATA AAATGTATGTGCA GAACAGAATATAC TTTAACTGCTGGA GTATGTGTTCCTA ATGTTTGTCGAGA | 78 | TGCTGGA G |
| ***PARASITES*** | *P. ovale* | 119 | CAGTTAATGATAA AGAATCGGGTTAT CTAACCATTGGTG GTATTGAAGATAA ATTTTACCAAGGA GAAAT | 70 | TTGGTGG T |
| ***PARASITES*** | *P. vivax* | 88 | TGGGGTATGACG TGTGTGAGGAGG AGGAGGAGGAGG ATGTGAAGGTGA AGGGAGAGAACT TCTCCAGTGAAGA CAGCG | 78 | GGAGGAT G |

### 2. Singleplex assay (Comparative example)

### 2.1. Protocol validation by means of standards.

### 2.1.1. Singleplex qPCR for the individual detection of Dientamoeba fragilis (probe 129), Giardia lamblia A (probe 6), Giardia lamblia B (Probe 70), Crystosporidium parvum/hominis (probe 4), Plasmodiun falciparum (probe 67), P. ovale (probe 119) and P. vivax (probe 88)

### 2.1.1.1. Singleplex qPCR reaction

| Indicate no. of reactions | 1 | 1 |
|---|---|---|
| | | |

| Reagent (concentration) | Volume (µl) | Volume (µl) |
|---|---|---|
| UPL/LNA probe (10 µM) | 0.5 | 0.5 |
| Primer-F (100 µM) | 0.15 | 0.15 |
| Primer-R (100 µM) | 0.15 | 0.15 |
| Master Probes | 10 | 10 |
| H2O | 4 | 4 |
| UNG | 0.2 | 0.2 |
| DNA | | 5 |

### 2.1.1.2. Thermal cycler program

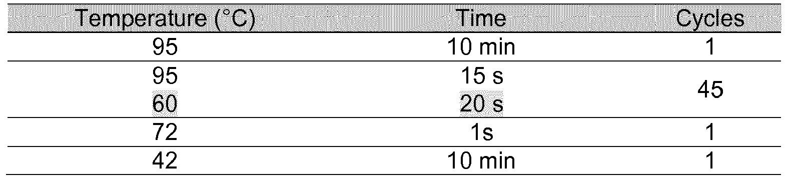

### 2.1.1.3. Detection limit of the target and amplification efficiency of each target in simplex format

To determine the detection limit of the target, 10-fold serial dilutions of a mixture of plasmid DNA (standard DNA) of the targets to be detected were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, "m" being the slope of the calibration line equation.

### 2.1.1.3.1.1. Results

| | Mean Ct obtained | | | |
|---|---|---|---|---|
| Standard plasmid DNA | *Dientamoeba fragilis* (probe 129) | *Giardia lamblia* A (probe 6) | *Giardia lamblia* B (probe 70) | *Crystosporidium parvum*/*hominis* (probe 4) |
| 10⁷ copies | 15.30 | 15.20 | 14.88 | 14.40 |
| 10⁶ copies | 18.71 | 18.61 | 18.32 | 17.93 |
| 10⁵ copies | 21.95 | 22.23 | 21.68 | 21.95 |
| 10⁴ copies | 25.59 | 26.51 | 24.97 | 25.41 |
| 10³ copies | 28.9 | 30.28 | 28.61 | 29.20 |
| 10² copies | 32.54 | 33.60 | 31.97 | 32.43 |
| 10 copies | 36.08 | 37.17 | 36.26 | 36.29^{*} |
| 1 copy | No Ct | No Ct | No Ct | No Ct |
| CN | No Ct | No Ct | No Ct | No Ct |

| | Mean Ct obtained | | | |
|---|---|---|---|---|
| | *Plasmodiun falciparum* (probe 67) | *P. ovale* (probe 119) | *P. vivax* (probe 88) | |
| 10⁷ copies | 14.03 | 14.88 | 19.23 | |
| 10⁶ copies | 17.49 | 18.16 | 22.50 | |
| 10⁵ copies | 20.74 | 21.49 | 25.84 | |
| 10⁴ copies | 24.23 | 25.37 | 29.32 | |
| 10³ copies | 27.66 | 28.64 | 32.29 | |
| 10² copies | 30.96 | 32.14 | 35.51 | |
| 10 copies | 34.91 | 35.26 | 39.66 | |
| 1 copy | No Ct | 37.93* | No Ct | |
| CN | No Ct | No Ct | No Ct | |

| | | | | |
|---|---|---|---|---|
| ^{*}Not detected in any replicates. ND: Not determined | | | | |

### 2.1.1.3.1.2. Dientamoeba fragilis (probe 129)

| **Standard curve parameters obtained** | |
|---|---|
| Slope of the line | -3.46 |
| Correlation coefficient (R2) | 0.999 |
| Efficiency | 94.5% |
| Detection limit: 10 copies | |

### 2.1.1.3.1.3. Giardia lamblia A (probe 6)

| **Standard curve parameters obtained** | |
|---|---|
| Slope of the line | -3.71 |
| Correlation coefficient (R²) | 0.993 |
| Efficiency | 86.0% |
| Detection limit: 10 copies | |

### 2.1.1.3.1.4. Giardia lamblia B (Probe 70)

| **Standard curve parameters obtained** | |
|---|---|
| Slope of the line | -3.51 |
| Correlation coefficient (R²) | 0.998 |
| Efficiency | 92.6% |
| Detection limit: 10 copies | |

### 2.1.1.3.1.5. Crystosporidium parvum/hominis (probe 4)

| **Standard curve parameters obtained** | |
|---|---|
| Slope of the line | -3.64 |
| Correlation coefficient (R²) | 0.996 |
| Efficiency | 88.2% |
| Detection limit: 10 copies | |

### 2.1.1.3.1.6. Plasmodiun falciparum (probe 67)

| **Standard curve parameters obtained** | |
|---|---|
| Slope of the line | -3.44 |
| Correlation coefficient (R²) | 0.998 |
| Efficiency | 95.0% |
| Detection limit: 10 copies | |

### 2.1.1.3.1.7. P. ovale (probe 119)

| **Standard curve parameters obtained** | |
|---|---|
| Slope of the line | -3.40 |
| Correlation coefficient (R²) | 0.998 |
| Efficiency | 96.8% |
| Detection limit: 10 copies | |

### 2.1.1.3.1.8. P. vivax (probe 88)

| **Standard curve parameters obtained** | |
|---|---|
| Slope of the line | -3.34 |
| Correlation coefficient (R²) | 0.995 |
| Efficiency | 98.9% |
| Detection limit: 10 copies | |

### 2.1.2. Parasites in stools: multiplex qPCR for the simultaneous detection of Dientamoeba fragilis (probe 129), Giardia lamblia A (probe 6), Giardia lamblia B (probe 70), Crystosporidium parvum/hominis (probe 4)

### 2.1.2.1. Multiplex qPCR reaction

| Indicate no. of reactions | 1 | |
|---|---|---|
| | | |

| Reagent (concentration) | Volume (µl) | Volurne (µl) |
|---|---|---|
| Probe 129 HEX (10 µM) | | 0.5 |
| Probe 6 Cy5 (10 µM) | | 0.5 |
| Probe 70 FAM (10 µM) | | 0.5 |
| Probe 4 Tex615 (10 µM) | | 0.5 |
| DFEF129 F (100 µM) | | 0.15 |
| DFEF129 R (100 µM) | | 0.15 |
| GLA6 F (100 µM) | | 0.15 |
| GLA6 R (100 µM) | | 0.15 |
| GLAB70 F (100 µM) | | 0.15 |
| GLAB70 R (100 µM) | | 0.15 |
| CryptoJ4 F (100 µM) | | 0.15 |
| CryptoJ4 R (100 µM) | | 0.15 |
| Maxter Mix Roche | | 10 |
| H2O | | 1.6 |
| UNG | | 0.2 |
| DNA | | 5 |
| Final volume | 0 | 20 |

### 2.1.2.3. Thermal cycler program

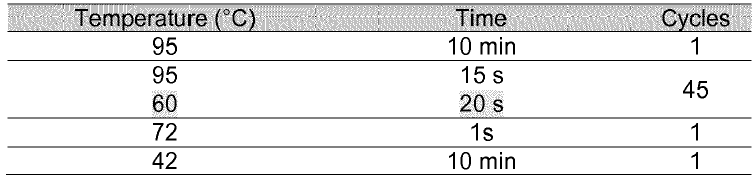

### 2.1.2.4. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m) -1, m being the slope of the calibration line equation.

### 2.1.2.4.1. Results

### 2.1.2.4.1.1. Dientamoeba fragilis (probe 129)

| **Standard plasmid DNA** | ***Dientamoeba fragilis* (probe 129)** | |
|---|---|---|
| | **Singleplex** | **Multiplex** |
| | **Mean Ct** | **Mean Ct** |
| 10⁷ copies | 15.30 | 15.90 |
| 10⁶ copies | 18.71 | 19.16 |
| 10⁵ copies | 21.95 | 22.54 |
| 10⁴ copies | 25.59 | 25.78 |
| 10³ copies | 28.9 | 29.04 |
| 10² copies | 32.54 | 32.51 |
| 10 copies | 36.08 | 35.42 |
| 1 copy | No Ct | No Ct |
| CN | No Ct | No Ct |

| **Standard curve parameters** | **Singleplex** | **Multiplex** |
|---|---|---|
| Slope of the line | -3.46 | -3.27 |
| Correlation coefficient (R²) | 0.999 | 0.998 |
| Efficiency | 94.5% | 101.9% |
| Detection limit | 10 copies | 10 copies |

| | | |
|---|---|---|
| ^{*}Not detected in any replicas. ND: Not determined | | |

### 2.1.1.3.1.3. Giardia lamblia A (probe 6)

| **Standard plasmid DNA** | ***Giardia lamblia* A (probe 6)** | |
|---|---|---|
| | **Singleplex** | **Multiplex** |
| | **Mean Ct** | **Mean Ct** |
| 10⁷ copies | 15.20 | 15.93 |
| 10⁶ copies | 18.61 | 19.88 |
| 10⁵ copies | 22.23 | 23.92 |
| 10⁴ copies | 26.51 | 26.99 |
| 10³ copies | 30.28 | 30.31 |
| 10² copies | 33.60 | 36.05 |
| 10 copies | 37.17 | 39.29 |
| 1 copy | No Ct | No Ct |
| CN | No Ct | No Ct |

| **Standard curve parameters** | **Singleplex** | **Multiplex** |
|---|---|---|
| Slope of the line | -3.71 | -3.88 |
| Correlation coefficient (R²) | 0.993 | 0.982 |
| Efficiency | 86.0% | 80.9% |
| Detection limit | 10 copies | 10 copies |

| | | |
|---|---|---|
| ^{*}Not detected in any replicas. | | |

### 2.1.2.4.1.2. Giardia lamblia B (probe 70)

| **Standard plasmid DNA** | ***Giardia lamblia* B (probe 70)** | |
|---|---|---|
| | **Singleplex** | **Multiplex** |
| | **Mean Ct** | **Mean Ct** |
| 10⁷ copies | 14.88 | 16.15 |
| 10⁶ copies | 18.32 | 19.55 |
| 10⁵ copies | 21.68 | 22.84 |
| 10⁴ copies | 24.97 | 26.24 |
| 10³ copies | 28.61 | 29.76 |
| 10² copies | 31.97 | 33.06 |
| 10 copies | 36.26 | 36.2 |
| 1 copy | No Ct | 38.73* |
| CN | No Ct | No Ct |

| **Standard curve parameters** | **Singleplex** | **Multiplex** |
|---|---|---|
| Slope of the line | -3.51 | -3.32 |
| Correlation coefficient (R²) | 0.998 | 0.998 |
| Efficiency | 92.6% | 100% |
| Detection limit | 10 copies | 10 copies |

| | | |
|---|---|---|
| * Not detected in any replicas. ND: Not determined | | |

### 2.1.2.4.1.3. Crystosporidium parvum/hominis (probe 4)

| **Standard plasmid DNA** | ***Crystosporidium parvum*/*hominis* (probe 4)** | |
|---|---|---|
| | **Singleplex** | **Multiplex** |
| | **Mean Ct** | **Mean Ct** |
| 10⁷ copies | 14.40 | 15.13 |
| 10⁶ copies | 17.93 | 19.71 |
| 10⁵ copies | 21.95 | 25.24 |
| 10⁴ copies | 25.41 | 29.36 |
| 10³ copies | 29.20 | 33.09 |
| 10² copies | 32.43 | 36.80 |
| 10 copies | 36.29* | No Ct |
| 1 copy | No Ct | No Ct |
| CN | No Ct | No Ct |

| **Standard curve parameters** | **Singleplex** | **Multiplex** |
|---|---|---|
| Slope of the line | -3.64 | -4.35 |
| Correlation coefficient (R²) | 0.996 | 0.990 |
| Efficiency | 88.2% | 69.7% |
| Detection limit | 10 copies | 100 copies |

| | | |
|---|---|---|
| *Not detected in any replicas. ND: Not determined | | |

### 2.1.3. Parasites in blood Mix 1: multiplex qPCR for the simultaneous detection of Plasmodiun falciparum (probe 67)lPlasmodium ovale (probe 119)

### 2.1.3.1. Multiplex qPCR reaction

| Indicate no. of reactions | 1 | |
|---|---|---|
| | | |

| Reagent (concentration) | Volume (µl) | Check |
|---|---|---|
| Probe 67 FAM (10 µM) | 0.5 | 0.5 |
| Probe 119 Cy5 (10 µM) | 0.5 | 0.5 |
| Pfal67F (100 µM) | 0.15 | 0.15 |
| Pfal67R (100 µM) | 0.15 | 0.15 |
| PovaF (100 µM) | 0.15 | 0.15 |
| PovaR (100 µM) | 0.15 | 0.15 |
| PvivaxF (100 µM) | 0.15 | 0.15 |
| PvivaxR (100 µM) | 0.15 | 0.15 |
| Maxter Mix Qiagen | 10 | 10 |
| H2O | 2.9 | 2.9 |
| UNG | 0.2 | 0.2 |
| DNA | 5 | 5 |
| Final volume | 15 | 20 |

### 2.1.3.2. Thermal cycler program

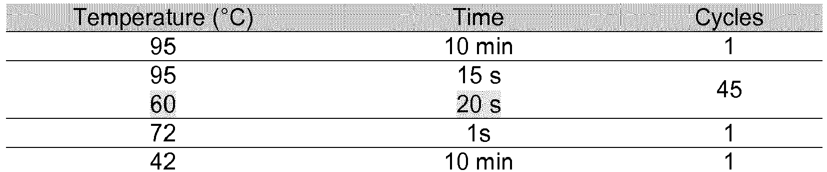

### 2.1.3.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m) -1, m being the slope of the calibration line equation.

### 2.1.1.3.1.3. Plasmodiun falciparum (probe 67)

| **Standard plasmid DNA** | ***Plasmodiun folciparum* (probe 67)** | |
|---|---|---|
| | **Singleplex** | **Multiplex** |
| | **Mean Ct** | **Mean Ct** |
| 10⁷ copies | 14.03 | 13.86 |
| 10⁶ copies | 17.49 | 17.11 |
| 10⁵ copies | 20.74 | 20.69 |
| 10⁴ copies | 24.23 | 24.12 |
| 10³ copies | 27.66 | 27.53 |
| 10² copies | 30.96 | 30.92 |
| 10 copies | 34.91 | 33.72 |
| 1 copy | No Ct | No Ct |
| CN | No Ct | No Ct |

| **Standard curve parameters** | **Singleplex** | **Multiplex** |
|---|---|---|
| Slope of the line | -3.44 | -3.37 |
| Correlation coefficient (R²) | 0.998 | 0.999 |
| Efficiency | 95.0% | 97.9% |
| Detection limit | 10 copies | 10 copies |

### 2.1.3.3.1.1. Plasmodium ovale (probe 119)

| **Standard plasmid DNA** | ***Plasmodium ovale* (probe 119)** | |
|---|---|---|
| | **Singleplex** | **Multiplex** |
| | **Mean Ct** | **Mean Ct** |
| 10⁷ copies | 14.88 | 13.30 |
| 10⁶ copies | 18.16 | 16.59 |
| 10⁵ copies | 21.49 | 20.26 |
| 10⁴ copies | 25.37 | 23.74 |
| 10³ copies | 28.64 | 26.87 |
| 10² copies | 32.14 | 30.22 |
| 10 copies | 35.26 | 33.72 |
| 1 copy | 37.93* | 36* |
| CN | No Ct | No Ct |

| **Standard curve parameters** | **Singleplex** | **Multiplex** |
|---|---|---|
| Slope of the line | -3.40 | -3.35 |
| Correlation coefficient (R²) | 0.998 | 0.998 |
| Efficiency | 96.8% | 98.7% |
| Detection limit | 10 copies | 10 copies |

| | | |
|---|---|---|
| * Not detected in any replicas | | |

### 2.1.4. Parasites in blood Mix 2: qPCR for the detection of Plasmodium vivax (probe 88)

### See singleplex results in sections 2.1.1.3.1.1. and 2.1.1.3.1.8.

### Example 10. MT Complex-VK qPCR kit for detection of M. tuberculosis complex

### 1. Product description

MT Complex-VK is a real-time PCR kit indicated for the detection of *M. tuberculosis* complex pstS11 target and whiB3 target and the endogenous *Bglo* gene. The endogenous gene works as a DNA extraction control and PCR inhibition control. It allows verifying that the extraction process has been developed correctly and detecting false negatives due to PCR inhibition. The kit contains the required reagents and enzymes mixed at ideal concentrations and designed to make diagnostic analysis easier.

The kit contains the following mixes:
- **Mix 1** provides a FAM-labeled LNA probe for the detection of *M. tuberculosis* complex whiB3 target, a Tex615-labeled LNA probe for the detection of *M. tuberculosis* complex pstS11 target and a Cy5-BHQ3®-labeled LNA probe for the detection of the *Bglo* gene (DNA extraction control/PCR inhibition control gene).

The channels through which fluorescence-related data is collected are described in detail in Table 1.

**Table 1: Reporters and Quenchers**

| Mix 1 | Reporter | Quencher |
|---|---|---|
| whiB3 | FAM | Roche |
| pstS11 | Tex615/ROX | Exiqon |
| *Bglo* gene | Cy5 | BHQ3® |

The UPL probe as well as the forward (F) and reverse (R) primers have the sequences provided in detail below:

| **Syndrome** | **Pathogen** | **Forward primer seq. (F)** | **Reverse primer seq. (R)** |
|---|---|---|---|
| ***Tuberculosi s*** | *M. tuberculosis* complex whiB3 | 5’- GGACTCATCGATGTTCTTCC A-3’ | 5'- GCCGACACATTTCCTTGG-3' |
| ***Tuberculosi s*** | *M. tuberculosis* complex pstS11 | 5’-TCCGAACGTCACGATCAC- 3’ | 5’- TCACCTTCCGACAGATAGG C-3 |

| **Syndrome** | **Pathogen** | **Probe No**. | **Amplicon seq**. | **Amplicon (bp)** | **Probe seq.** |
|---|---|---|---|---|---|
| ***Tuberculosi s*** | *M. tuberculosis* complex whiB3 | 152 | GGACTCATCGATGTTC TTCCATCCCGACGGC GAGCGTGGCCGTGCC CGAACGCAGCGCGAA CAACGCGCCAAGGAA ATGTGTCGGC | 86 | GACGGC GA |
| ***Tuberculosi s*** | *M. tuberculosis* complex pstS11 | 70 | TCCGAACGTCACGATC ACCGCTCAGGGCACC GGTTCTGGTGCCGGG ATCGCGCAGGCCGCC GCCGGGACGGTCAAC ATTGGGGCCTCCGAC GCCTATCTGTCGGAAG GTGA | 111 | GGCGGC GG |

The probe as well as primers for the *Bglo* gene have the sequences provided in detail below:

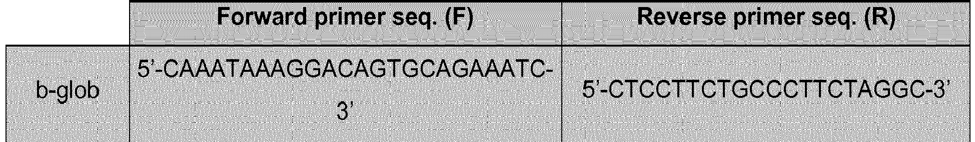

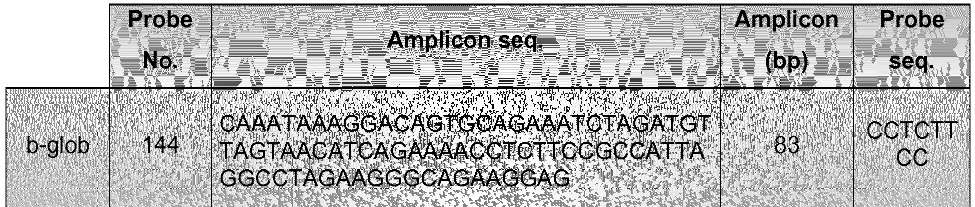

### 2. Singleplex assay (Comparative example)

### 2.1. Protocol validation by means of standards

### 2.1.1. Singleplex qPCR for the detection of M. tuberculosis complex target 1 and target 2

### 2.1.1.1. qPCR reaction

| Indicate no. of reactions | 1 | 1 |
|---|---|---|
| | | |

| Reagent (concentration) | Volume (µl) | Volume (µl) |
|---|---|---|
| Probe UPL (10 µM) | 0.5 | 0.5 |
| First-F (100 µM) | 0.15 | 0.15 |
| Primer-R (100 µM) | 0.15 | 0.15 |
| Master Probes | 10 | 10 |
| H2O | 4 | 4 |
| UNG | 0.2 | 0.2 |
| DNA | 5 | 5 |
| Final volume | 20 | 20 |

### 2.1.1.2. Thermal cycler program

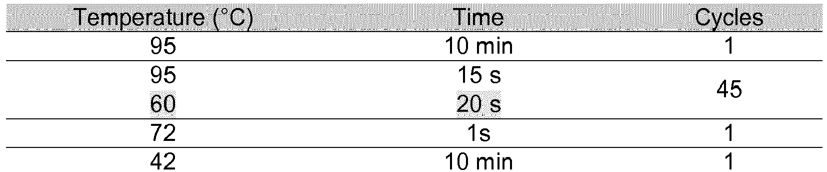

### 2.1.1.3 Detection limit of the target and amplification efficiency of each target in simplex format

To determine the detection limit of the target, 10-fold serial dilutions of a mixture of plasmid DNA (standard DNA) of the targets to be detected were tested in a range of concentration usually varying between 10⁷ copies and 1 copy per well using 3 replicates per dilution.

Calibration curve parameters were analyzed to determine qPCR amplification efficiency. Efficiency: 10(-1/m) -1, "m" being the slope of the calibration line equation.

**2.1.1.3.1. Results**

| | Mean Ct obtained | | |
|---|---|---|---|
| Standard plasmid DNA | whiB3 (probe 152) | pstS11 (probe 70) | Endogenous *Bglo* gene (probe 144) |
| 10⁷ copies | 19.36 | 17.86 | 15.35 |
| 10⁶ copies | 22.93 | 21.53 | ND |
| 10⁵ copies | 26.53 | 25.16 | 21.73 |
| 10⁴ copies | 30.16 | 28.76 | 24.76 |
| 10³ copies | 33.83 | 32.19 | 28.22 |
| 10² copies | 36.06 | 36.14 | 31.92 |
| 10 copies | 38.64 | 37.94 | 34.32 |
| 1 copy | No Ct | No Ct | 37.06* |
| CN | No Ct | No Ct | No Ct |

| | | | |
|---|---|---|---|
| ^{*} Not detected in any replicates. ND: Not determined | | | |

### 2.1.1.3.1.1. whiB3 (probe 152)

| **Standard curve parameters obtained** | |
|---|---|
| Slope of the line | -3.30 |
| Correlation coefficient (R²) | 0.993 |
| Efficiency | 100.7% |
| Detection limit: 10 copies | |

### 2.1.1.3.1.2. pstS11 (probe 70)

| **Standard curve parameters obtained** | |
|---|---|
| Slope of the line | -3.44 |
| Correlation coefficient (R²) | 0.997 |
| Efficiency | 95% |
| Detection limit: 10 copies | |

### 2.1.1.3.1.3. Bglo gene Cy5 (probe 144)

| **Standard curve parameters obtained** | |
|---|---|
| Slope of the line | -3.27 |
| Correlation coefficient (R²) | 0.997 |
| Efficiency | 102% |
| Detection limit: 10 copies | |

### 2.1.2. Mix 1: multiplex qPCR for the simultaneous detection of whiB3 (probe 152), pstS11 (probe 70) and the endogenous Bglo gene (probe 144)

### 2.1.2.1. Multiplex qPCR reaction

| Indicate n. of reactions | 1 | |
|---|---|---|
| | | |

| Reagent (concentration) | Volume (µl) | Check |
|---|---|---|
| WhiB3 FAM Probe 152 (10 µM) | 0.5 | 0.5 |
| PstS11 tex615 Probe 70 (10 µM) | 0.5 | 0.5 |
| Probe Bglo144Cy5 (10 µM) | 0.5 | 0.5 |
| WhiB3 152-F (100 µM) | 0.15 | 0.15 |
| WhiB3 152-R (100 µM) | 0.15 | 0.15 |
| PstS11 70F (100 µM) | 0.15 | 0.15 |
| PstS11 70R (100 µM) | 0.15 | 0.15 |
| Bglo144F (100 µM) | 0.15 | 0.15 |
| Bglol44R (100 µM) | 0.15 | 0.15 |
| Master Mix Roche | 10 | 10 |
| H2O | 2.4 | 2.4 |
| UNG | 0.2 | 0.2 |
| DNA | 5 | 5 |
| Final volume | 20 | 20 |

### 2.1.2.2. Thermal cycler program

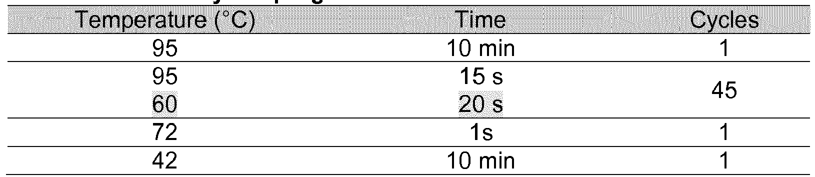

### 2.1.2.3. Detection limit of the target and multiplex amplification efficiency

To determine the detection limit of the target, 10-fold serial dilutions of a mixture of plasmid DNA (standard DNA) of the targets to be detected were tested in a range of concentration usually varying between 10⁷ copies and 1 copy per well, 3 replicates per dilution.

Calibration curve parameters were analyzed to determine qPCR amplification efficiency. Efficiency: 10(-1/m) -1, m being the slope of the calibration line equation.

### 2.1.2.3.1. Results

**2.1.2.3.1.1. whiB3 (probe 152)**

| **Standard plasmid DNA** | **whiB3 (probe 152)** | |
|---|---|---|
| | **Singleplex** | **Multiplex** |
| | **Mean Ct** | **Mean Ct** |
| 10⁷ copies | 19.36 | 15.82 |
| 10⁶ copies | 22.93 | 19.81 |
| 10⁵ copies | 26.53 | 23.2 |
| 10⁴ copies | 30.16 | 26.55 |
| 10³ copies | 33.83 | 30.25 |
| 10² copies | 36.06 | 32.41 |
| 10 copies | 38.64 | 36.11 |
| 1 copy | No Ct | 39.55^{*} |
| CN | No Ct | No Ct |

| **Standard curve parameters** | **Singleplex** | **Multiplex** |
|---|---|---|
| Slope of the line | -3.30 | -3.32 |
| Correlation coefficient (R²) | 0.993 | 0.995 |
| Efficiency | 100.7% | 99.7% |
| Detection limit | 10 copies | 10 copies |

| | | |
|---|---|---|
| * Not detected in any replicas. ND: Not determined | | |

**2.1.2.3.1.2. pstS11 (probe 70)**

| **Standard plasmid DNA** | **pstS11 (probe 70)** | |
|---|---|---|
| | **Singleplex** | **Multiplex** |
| | **Mean Ct** | **Mean Ct** |
| 10⁷ copies | 17.86 | 16.14 |
| 10⁶ copies | 21.53 | 19.92 |
| 10⁵ copies | 25.16 | 23.28 |
| 10⁴ copies | 28.76 | 26.84 |
| 10³ copies | 32.19 | 30.29 |
| 10² copies | 36.14 | 33.94 |
| 10 copies | 37.94 | No Ct |
| 1 copy | No Ct | No Ct |
| CN | No Ct | No Ct |

| **Standard curve parameters** | **Singleplex** | **Multiplex** |
|---|---|---|
| Slope of the line | -3.44 | -3.35 |
| Correlation coefficient (R²) | 0.997 | 0.999 |
| Efficiency | 95% | 92.0% |
| Detection limit | 10 copies | 100 copies |

| | | |
|---|---|---|
| ^{*} Not detected in any replicas. ND: Not determined | | |

**2.1.2.3.1.3. Bglo gene (probe 144)**

| **Standard plasmid DNA** | ***Bglo* gene (probe 144)** | |
|---|---|---|
| | **Singleplex** | **Multiplex** |
| | **Mean Ct** | **Mean Ct** |
| 10⁷ copies | 15.35 | 15.71 |
| 10⁶ copies | ND | 19.1 |
| 10⁵ copies | 21.73 | 22.63 |
| 10⁴ copies | 24.76 | 26.05 |
| 10³ copies | 28.22 | 29.39 |
| 10² copies | 31.92 | 32.3 |
| 10 copies | 34.32 | 36.41 |
| 1 copy | 37.06^{*} | No Ct |
| CN | No Ct | No Ct |

| **Standard curve parameters** | **Singleplex** | **Multiplex** |
|---|---|---|
| Slope of the line | -3,27 | -3.39 |
| Correlation coefficient (R²) | 0,997 | 0.998 |
| Efficiency | 102% | 97.2% |
| Detection limit | 10 copies | 10 copies |

| | | |
|---|---|---|
| ^{*} Not detected in any replicas. ND: Not determined | | |

## Claims

1. An *in vitro* method for the detection, quantification and identification of microorganisms of two or more different strains or species in one and the same sample, preferably biological sample, more preferably isolated from a human subject, through polymerase chain reaction (PCR), preferably performed in a quantitative manner in real time, which comprises detecting, identifying and quantifying the presence or absence of said microorganisms in the biological sample through the determination and quantification of the presence or absence of amplicons from the polymerase chain reaction (PCR);
wherein said polymerase chain reaction (PCR) uses DNA, complementary DNA (cDNA) or single-stranded DNA obtained by the reverse transcription of ribonucleic acid (RNA) of the biological sample, at least one pair of oligonucleotide initiators or primers for each of the pathogens to be identified as well as at least one labeled probe for each of the pathogens to be identified, dNTPs, a suitable reaction buffer and a heat-stable DNA polymerase, wherein each of said labeled probes consist of a fluorophore-labeled oligonucleotide with a length of 8 to 9 nucleotides, and wherein each of said probes is labeled with a different fluorophore.

2. The method according to claim 1, wherein said method determines the presence or absence of three or more different strains or species of microorganisms in one and the same biological sample from a subject, and wherein said polymerase chain reaction (PCR) uses at least three pairs of specific primers and at least three fluorescently labeled probes, wherein each of said fluorescently labeled probes consists of a fluorophore-labeled oligonucleotide with a length of 8 to 9 nucleotides, and wherein each of said probes is labeled with a different fluorophore.

3. The method according to claim 1, wherein said method determines the presence or absence of four or more different strains or species of microorganisms in one and the same biological sample from a subject, and wherein said polymerase chain reaction (PCR) uses at least four pairs of specific primers and at least four fluorescently labeled probes, wherein each of said fluorescently labeled probes consists of a fluorophore-labeled oligonucleotide with a length of 8 to 9 nucleotides, and wherein each of said probes is labeled with a different fluorophore.

4. The method according to any of claims 1 to 3, wherein said probes consist of synthetic oligonucleotides.

5. The method according to claim 4, wherein said probes consist of LNA (Locked Nucleic Acids)-type synthetic oligonucleotides.

6. The method according to any of the preceding claims, wherein said probes consist of an oligonucleotide with a length of 8 nucleotides.

7. The method according to any of the preceding claims, wherein said polymerase chain reaction (PCR) amplifies amplicons with a length between 60 and 120 nucleotides.

8. The method according to any of the preceding claims, wherein the fluorescently labeled probe is a Taqman-type probe.

9. The method according to any of the preceding claims, wherein the subject is a human.

10. The method according to any of the preceding claims, wherein said microorganisms are pathogens.

11. The method according to claim 10, wherein said pathogen is selected from the list consisting of *Escherichia coli*, *Streptococcus agalactiae*, *Listeria monocytogenes*, *Mycobacterium tuberculosis* complex, *Streptococcus pneumoniae*, *S*. *pyogenes*, *Haemophilus influenzae, Staphylococcus aureus, Kingella kingae, Bordetella pertussis, B. parapertussis, B. holmesii, Mycoplasma pneumoniae, Coxiella burnetti, Chlamydia pneumoniae, Legionella pneumophila,* herpes simplex virus 1, herpes simplex virus 2, varicella zoster virus, VHS-8, herpesvirus 4, cytomegalovirus, adenovirus, *Candida albicans, C. parapsilopsis, C. glabrata, C. krusei, C. tropicalis, C. guilliermondii, Aspergillus flavus, A. fumigatus, Pneumocystis jiroveci, Dientamoeba fragilis*, *Giardia lamblia* A*, Giardia lamblia* B, *Crystosporidium parvumlhominis, Plasmodiun falciparum, P. ovale* and/or *P. vivax.*

12. The method according to any of the preceding claims, wherein before determining the presence or absence of the microorganisms, treatment is carried out on the reaction mixture which will be used for carrying out polymerase chain reaction (PCR) performed in a quantitative manner in real time with uracil-DNA-glycosylase (UNG).

13. The method according to claim 12, wherein said UNG originates from Atlantic cod (*Gadus morhua*).

14. The method according to any of the preceding claims, wherein said method is for obtaining data useful for the diagnosis of a pathology caused by a microorganism based on the presence or absence of said microorganism in a biological sample, preferably obtained from a human subject.

15. The method according to any of claims 1 to 14, wherein said microorganisms are herpes simplex virus 1, herpes simplex virus 2 and varicella zoster virus and wherein said method is **characterized by** comprising probe 63, probe 157 and probe 142.

16. The method according to claim 15, wherein said method is for obtaining data useful for the diagnosis of viral meningitis based on the presence or absence of at least one of said microorganisms in a biological sample, preferably obtained from a human subject.

17. The method according to any of claims 1 to 14, wherein said microorganisms are *Escherichia coli, Streptococcus agalactiae* and *Listeria monocytogenes* and wherein said method is **characterized by** comprising probe 9, probe 56 and probe 142.

18. The method according to claim 17, wherein said method is for obtaining data useful for the diagnosis of neonatal sepsis based on the presence or absence of at least one of said microorganisms in a biological sample, preferably obtained from a human subject.

19. The method according to any of claims 1 to 14, wherein said microorganisms are *Streptococcus pneumoniae, S. pyogenes, Haemophilus influenzae, Staphylococcus aureus* and *Kingella kingae* and wherein said method is **characterized by** comprising probe 129, probe 38, probe 110 and probe 84.

20. The method according to claim 19, wherein said method is for obtaining data useful for the diagnosis of infectious arthritis based on the presence or absence of at least one of said microorganisms in a biological sample, preferably obtained from a human subject.

21. The method according to any of claims 1 to 14, wherein said microorganisms are *Candida albicans, C. parapsilopsis, C. glabrata, C. krusei, C. tropicalis, C. guilliermondii, Aspergillus flavus* and *A. fumigatus* and wherein said method is **characterized by** comprising probe 131, probe 67, probe 155, probe 4, probe 147, probe 129, probe 84 and probe 70.

22. The method according to claim 21, wherein said method is for obtaining data useful for the diagnosis of invasive fungal infections based on the presence or absence of at least one of said microorganisms in a biological sample, preferably obtained from a human subject.

23. The method according to any of claims 1 to 14, wherein said microorganisms are VHS-8, herpesvirus 4 (Epstein Barr), cytomegalovirus and adenovirus and wherein said method is **characterized by** comprising probe 150, probe 40 and probe 108.

24. The method according to claim 23, wherein said method is for obtaining data useful for the diagnosis of viral infections in transplant recipients based on the presence or absence of at least one of said microorganisms in a biological sample, preferably obtained from a human subject.

25. The method according to any of claims 1 to 14, wherein said microorganisms are *Bordetella pertussis, B. parapertussis* and *B. holmesii* and wherein said method is **characterized by** comprising probe 86, probe 40 and probe 81.

26. The method according to claim 25, wherein said method is for obtaining data useful for the diagnosis of whooping cough based on the presence or absence of at least one of said microorganisms in a biological sample, preferably obtained from a human subject.

27. The method according to any of claims 1 to 14, wherein said microorganisms are *Mycoplasma pneumoniae, Coxiella bumetti, Chlamydia pneumoniae, Legionella pneumophila* and *Pneumocystis jiroveciii* and wherein said method is **characterized by** comprising probe 150, probe 59, probe 67, probe 60 and probe 141.

28. The method according to claim 27, wherein said method is for obtaining data useful for the diagnosis of atypical pneumoniae based on the presence or absence of at least one of said microorganisms in a biological sample, preferably obtained from a human subject.

29. The method according to any of claims 1 to 14, wherein said microorganisms are *Dientamoeba fragilis, Giardia lamblia* A*, Giardia lamblia* B*, Crystosporidium parvum*/*hominis*, *Plasmodiun falciparum, P. ovale* and *P. vivax* and wherein said method is **characterized by** comprising probe 129, probe 6, probe 70, probe 4, probe 67, probe 119 and probe 88.

30. The method according to claim 29, wherein said method is for obtaining data useful for the diagnosis of the presence of parasites based on the presence or absence of at least one of said microorganisms in a biological sample, preferably obtained from a human subject.

31. The method according to any of claims 1 to 14, wherein said microorganism is *M*. *tuberculosis* and wherein said method is **characterized by** comprising probe 152 and probe 70.

32. The method according to claim 31, wherein said method is for obtaining data useful for the diagnosis of tuberculosis based on the presence or absence of at least one target selected from the list consisting of the pstS11 target and the whiB3 target in a biological sample, preferably obtained from a human subject.

33. *In vitro* use of a kit or device comprising at least two pairs of specific primers and at least two fluorescently labeled probes, wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

34. *In vitro* use of a kit comprising at least three pairs of specific primers and at least three fluorescently labeled probes, wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

35. *In vitro* use of a kit comprising at least four pairs of specific primers and at least four fluorescently labeled probes, wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

36. *In vitro* use of a kit comprising at least five pairs of specific primers and at least five fluorescently labeled probes, wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

37. *In vitro* use of a kit comprising at least six pairs of specific primers and at least six fluorescently labeled probes, wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

38. *In vitro* use of a kit comprising at least seven pairs of specific primers and at least seven fluorescently labeled probes, wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

39. *In vitro* use of a kit comprising at least eight pairs of specific primers and at least eight fluorescently labeled probes, wherein each of said fluorescently labeled probes consists of a fluorophore-labeled oligonucleotide with a length of 8 to 9 nucleotides, and wherein each of said probes is labeled with a different fluorophore, for determining the presence or absence of two or more different strains or species of microorganisms in one and the same isolated biological sample.

40. Use according to any of claims 33 to 39, wherein said kit additionally comprises at least one of the following elements: dNTPs, a suitable reaction buffer, a heat-stable DNA polymerase, a UNG, preferably the UNG of Atlantic cod (*Gadus morhua*), and a positive control, preferably B-globin.

41. A kit comprising at least three fluorescently labeled probes, wherein each of said fluorescently labeled probes consists of a fluorophore-labeled oligonucleotide with a length of 8 to 9 nucleotides selected from the list consisting of probe 63, probe 157 and probe 142, and wherein each of said probes is labeled with a different fluorophore.

42. A kit comprising at least three fluorescently labeled probes, wherein each of said fluorescently labeled probes consists of a fluorophore-labeled oligonucleotide with a length of 8 to 9 nucleotides selected from the list consisting of probe 9, probe 56 and probe 142, and wherein each of said probes is labeled with a different fluorophore.

43. A kit comprising at least four fluorescently labeled probes, wherein each of said fluorescently labeled probes consists of a fluorophore-labeled oligonucleotide with a length of 8 to 9 nucleotides selected from the list consisting of probe 129, probe 38, probe 110 and probe 84, and wherein each of said probes is labeled with a different fluorophore.

44. A kit comprising at least eight fluorescently labeled probes, wherein each of said fluorescently labeled probes consists of a fluorophore-labeled oligonucleotide with a length of 8 to 9 nucleotides selected from the list consisting of probe 131, probe 67, probe 155, probe 4, probe 147, probe 129, probe 84 and probe 70, and wherein each of said probes is labeled with a different fluorophore.

45. A kit comprising at least three fluorescently labeled probes, wherein each of said fluorescently labeled probes consists of a fluorophore-labeled oligonucleotide with a length of 8 to 9 nucleotides selected from the list consisting of probe 150, probe 40 and probe 108, and wherein each of said probes is labeled with a different fluorophore.

46. A kit comprising at least three fluorescently labeled probes, wherein each of said fluorescently labeled probes consists of a fluorophore-labeled oligonucleotide with a length of 8 to 9 nucleotides selected from the list consisting of probe 86, probe 40 and probe 81, and wherein each of said probes is labeled with a different fluorophore.

47. A kit comprising at least five fluorescently labeled probes, wherein each of said fluorescently labeled probes consists of a fluorophore-labeled oligonucleotide with a length of 8 to 9 nucleotides selected from the list consisting of probe 150, probe 59, probe 67, probe 60 and probe 141, and wherein each of said probes is labeled with a different fluorophore.

48. A kit comprising at least seven fluorescently labeled probes, wherein each of said fluorescently labeled probes consists of a fluorophore-labeled oligonucleotide with a length of 8 to 9 nucleotides selected from the list consisting of probe 129, probe 6, probe 70, probe 4, probe 67, probe 119 and probe 88, and wherein each of said probes is labeled with a different fluorophore.

49. A kit comprising at least two fluorescently labeled probes, wherein each of said fluorescently labeled probes consists of a fluorophore-labeled oligonucleotide with a length of 8 to 9 nucleotides selected from the list consisting of probe 152 and probe 70, and wherein each of said probes is labeled with a different fluorophore.

50. A kit according to any of claims 41 to 49, wherein said kit further comprises any of the elements defined in claim 32.

51. *In vitro* use of the kit according to claim 41 for determining the presence or absence of herpes simplex virus 1, herpes simplex virus 2 and/or varicella zoster virus in one and the same biological sample.

52. *In vitro* use of the kit according to claim 42 for determining the presence or absence of *Escherichia coli, Streptococcus agalactiae* and/or *Listeria monocytogenes* in one and the same biological sample.

53. *In vitro* use of the kit according to claim 41 for obtaining data useful in the diagnosis of viral meningitis.

54. *In vitro* use of the kit according to claim 42 for obtaining data useful in the diagnosis of neonatal sepsis.

55. *In vitro* use of the kit according to claim 43 for determining the presence or absence of *Streptococcus pneumoniae, S. pyogenes, Haemophilus influenzae, Staphylococcus aureus* and/or *Kingella kingae* in one and the same biological sample.

56. *In vitro* use of the kit according to claim 43 for obtaining data useful in the diagnosis of infectious arthritis.

57. *In vitro* use of the kit according to claim 44 for determining the presence or absence of *Candida albicans, C. parapsilopsis, C. glabrata, C. krusei, C. tropicalis, C. guilliermondii, Aspergillus flavus* and/or *A. fumigatus* in one and the same biological sample.

58. *In vitro* use of the kit according to claim 44 for obtaining data useful in the diagnosis of fungal infection.

59. *In vitro* use of the kit according to claim 45 for determining the presence or absence of VHS-8, herpesvirus 4 (Epstein Barr), cytomegalovirus and/or adenovirus in one and the same biological sample.

60. *In vitro* use of the kit according to claim 45 for obtaining data useful in the diagnosis of virosis in immunosuppressed individuals.

61. *In vitro* use of the kit according to claim 46 for determining the presence or absence of *Bordetella pertussis, B. parapertussis* and *B. holmesii* in one and the same biological sample.

62. *In vitro* use of the kit according to claim 46 for obtaining data useful in the diagnosis of whooping cough.

63. *In vitro* use of the kit according to claim 47 for determining the presence or absence of *Mycoplasma pneumoniae, Coxiella burnetti, Chlamydia pneumoniae, Legionella pneumophila* and *Pneumocystis jiroveciii* in one and the same biological sample.

64. *In vitro* use of the kit according to claim 47 for obtaining data useful in the diagnosis of atypical pneumoniae.

65. *In vitro* use of the kit according to claim 48 for determining the presence or absence of *Dientamoeba fragilis, Giardia lamblia* A*, Giardia lamblia* B*, Crystosporidium parvum*/*hominis, Plasmodiun falciparum, P. ovale* and *P. vivax* in one and the same biological sample.

66. *In vitro* use of the kit according to claim 48 for obtaining data useful in the diagnosis of the presence of parasites in stools and/or blood.

67. *In vitro* use of the kit according to claim 49 for determining the presence or absence of *M. tuberculosis* in one and the same biological sample.

68. *In vitro* use of the kit according to claim 49 for obtaining data useful in the diagnosis of tuberculosis.
